# EUROPEAN PATENT APPLICATION

(11) **EP 2 486 910 A2**
(43) Date of publication of application: **15.08.2012**
(21) Application number: 11190908.1
(22) Date of filing: 26.10.2007
(51) Int. Cl.: A61J 1/20

(54) **Multi-chambered apparatus comprising a dispenser head**

(30) Priority: 27.10.2006 US 863179 P; 29.01.2007 US 887095 P; 18.07.2007 US 950549 P
(62) Divisional of application: 07868597.1
(71) Applicant: The Curators Of The University Of Missouri, Columbia, Missouri 65211 (US)
(72) Inventor: Phillips, Jeffrey O., Ashland, MO 65010 (US)
(74) Representative: Haile, Alison Victoria

(57) **Abstract**

The present disclosure relates to a multi-chambered apparatus comprising:
a first chamber wherein one or more first pharmaceutically active agents, protective buffers or thickeners can be contained;
a second chamber wherein one or more second pharmaceutically active agents, flavoring agents, sweeteners, disintegrants, additives, buffering agents, or thickeners can be contained; and
a dispenser head.

## Description

### RELATED APPLICATIONS

This application claims priority to U.S. Provisional Application Serial No. 60/950,549, filed on Jul. 18, 2007; U.S. Provisional Application Serial No. 60/887,095, filed on Jan. 29, 2007; U.S. Provisional Application Serial No. 60/863,179, filed on Oct. 27. 2006; PCT/US2007/060723 filed Jan. 18, 2007, which claims priority to U.S. Provisional Application Serial No. 60/760,256, filed on Jan. 19, 2006, now abandoned; PCT/US2006/040756 filed Oct. 18, 2006, which claims priority to U.S. Provisional Application Serial No. 60/728,125, filed on Oct. 19, 2005, now abandoned; PCT/US2006/015939 filed Apr. 25, 2006, which claims priority to U.S. Provisional Application Serial No. 60/675,122, filed on Apr. 26, 2005, now abandoned; PCT/US2006/015982 filed Apr. 25, 2006, which claims priority to U.S. Provisional Application Serial No. 60/675,133, filed on Apr. 26, 2005, now abandoned; and PCT/US2006/015983 filed Apr. 25, 2006, which claims priority to U.S. Provisional Application Serial No. 60/675,260, filed on Apr. 26, 2005, now abandoned. This application claims priority to all such previous applications, and such applications are hereby incorporated herein by reference.

### FIEL OF THE INVENTION

Described herein are pharmaceutical compositions comprising at least one acid labile pharmaceutical agent or related compound, such as tenatoprazole, s-tenatoprazole, omeprazole, lansoprazole, pantoprazole, esomeprazole, s-lansoprazole and at least one other pharmaceutically active agent. Also described herein is an apparatus for administering multidosing formulations for administration of pharmaceuticals. Methods of for using such compositions and apparatus are also provided.

### BACKGROUND

As used herein, the phrase "acid labile pharmaceutical agent" refers to any pharmacologically active drug subject to acid-catalyzed degradation. One class of acid labile pharmaceutical agents is a class of antisecretory agents that do not exhibit anticholinergic or H₂ histamine antagonistic properties, but that suppress gastric acid secretion by the specific inhibition of the H⁺, K⁺-ATPase enzyme system at the secretory surface of the gastric parietal cell (hereinafter "proton pump inhibitors" or "PPIs"). These agents provide a more specific class of inhibitors of gastric acid secretion in mammals, such as humans, by blocking the final step of acid production.

One particular class of PPIs includes substituted benzimidazole compounds that contain a sulfinyl group bridging substituted benzimidazole and pyridine rings. Another class of PPIs is the class of substituted aryl-imidazoles, such as substituted bicyclic aryl-imidazoles. Still another particular class of PPIs are the substituted imidazopyridines, which differ from substituted benzimidazole compounds in that they have an imidazopyridine moiety, instead of a benzimidazole moiety. At neutral to alkaline pH, these classes of PPIs are chemically stable, lipid-soluble compounds that have little or no inhibitory activity.

It is believed that the neutral PPIs reach parietal cells from the blood and diffuse into the secretory canaliculi where they become protonated and thereby trapped. The protonated agent is then believed to rearrange to form a sulfenic acid and a sulfenamide. The sulfenamide, in turn, is thought to interact covalently with sulfhydryl groups at critical sites in the extracellular (luminal) domain of the membrane-spanning H⁺, K⁺-ATPase. *See e.g.* Hardman et al., Goodman & Gilman's The Pharmacological Basis of Therapeutics, p. 907, 9th ed. (1996).

Unfortunately, commercially available substituted benzimidazole compounds are unstable at neutral or acidic pH and undergo decomposition in gastrointestinal fluid upon oral administration, thereby resulting in loss of therapeutic activity. To overcome this acid instability, such compounds are typically formulated for oral delivery as enteric coated solid dosage forms, for example enteric coated tablets, which coating protects the drug from contact with acidic stomach secretions. An undesirable consequence of such enteric coating is that therapeutic onset time is significantly delayed by comparison with non-enteric coated dosage forms. Such prolonged time to therapeutic onset is particularly undesirable for patients in need of rapid relief from one or more of the above described disorders or symptoms. In fact, it often it takes 4 or 5 days for enteric coated PPIs to exert their maximal effect on gastric pH.

Specific examples of enteric coated PPIs include omeprazole (Prilosec®), lansoprazole (Prevacid®), perprazole (also referred to as esomeprazole; Nexium®), rabeprazole (Aciphex®), and pantoprazole (Protonix®). Omeprazole, a substituted benzimidazole, has the following chemical name: 5-methoxy-2-[(4-methoxy-3,5-dimethyl-2-pyridinyl) methyl] sulfinyl]-1 benzimidazole. An example of a non-enteric coated PPI is omeprazole (Zegerid®).

U.S. Patent No. 4,786,505 to Lovgren et al. discloses that a pharmaceutical oral solid dosage form of omeprazole must be protected from contact with acidic gastric juice by an enteric coating to maintain its pharmaceutical activity. That patent describes an enteric coated omeprazole preparation containing an alkaline core comprising omeprazole, a subcoating over the core, and an enteric coating over the subcoating.

One recent attempt to overcome acid instability associated with PPIs is disclosed in U.S. Patent No. 6,617,338 to Mali et al. The Mali patent discloses use of polymerized benzimidazoles for the treatment or prevention of peptic ulcers, gastrointestinal inflammatory disease or gastrointestinal disorder. Polymerized benzimidazoles disclosed therein are said to be more acid stabile than respective parent benzimidazole moieties. In fact, Mali *et al.,* state that the polymeric benzimidazoles of that invention are found to be acid stable due to the polymeric N-substitution and therefore do not disintegrate in the gastrointestinal fluid and are suitable for administration without enteric coating. However, it is presently believed that polymeric benzimidazole compositions disclosed by Mali *et al.,* will not adequately overcome the *in vivo* acid degradation problems described above.

Other commercially available acid-labile pharmaceutical agents try to address the acid instability by administering them in solid form or at a certain pH in a solution or suspension. For example, PPIs often need to be combined with a buffer (such as sodium bicarbonate) when administered. The pKa of a PPI ranges from about 3 to about 5, and the PPI and buffer formulation is often stored as a powder. Upon the addition of water, such powder becomes a suspension for oral administration, which is suitable for use as a single dosage and should be used in a short period of time. However, a fresh suspension has to be made for each administration, since the resulting suspension cannot be stored for continued dosing.

In other words, known available PPI pharmaceuticals are not formulated as true suspensions that are capable of being used for administering titratable dosages, or they are available as oral suspensions, where the entire contents of a sachet must be given at one time. Current formulations experience difficulty in maintaining suitable stability of the product, or in proving the stability of the product after it has been constituted with water and allowed to sit beyond the initial dosing period for which the particular dose was constituted. This type of formulation cannot be formulated in advance in a multidose formulation, for example a bottle from which multiple doses of medicine can be withdrawn. As a result, embodiments that employ single-dose formulations or dose-titratable sachets are inconvenient or wasteful, since the unused portion of the dose must be discarded.

There are other instances in which two or more pharmaceuticals in liquid or suspension forms need to be administered together. For example, two or more pharmaceuticals might be needed at a certain ratio as one dose for multiple doses, but the pharmaceuticals may not be compatible for mixing or storing as a pre-made mixture for continued dosing. Current methods call for the pharmaceuticals to be measured individually and administered individually or as a freshly prepared mixture for each dose.

In another example in which there is a need for multiple administrations of different compositions, the pharmaceutical can be administered with an additive to mask the bitter taste. However, excipients and additives such as flavoring, sugars, sweeteners, thickeners can interfere with the stability of the pharmaceuticals when stored for an extending period beyond immediate administration. Adding the right amount of additives or excipients at each dosing can also be labor and time consuming.

Therefore, there is a need for a method for preparing multidosing formulations for oral administration of pharmaceuticals, additives, excipients, buffering agents, suspensions, liquids, reagents or solutions.

In addition, currently available PPI medications have a delay in reaching maximal effect (for example, maintaining a gastric pH above about 3.5) such that it can take approximately five days to reach a steady state effect. Therefore, it would be desirable to have a significant effect on gastric pH on Day 1 with the first dose of the medication.

There is a continuing need for combination therapy to treat multiple symptoms associated with acid-caused disorders. In addition, there is a need to address gastric-acid related disorders in non-human subjects, such as horses.

A significant advance in the treatment of gastric acid-related diseases and disorders would result from pharmaceutical compositions which provide rapid inhibition or neutralization of gastric acid and continuous inhibition or neutralization of gastric acid from the first dose. An advance in the art would result if compositions overcoming these and other problems could be prepared. A further advance would allow for multiple-dose containment and administration of product.

### SUMMARY

In various embodiments, the present disclosure provides pharmaceutical compositions comprising a first proton pump inhibitor (PPI1), having a therapeutically effective portion, which is optionally enteric coated; a subsequent proton pump inhibitor (PPI2), having a therapeutically effective portion, which is optionally enteric coated; and one or more buffering agents.

Another embodiment provides for a pharmaceutical composition comprising tenatoprazole, having a therapeutically effective portion of which is optionally not enteric coated, and one or more buffering agents.

Another embodiment discloses a pharmaceutical composition comprising a proton pump inhibitor in a salt form, having a therapeutically effective portion which is optionally enteric coated, a proton pump inhibitor in a free base form, having a therapeutically effective portion which is optionally enteric coated, and one or more buffering agents.

Still another embodiment described herein discloses a pharmaceutical composition comprising a proton pump inhibitor in a salt form, having a therapeutically effective portion which is optionally enteric coated, and one or more buffering agents.

Another embodiment of the disclosure provides for the addition of an H2-receptor antagonist ("H2 blocker") with at least one proton pump inhibitor, with or without a buffering agent.

Embodiments of this disclosure also provide for a multi-chambered apparatus comprising (a) multiple chambers wherein one chamber comprises a pharmaceutically active agent, an optional protective buffer and/or an optional thickener, a second chamber comprises one or more flavoring agents, sweeteners, disintegrants, additives, buffering agents, or thickeners, and the formulations in the chambers can be in the form of a suspension, liquid, reagent, solution, or dry form constituted with water or other suitable liquid; (b) a dispenser head with multiple pumps that offer volumetric dispensing; and (c) connectors connecting the dispenser head to the chambers. In one embodiment, the outlet or outlets of the dispensing head can be connected to an oral syringe for delivering the combined output from the multiple chambers to a single oral syringe for administration.

In another embodiment, the apparatus comprises multiple chambers which are syringe bodies. These syringe bodies utilize syringe plungers that can be connected such that actuation of the syringes delivers the contents of the syringes in synchronous fashion. The connecting of the syringes is reversible, permitting the separate filling of each chamber as well as the rinsing and cleaning of the chambers for reuse. Furthermore, the syringe outlet or outlets can be connected to an oral syringe for delivering the combined output from the multiple chambers to a single oral syringe for administration.

Methods of using such compositions and apparatus for treating a patient in need of therapy for various diseases and disorders, including gastric acid related disorders such as, but not limited, to nocturnal acid breakthrough (NAB) and gastroparesis (slow stomach emptying), are also provided.

Other objects, features and advantages will be set forth in the Detailed Description that follows, and in part will be apparent from the description or may be learned by practice of the embodiments disclosed herein. These objects and advantages will be realized and attained by the processes and compositions particularly pointed out in the written description and claims hereof.

### DESCRIPTION OF THE DRAWINGS

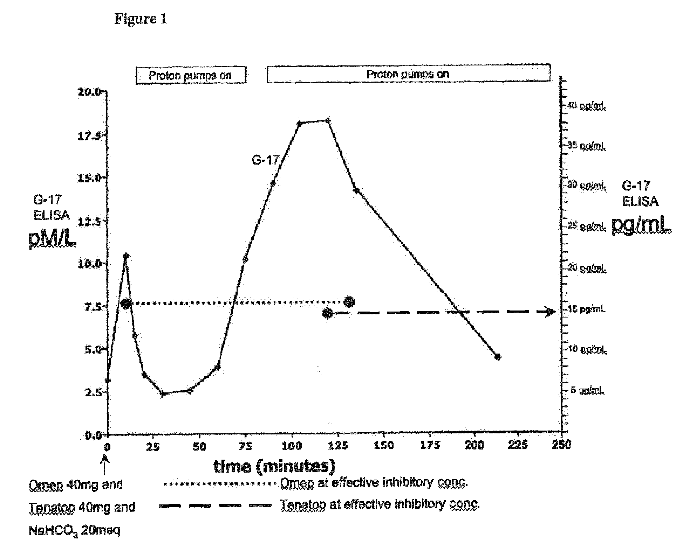

Figure 1 is a line graph illustrating that a substantial number of proton pumps can be blocked from the first dose of PPI (or other acid labile pharmaceutical agent). As indicated by the second proton pump activity curve, an additional number of proton pumps can be blocked if sufficient PPI (or other acid labile pharmaceutical agent) is present to correspond to a second stimulation of proton pumps. The use of Zegerid® (omeprazole with sodium bicarbonate), which contains a Gastrin stimulant (sodium bicarbonate) plus a delayed Gastrin stimulant (a PPI) produces two peaks of Gastrin (as measured by Gastrin 17). The second peak is a marker of proton pump activity that is not sufficiently inhibited by the administration of the omeprazole (since the omeprazole is not present in sufficient quantities in the parietal cell due to its short half-life). However, it is inhibited by the longer acting and later acting PPI (e.g., tenatoprazole or pantoprazole).

Figure 2 is a line graph illustrating that the use of a Gastrin stimulant such as coffee can produce a single spike in Gastrin, while the use of an acidic beverage (such as lemon juice and water) produces a decline in Gastrin. This figure illustrates the sensitive and immediate nature of the response of Gastrin (as measured by Gastrin 17) which occurs as the first step in the pathway for parietal cell activation and the stimulation of proton pumps.

Figure 3 is a line graph illustrating that first dose mean data based on results from a three subject cross-over study shows that tenatoprazole sodium (80 mg) and sodium bicarbonate (20 mEq) had a faster effect on gastric pH than tenatoprazole in free base form (80 mg) with sodium bicarbonate (20 mEq) for the first ten hours after dosing. In this study, sodium bicarbonate was used as a control. The dose was administered at 9:45 p.m.

Figure 4 is a line graph illustrating that, for the twenty-four hour period after dosing (without redosing), the mixture of tenatoprazole sodium (40 mg) and tenatoprazole base (40 mg) with sodium bicarbonate (20 mEq) controlled gastric pH better than either the salt form of tenatoprazole with sodium bicarbonate or the base form of tenatoprazole with sodium bicarbonate.

Figure 5 is an illustration of one example of a multi-chambered apparatus according to the present disclosure.

Figure 6 is an illustration of a multi-chambered apparatus connected to an optional syringe for oral administration according to the present disclosure.

Figure 7 is an illustration of a different dual chambered apparatus according to the present disclosure.

Figure 8 is an illustration of a multi-chambered apparatus according to the present disclosure comprising dual chambers connected to an optional syringe for oral administration.

Figure 9 is an illustration of a dispenser head of a multi-chambered apparatus according to the present disclosure.

Figure 10 is an illustration of an optional and removable attachment for the head of a multi-chambered apparatus according to the present disclosure.

Figure 11 is a line graph illustrating the concentration of omeprazole versus time in a solution of 120 mg omeprazole + 200 mg cimetidine + 1680 mg sodium bicarbonate.

Figure 12 is a line graph indicating the serum concentration profile after the subject received 40 mg omeprazole immediate release (Zegerid^{®}). The half-life was about 0.9 hours.

Figure 13 is a line graph from W.B. Im, 260 J Biol. Chem. 4591-4597 (1985). The data from this study demonstrates that when rats were pretreated with cimetidine a much reduced effect was seen as compared to control (no pretreatment) or a secretogue (carbachol) which increased the effect of omeprazole on H⁺, K⁺-ATPase activity. This study represents the conventional belief that there is no difference on acid output between a placebo and a composition comprising an H2 blocker and a PPI, such as cimetidine and omeprazole.

Figure 14 is a table of data from De Graef, J et al., 91 Gastroenterology 333-337 (1986). The study by De Graef *et al.* demonstrates the conventional belief that there is no difference on acid output between a placebo and a composition comprising an H2 blocker and a PPI, such as cimetidine and omeprazole.

Figure 15 is a line graph from De Graef, J et al., 91 Gastroenterology 333-337 (1986). The study by De Graef *et al.* demonstrates the conventional belief that there is no difference on acid output between a placebo and a composition comprising an H2 blocker and a PPI, such as cimetidine and omeprazole.

Figure 16 shows plasma omeprazole concentration after administration of a composition of Example 1 to a 545 kg horse.

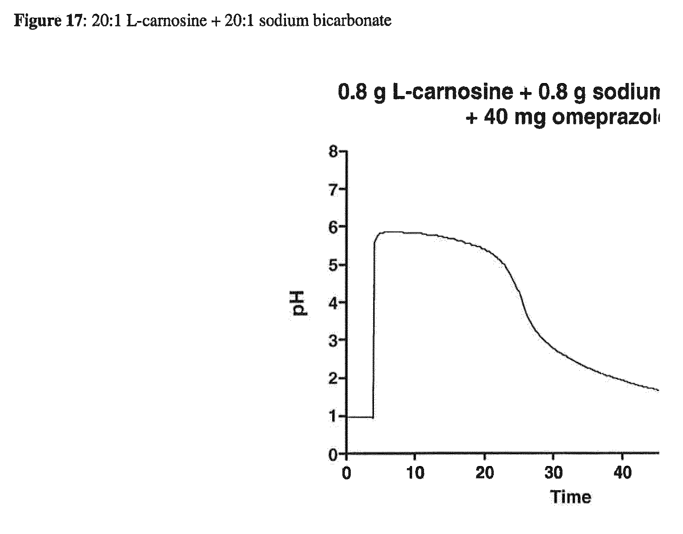

Figure 17 is a line graph illustrating the affect on pH of 800 mg of L-camosine, 800 mg sodium bicarbonate and 40 mg omeprazole (20:1 ratio of L-carnosine to omeprazole and sodium bicarbonate to omeprazole).

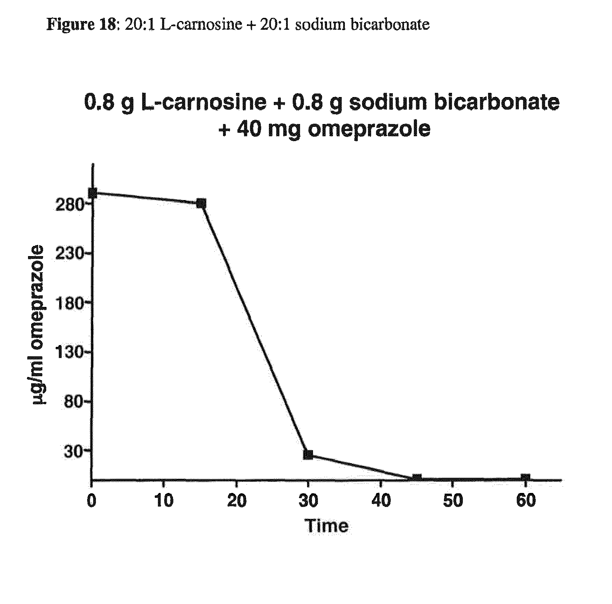

Figure 18 is a line graph illustrating the degradation of 40 mg omeprazole when it is administered with 800 mg of L-carnosine and 800 mg sodium bicarbonate (20:1 ratio of L-carnosine to omeprazole and sodium bicarbonate to omeprazole).

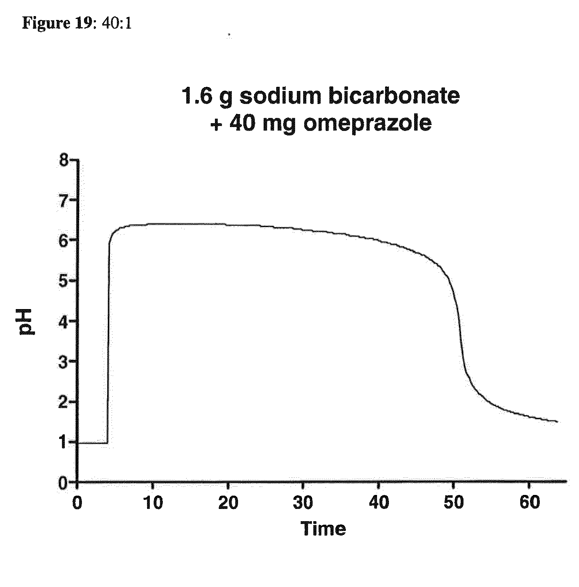

Figure 19 is a line graph illustrating the effect on pH of 1600 mg sodium bicarbonate and 40 mg omeprazole (40:1 ratio).

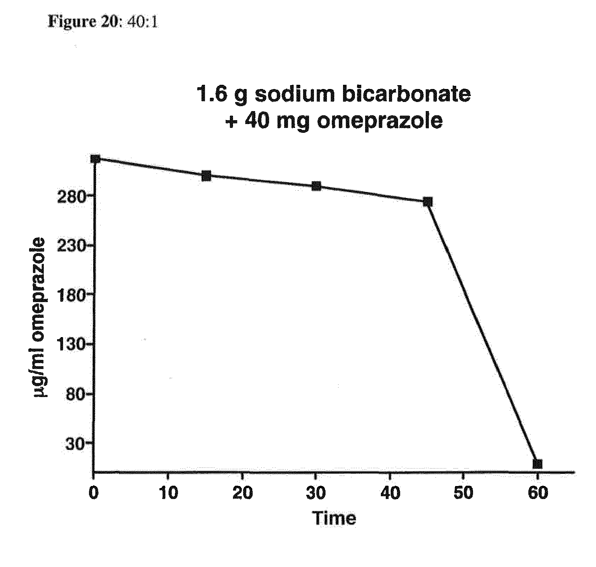

Figure 20 is a line graph illustrating the degradation of 40 mg omeprazole when it is administered with 1600 mg sodium bicarbonate (40:1 ratio).

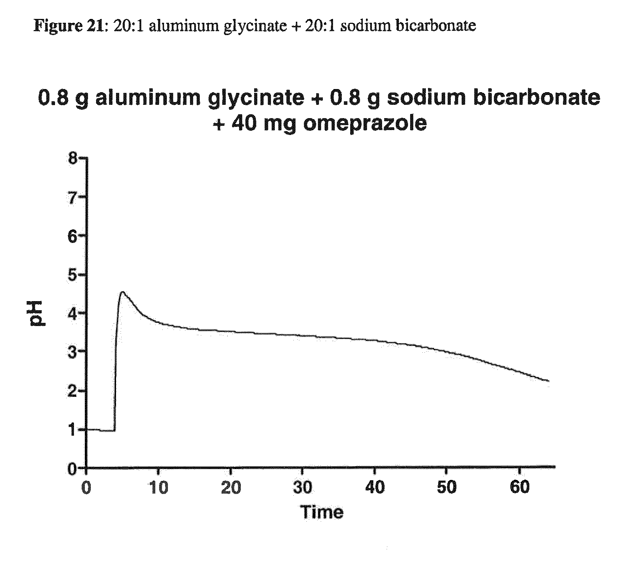

Figure 21 is a line graph illustrating the affect on pH of 800 mg of aluminum glycinate, 800 mg sodium bicarbonate and 40 mg omeprazole (20:1 ratio of aluminum glycinate to omeprazole and sodium bicarbonate to omeprazole).

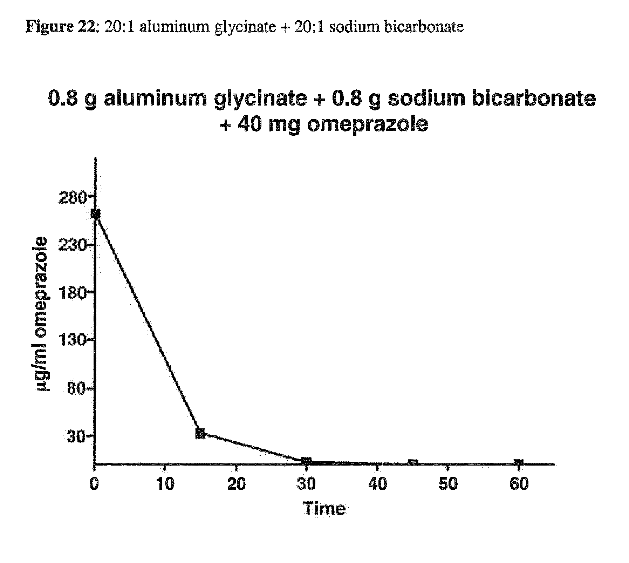

Figure 22 is a line graph illustrating the degradation of 40 mg omeprazole when it is administered with 800 mg of aluminum glycinate and 800 mg sodium bicarbonate (20:1 ratio of aluminum glycinate to omeprazole and sodium bicarbonate to omeprazole).

### DETAILED DESCRIPTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any way. Embodiments illustrated under any heading may be combined with embodiments illustrated under any other heading.

It has been discovered that a pharmaceutical composition comprising various combinations of acid-labile drugs, such as proton pump inhibitors, combinations of the salt form and the base form of the same proton pump inhibitor, or combinations of the salt form and the base form of two different proton pump inhibitors, can provide superior gastric acid inhibition than the use of a single type of proton pump inhibitor, with or without a buffer.

It is therefore provided herein a pharmaceutical composition comprising:
a) a first proton pump inhibitor (PPI1), having a therapeutically effective portion which is optionally enteric coated;
b) a second proton pump inhibitor (PPI2), having a therapeutically effective portion which is optionally enteric coated;
c) one or more buffering agents; and
d) optionally one or more of the following: L-carnosine, H2 blockers, NSAIDs, anti-H. pylori active substances, protein components, motility agents, iron, Vitamin B₁₂, pentagastrin or other pharmaceutical agents.

It is also provided herein a pharmaceutical composition comprising:
a) tenatoprazole, having a therapeutically effective portion which is optionally enteric coated;
b) one or more buffering agents; and
c) optionally one or more of the following: L-camosine, H2 blockers, NSAIDs, anti-H. pylori active substances, protein components, motility agents, iron, Vitamin B₁₂, pentagastrin or other pharmaceutical agents.

Provided herein is yet another pharmaceutical composition comprising:
a) a proton pump inhibitor in salt form, having a therapeutically effective portion which is optionally enteric coated;
b) a proton pump inhibitor in free base form, having a therapeutically effective portion which is optionally enteric coated;
c) one or more buffering agents; and
d) optionally one or more of the following: L-camosine, H2 blockers, NSAIDs, anti-H. pylori active substances, protein components, motility agents, iron, Vitamin B₁₂, pentagastrin or other pharmaceutical agents.

It has also been discovered that that the addition of L-carnosine to a long-acting and short-acting proton pump inhibitor can produce long lasting effects on gastric acid control starting with the first dosage.

It is therefore provided herein a pharmaceutical composition comprising:
a) pantoprazole;
b) lansoprazole;
c) L-carnosine; and
d) optionally one or more of the following: buffering agents, NSAIDs, anti-H. pylori active substances, protein components, motility agents, iron, Vitamin B₁₂, pentagastrin or other pharmaceutical agents.

It is also therefore provided herein a pharmaceutical composition comprising:
a) tenatoprazole;
b) pantoprazole;
c) L-carnosine; and
d) optionally one or more of the following: buffering agents, NSAIDs, anti-H. pylori active substances, protein components, motility agents, iron, Vitamin B₁₂, pentagastrin or other pharmaceutical agents.

It has further been discovered that that the addition of an H2-receptor antagonist ("H2 blocker") with at least one proton pump inhibitor can produce long lasting effects on gastric acid control starting with the first dosage, with or without a buffering agent.

It is therefore provided herein a pharmaceutical composition comprising:
a) at least one proton pump inhibitor (PPI), having a therapeutically effective portion which is optionally enteric coated;
b) an H2 blocker; and
d) optionally one or more of the following: buffering agents, L-camosine, H2 blockers, NSAIDs, anti-H. pylori active substances, protein components, motility agents, iron, Vitamin B₁₂, pentagastrin or other pharmaceutical agents.

It has also been discovered that it is possible to administer an acid labile pharmaceutical agent while maintaining its stability by utilizing a multidose method of administration. Such a method minimizes the interference of additives, excipients, flavoring agents, disintegrants, thickeners, buffering agents, suspensions, liquids, reagents or solutions with the pharmaceutical agents. Therefore, provided herein is a multi-chambered apparatus and its method of use.

Methods of using such compositions and apparatus for treating a patient, including patients with or without gastroparesis (slow stomach emptying), in need of therapy for various diseases and disorders, including gastric acid related disorders such as, but not limited, to nocturnal acid breakthrough (NAB), duodenal ulcer, gastric ulcer, stress erosions and ulceration, stress-related mucosal damage, gastric and duodenal erosions and ulceration, acid dyspepsia, gastroesophageal reflux disease (GERD), severe erosive esophagitis, poorly responsive symptomatic gastroesophageal reflux disease, acid reflux, heartburn, nighttime heartburn symptoms, esophageal ulcers and erosions, Barrett's esophagus, precancerous and cancerous lesions of the esophagus induced by acid exposure, acid hypersecretory conditions, gastrointestinal pathological hypersecretory conditions (such as Zollinger Ellison Syndrome), gastrointestinal bleeding, acute upper gastrointestinal bleeding, non-ulcer dyspepsia, heartburn, ulcers induced by NSAIDs, atypical reflux conditions, laryngitis, chronic cough, otitis media, sinusitis, eye pain, globus sensation, esophagitis, erosive esophagitis, adenocarcinoma of the esophagus, gastrinoma, *Helicobacter pylori* (*H*. *pylori*) infection, irritable bowel syndrome (IBS), inflammatory bowel disease (IBD), ulcerative colitis, pre- and post-operative acid aspiration, Crohn's disease, asthma, laryngitis, sleep apnea, sleep disturbance, psoriasis, intensive care therapy, and diseases related to any of the above-mentioned conditions are also provided.

### Proton Pump Inhibitors

Compositions of the disclosure comprise at least one pharmaceutically acceptable acid labile pharmaceutical agent. For example, embodiments disclosed herein comprise at least one H⁺, K⁺-ATPase proton pump inhibitor (PPI). The term proton pump inhibitor or PPI means any acid labile pharmaceutical agent possessing pharmacological activity as an inhibitor of H⁺, K⁺-ATPase. Classes of PPIs include but are not limited to: substituted aryl-imidazoles, substituted bicyclic aryl-imidazoles, substituted benzimidazole compounds, and substituted imidazopyridines.

A PPI can, if desired, be in any form such as a free base, free acid, salt, ester, hydrate, anhydrate, salt hydrate, amide, enantiomer, isomer, tautomer, prodrug, polymorph, derivative, or the like, provided that the free base, free acid, salt, ester, hydrate, anhydrate, salt hydrate, amide, enantiomer, isomer, tautomer, prodrug, polymorph, or any other pharmacologically suitable derivative is therapeutically active or undergoes conversion within or outside of the body to a therapeutically active form.

In one embodiment, illustrative PPIs are those compounds of Formula (A): 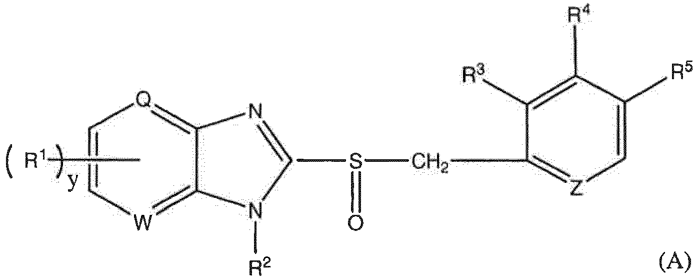 wherein

R¹ is hydrogen, alkyl, halogen, cyano, carboxy, carboalkoxy, carboalkoxyalkyl, carbamoyl, carbamoylalkyl, hydroxy, alkoxy which is optionally fluorinated, hydroxyalkyl, trifluoromethyl, acyl, carbamoyloxy, nitro, acyloxy, aryl, aryloxy, alkylthio, or alkylsulfinyl;

R² is hydrogen, alkyl, acyl, acyloxy, alkoxy, amino, aralkyl, carboalkoxy, carbamoyl, alkylcarbamoyl, dialkylcarbamoyl, alkylcarbonylmethyl, alkoxycarbonylmethyl, or alkylsulfonyl;

R³ and R⁵ are the same or different and each is hydrogen, alkyl, C₁₋₄ lower alkyl (*e.g*. methyl, ethyl, *etc*.), alkoxy, amino, or alkoxyalkoxy;

R⁴ is hydrogen, alkyl, C₁₋₄ lower alkyl (*e.g*. methyl, ethyl, *etc*.), alkoxy which may optionally be fluorinated, or alkoxyalkoxy;

is nitrogen, CH, or CR¹;

W is nitrogen, CH, or CR¹;

y is an integer of 0 through 4; and

Z is nitrogen, CH, or CR¹;

or a free base, salt, ester, hydrate, salt hydrate, amide, enantiomer, isomer, tautomer, prodrug, polymorph, or derivative thereof.

One specific example of a PPI is tenatoprazole (TU-199, also called benatoprazole), or 5-methoxy-2-[[(4-methoxy-3,5-dimethyl-2-pyridyl)methyl]sulfinyl]imidazo[4,5-b]pyridine, described in EP 0254588, hereby incorporated by reference herein in its entirety. An alternative IUPAC name for tenatoprazole is 3-methoxy-8-[(4-methoxy-3,5-dimethyl-pyridin-2-yl)methyl sulfinyl]-2,7,9-triazabicyclo[4.3.0]nona-2,4,8,10-tetraene. Because of its relatively long elimination profile, tenatoprazole can be used for the treatment of conditions such as gastroesophageal reflux disease, gastrointestinal bleeding and dyspepsia, as described in the French patent application 0213113, hereby incorporated by reference herein in its entirety. Tenatoprazole is a proton pump inhibitor which is similar to the chemical structure of omeprazole (Merck Index No. 6913; CAS No. 73590-58-6), or 5-methoxy-2-[[(4-methoxy-3, 5-dimethyl-2-pyridinyl) methyl] sulfinyl]-1H benzimidazole. Omeprazole is a widely used proton pump inhibitor of the class of substituted benzimidazoles. Tenatoprazole belongs to the class of substituted imidazopyridines and has an imidazo[4,5-b]pyridine moiety whereas omeprazole has a benzimidazole moiety.

Specific examples of suitable PPIs include esomeprazole (also referred to as S-omeprazole), ilaprazole (U.S. Pat. No. 5,703,097), tenatoprazole (or benatoprazole), omeprazole, lansoprazole, s-lansoprazole, rabeprazole, hydroxyomeprazole, pantoprazole, pariprazole, leminoprazole, dontoprazole, habeprazole, perprazole, ransoprazole, and nepaprazole, or a free base, a free acid, a salt, hydrate, ester, salt hydrate, amide, enantiomer, isomer, tautomer, polymorph, prodrug, or derivative of such compounds.

Other acid labile pharmaceutical agents include, but are not limited to: soraprazan (Altana); AZD-0865 (AstraZeneca); YH-1885 (PCT Publication WO 96/05177) (SB-641257) (2-pyrimidinamine, 4-(3,4-dihydro-1-methyl-2(1H)-isoquinolinyl)-N-(4-fluorophenyl)-5,6-dimethyl-monohydrochloride) (YuHan); BY-112 (Altana); SPI-447 (Imidazo(1,2-a)thieno(3,2-c)pyridin-3-amine,5-methyl-2-(2-methyl-3-thieny-1) (Shinnippon); 3-hydroxymethyl-2methyl-9-phenyl-7H-8,9-dihydro-pyrano(2,- 3-c)-imidazo(1,2-a)pyridine (PCT Publication WO 95/27714) (AstraZeneca); Pharmaprojects No. 4950 (3-hydroxymethyl-2-methyl-9-phenyl-7H-8,9-dihydro- -pyrano(2,3-c)-imidazo(1,2-a)pyridine) (AstraZeneca, ceased) WO 95/27714; Pharmaprojects No. 4891 (EP 700899) (Aventis); Pharmaprojects No. 4697 (PCT Publication WO 95/32959) (AstraZeneca); H-335/25 (AstraZeneca); T-330 (Saitama 335) (Pharmacological Research Lab); Pharmaprojects No. 3177 (Roche); BY-574 (Altana); Pharmaprojects No. 2870 (Pfizer); AU-1421 (EP 264883) (Merck); AU-2064 (Merck); AY-28200 (Wyeth); Pharmaprojects No. 2126 (Aventis); WY-26769 (Wyeth); pumaprazole (PCT Publication WO 96/05199) (Altana); YH-1238 (YuHan); Pharmaprojects No. 5648 (PCT Publication WO 97/32854) (Dainippon); BY-686 (Altana); YM-020 (Yamanouchi); GYKI-34655 (Ivax); FPL-65372 (Aventis); Pharmaprojects No. 3264 (EP 509974) (AstraZeneca); nepaprazole (To a Eiyo); HN-11203 (Nycomed Pharma); OPC-22575; pumilacidin A (BMS); saviprazole (EP 234485) (Aventis); SKand F-95601 (GSK, discontinued); Pharmaprojects No. 2522 (EP 204215) (Pfizer); S-3337 (Aventis); RS-13232A (Roche); AU-1363 (Merck); SKand F-96067 (EP 259174) (Altana); SUN 8176 (Daiichi Phama); Ro-18-5362 (Roche); ufiprazole (EP 74341) (AstraZeneca); and Bay-p-1455 (Bayer); or a free base, free acid, salt, hydrate, ester, salt hydrate, amide, enantiomer, isomer, tautomer, polymorph, prodrug, or derivative of such compounds.

Still other embodiments contemplated by the present disclosure include, but are not limited to those described in the following U.S. Pat. Nos.: 4,628,098; 4,689,333; 4,786,505; 4,853,230; 4,965,269; 5,021,433; 5,026,560; 5,045,321; 5,093,132; 5,430,042; 5,433,959; 5,576,025; 5,639,478; 5,703,110; 5,705,517; 5,708,017; 5,731,006; 5,824,339; 5,840,737; 5,855,914; 5,879,708; 5,948,773; 6,017,560; 6,123,962; 6,187,340; 6,296,875; 6,319,904; 6,328,994; 4,255,431; 4,508,905; 4,636,499; 4,738,974; 5,690,960; 5,714,504; 5,753,265; 5,817,338; 6,093,734; 6,013,281; 6,136,344; 6,183,776; 6,328,994; 6,479,075; 6,489,346; 6,559,167; 6,645,988; 6,699,885; 7,101,573; 7,109,161.

Still other embodiments contemplated by the present disclosure include, but are not limited to those described in the following: EP 0254588; EP 0005129.

Other embodiments contemplated by the present disclosure include, but are not limited to those described in the following PCT Publications: WO 94/27988; WO 05/044223; WO 06/043280.

Still other embodiments contemplated by the present disclosure include, but are not limited to those described in the following U.S. Application Nos.: 20020192299;20040131675;20040146554;20040248939;20040248942; 20050003005;20050031700;20050037070;20050054682;20050112193; 20050220870;20050222210;20050239845;20050244517;20050249806; 20050249811;20050266071;20050288334;20050277672;20050277673; 20050277671;20060024238;20060134210;20060147522;20060159760; 20060167262; 20060173045; 20060204585.

The foregoing lists of suitable acid inhibitors are meant to be illustrative and not exhaustive as a person of ordinary skill in the art would recognize that there are many other suitable acid inhibitors which could be created.

### Polymeric Benzimidazoles

Compositions of the present invention comprise a polymeric benzimidazole, also referred to herein as "polymer-based benzimidazoles", or "polymerized benzimidazoles". The term "polymeric benzimidazole" and the interchangeable terms listed immediately above refer to a compound comprising a benzimidazole moiety, wherein the compound is bonded or linked to a least one pharmaceutically acceptable polymer. The term "benzimidazole moiety" refers to the benzimidazole moiety of formulas (II) and (III) as shown herein below, not including any pharmaceutically acceptable polymer substituted at one of the nitrogens of the pentameric ring of the benzimidazole ring.

Benzimidazole moiety-containing compounds or drugs useful in the present invention include any pharmacologically active benzimidazole compounds that are susceptible to acid catalyzed degradation and that can be substituted at the N position of the benzimidazole ring. The term "pharmacologically active benzimidazole compound" includes any such compound or drug which is capable of producing a pharmacological response in a subject, irrespective of whether therapeutic, diagnostic, or prophylactic in nature.

While not wishing to be bound by any one theory, the acidic decomposition of an acid-labile benzimidazole compound is believed to be due to an acid catalyzed reaction as described by, for example, G. Rackur et al., in Biochem. Biophys. Res. Commun. 1985: 128(1). p. 477-484. Thus, pharmacologically active agents useful in the present invention are those which are degraded by acids, even organic acids, or are degraded in acid catalyzed reactions.

A particularly preferred class of benzimidazole moiety-containing acid-labile compounds suitable for polymeric N-substitution and preparation of polymeric benzimidazoles useful in the present invention include substituted benzimidazole compounds possessing pharmacological activity as an inhibitor of H⁺, K⁺-ATPase (*i.e*. proton pump inhibitors). Such proton pump inhibitors can, if desired, be in the form of a free base, a free acid, a salt, an ester, a hydrate, an amide, an enantiomer, an isomer, a tautomer, a prodrug, a polymorph, a derivative or the like, and can be in racemic or enantiomeric form.

Polymeric benzimidazoles suitable for use in the present invention can be prepared by any process known in the art. For example, otherwise acid-labile benzimidazole compounds can be used as starting agents for formation of polymeric benzimidazoles by polymeric N-substitution as described in U.S. Patent No. 6,617,338, which is hereby incorporated by reference in its entirety. Without being be bound by theory, it is believed that upon oral administration of these polymeric N-substituted benzimidazoles with a buffering agent or protein component as descirbed herein, enzymes and chemicals in the gastrointestinal fluid cleave at the hydrolysable group (*e.g*. (E) of formula (I)) to release an N-substituted benzimidazole derivative that may or may not be the original starting benzimidazole moiety (*e.g*. moiety B below) having a portion of the polymer remaining attached thereto. Without being held to a particular theory, it is also presently believed that the N-substituted benzimidazole derivative, in the presence of the buffering agent or protein component, maintains its pharmacological activity as an inhibitor of H⁺, K⁺-ATPase, while the cleaved portion of the polymer becomes an inert, non-toxic and/or non-absorbable metabolite that is excreted from the body.

In one embodiment, suitable polymeric benzimidazoles are those compounds of formula (I): 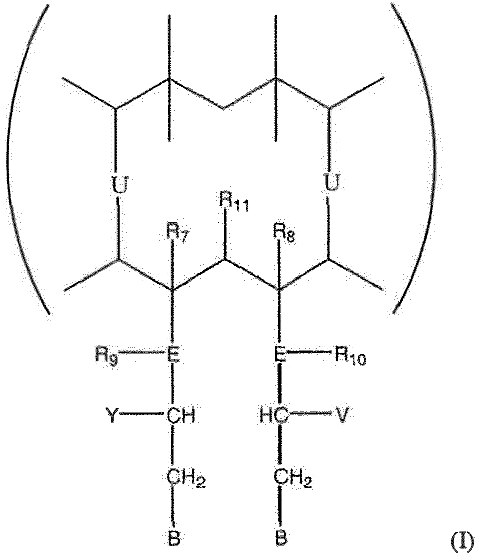
wherein R₇, R₈, R₉, and R₁₀, are independently H or a lower alkyl (preferably methyl or ethyl), for example a C₁₋₄ alkyl;
U is

   -OCOCH₂COO-,

   -CONHCH₂NHCO-, or 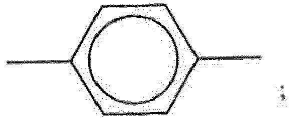
R₁₁ is H, CH₃, C₂H₅, or CONH₂;
Y and V are independently or NH₂;
E is -COO-; and
B is a benzimidazole moiety of formula (II): 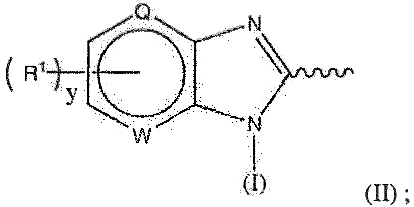
   wherein R¹ is hydrogen, alkyl, halogen, cyano, carboxy, carboalkoxy, carboalkoxyalkyl, carbamoyl, carbamoylalkyl, hydroxy, alkoxy which may optionally be fluorinated, hydroxyalkyl, trifluoromethyl, acyl, carbamoyloxy, nitro, acyloxy, aryl, aryloxy, alkylthio, or alkylsulfinyl;
   Q is nitrogen, CH, or CR¹;
   W is nitrogen, CH, or CR¹; and
   y is an integer of 0 through 4;
   or a salt, an ester, a hydrate, an amide, an enantiomer, an isomer, a tautomer, a polymorph, a polymorph, a prodrug, or a derivative thereof.
In another embodiment, (B) is a benzimidazole moiety of formula (III): 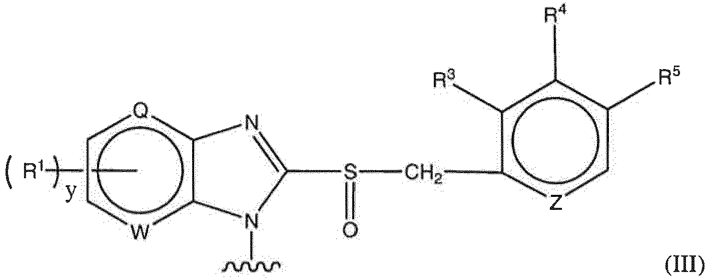 wherein:
R¹ is hydrogen, alkyl, halogen, cyano, carboxy, carboalkoxy, carboalkoxyalkyl, carbamoyl, carbamoylalkyl, hydroxy, alkoxy which may optionally be fluorinated, hydroxyalkyl, trifluoromethyl, acyl, carbamoyloxy, nitro, acyloxy, aryl, aryloxy, alkylthio, or alkylsulfinyl;
R³ and R⁵ are independently hydrogen, alkyl, alkoxy, amino, or alkoxyalkoxy;
R⁴ is hydrogen, alkyl, alkoxy which may optionally be fluorinated, or alkoxyalkoxy;
Q is nitrogen, CH, or CR¹;
W is nitrogen, CH, or CR¹;
y is an integer of 0 through 4; and
Z is nitrogen, CH, or CR¹;
or a salt, an ester, a hydrate, an amide, an enantiomer, an isomer, a tautomer, a polymorph, a prodrug, or a derivative thereof.

Specific examples of suitable PPIs for use as the benzimidazole moiety (B) include esomeprazole (also referred to as S-omeprazole), ilaprazole (U.S. Pat. No. 5,703,097), lansoprazole, omeprazole, pantoprazole, pariprazole, rabeprazole, tenatoprazole, and nepaprazole or a free base, a free acid, or a salt, hydrate, ester, amide, enantiomer, isomer, tautomer, polymorph, prodrug, or derivative of such compounds.

In one embodiment, the present disclosure provides pharmaceutical compositions comprising at least one polymerized benzimidazole, for example a polymerized proton pump inhibitor, one or more buffering agents and one or more optional pharmaceutical excipients. Such compositions can further comprise a protein component.

In another embodiment, the present disclosure provides pharmaceutical compositions comprising at least one polymerized benzimidazole, for example a PPI, a protein component and one or more optional pharmaceutical excipients. Such compositions can further comprise a buffering agent.

In yet another embodiment, the present disclosure provides an orally deliverable pharmaceutical composition comprising a polymerized PPI and one or more buffering agents (and/or a protein component) wherein upon oral administration of the composition to a plurality of fasted adult human subjects, the subjects exhibit an average plasma concentration of the PPI moiety of at least about 0.1 mg/ml at any time within about 30 minutes after administration.

Gastric acid inhibitors, including proton pump inhibitors as well as their salts, hydrates, esters, salt hydrates, amides, enantiomers, isomers, tautomers, polymorphs, prodrugs, and derivatives may be prepared using standard procedures that a person of ordinary skill in the art of synthetic organic chemistry would recognize. See, *e.g.,* March, Advanced Organic Chemistry: Reactions, Mechanisms and Structure, 4th Ed. (New York: Wiley-Interscience, 1992); Leonard et al., Advanced Practical Organic Chemistry (1992); Howarth et al., Core Organic Chemistry (1998); and Weisermel et al., Industrial Organic Chemistry (2002).

"Pharmaceutically acceptable salts," or "salts," include the salt of a proton pump inhibitor prepared from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic, methanesulfonic, ethanesulfonic, benzenesulfonic, pantothenic, toluenesulfonic, 2-hydroxyethanesulfonic, sulfanilic, cyclohexylaminosulfonic, algenic, beta-hydroxybutyric, galactaric and galacturonic acids.

In one embodiment, acid addition salts are prepared from the free base forms using, for example, methodologies involving reaction of the free base with a suitable acid. Suitable acids for preparing acid addition salts include both organic acids, *e.g*., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, *e.g.*, hydrochloric acid, hydrobromic acid, sulfuric acid, nitric acid, phosphoric acid, and the like.

In other embodiments, an acid addition salt is reconverted to the free base by treatment with a suitable base. In a further embodiment, the acid addition salts of the proton pump inhibitors are halide salts, which are prepared, for example, using hydrochloric or hydrobromic acids. In still other embodiments, the basic salts are alkali metal salts, *e.g*., sodium salt.

Salt forms of proton pump inhibitors include, but are not limited to: a sodium salt form such as esomeprazole sodium, omeprazole sodium, tenatoprazole sodium, rabeprazole sodium, pantoprazole sodium; a magnesium salt form such as esomeprazole magnesium or omeprazole magnesium, described in U.S. Pat. No. 5,900,424; a calcium salt form; a potassium salt form such as the potassium salt of esomeprazole, described in U.S. Pat. No. 6,511,996; salt hydrate forms including but not limited to sodium hydrate salt forms, for example tenatoprazole sodium hydrate or omeprazole sodium hydrate. Other salts of esomeprazole are described in U.S. Pat. Nos.: 4,738,974 and 6,369,085. Salt forms of pantoprazole and lansoprazole are discussed in U.S. Pat. Nos. 4,758,579 and 4,628,098, respectively.

The foregoing list of suitable salts of proton pump inhibitors is meant to be illustrative and not exhaustive as a person of ordinary skill in the art would recognize that other pharmaceutically acceptable salts of a proton pump inhibitor could be created.

In one embodiment, preparation of esters involves functionalizing hydroxyl and/or carboxyl groups that may be present within the molecular structure of the drug. In another embodiment, the esters are acyl-substituted derivatives of free alcohol groups, *e.g*., moieties derived from carboxylic acids of the formula RCOOR₁ where R₁ is a lower alkyl group. Esters can be reconverted to the free acids, if desired, by using procedures including but not limited to hydrogenolysis or hydrolysis.

"Amides" may be prepared using techniques known to those skilled in the art or described in the pertinent literature. For example, amides may be prepared from esters, using suitable amine reactants, or they may be prepared from an anhydride or an acid chloride by reaction with an amine group such as ammonia or a lower alkyl amine.

"Tautomers" of substituted bicyclic aryl-imidazoles include, *e.g*., tautomers of omeprazole such as those described in U.S. Pat. Nos. 6,262,085; 6,262,086; 6,268,385; 6,312,723; 6,316,020; 6,326,384; 6,369,087; and 6,444,689.

An exemplary "isomer" of a substituted bicyclic aryl-imidazole is the isomer of omeprazole including but not limited to isomers described in: Oishi et al., Acta Cryst. (1989), C45, 1921-1923; U.S. Pat. No. 6,150,380; U.S. Patent Publication No. 02/0156284; and PCT Publication No. WO 02/085889.

Exemplary "polymorphs" include, but are not limited to, those described in PCT Publication No. WO 92/08716, and U.S. Pat. Nos. 4,045,563; 4,182,766; 4,508,905; 4,628,098; 4,636,499; 4,689,333; 4,758,579; 4,783,974; 4,786,505; 4,808,596; 4,853,230; 5,026,560; 5,013,743; 5,035,899; 5,045,321; 5,045,552; 5,093,132; 5,093,342; 5,433,959; 5,464,632; 5,536,735; 5,576,025; 5,599,794; 5,629,305; 5,639,478; 5,690,960; 5,703,110; 5,705,517; 5,714,504; 5,731,006; 5,879,708; 5,900,424; 5,948,773; 5,997,903; 6,017,560; 6,123,962; 6,147,103; 6,150,380; 6,166,213; 6,191,148; 5,187,340; 6,268,385; 6,262,086; 6,262,085; 6,296,875; 6,316,020; 6,328,994; 6,326,384; 6,369,085; 6,369,087; 6,380,234; 6,428,810; 6,444,689; and 6,462,0577.

In one embodiment, at least one proton pump inhibitor is not enteric coated. In another embodiment, a portion of at least one proton pump inhibitor is optionally enteric coated. In another embodiment, a therapeutically effective portion of at least one proton pump inhibitor is optionally enteric coated. In another embodiment, about 5%, about 15%, about 20%, about 30%, about 40%, about 50% or about 60% of at least one proton pump inhibitor is optionally enteric coated. In another embodiment, a portion of at least one proton pump inhibitor comprises a "thin enteric coat." The term "thin enteric coat" herein refers to a pH sensitive coating that is applied in a manner or amount such that it delays release of the coated substance in gastrointestinal fluid for a period of time, but ultimately allows release of some of the coated substance prior to passage into the duodenum.

In one embodiment, at least one proton pump inhibitor has a D₉₀, D₈₀, D₇₀ or D₅₀ particle size, by weight or by number, of less than about 900 µm, less than about 800 µm, less than about 700 µm, less than about 600 µm, less than about 500 µm, less than about 400 µm, less than about 300 µm, less than about 200 µm, less than about 150 µm, less than about 100 µm, less than about 80 µm, less than about 60 µm, less than about 40 µm, less than about 35 µm, less than about 30 µm, less than about 25 µm, less than about 20 µm, less than about 15 µm, less than about 10 µm, or less than about 5 µm.

another embodiment, compositions are provided wherein a micronized proton pump inhibitor is of a size which allows greater than about 90%, greater than about 75%, or greater than about 50% of the proton pump inhibitor to be released from the dosage unit within about 1 hour, within about 50 minutes, within about 40 minutes, within about 30 minutes, within about 20 minutes, within about 10 minutes, or within about 5 minutes after placement in a standard dissolution test.

In still another embodiment, compositions of the disclosure comprise two PPIs in a total amount of about 1 mg to about 3000 mg, about 1 mg to about 2000 mg, about 1 mg to about 1000 mg, about 1 mg to about 750 mg, about 1 mg to about 500 mg, about 1 mg to about 300 mg, about 5 mg to about 250 mg, about 5 mg to about 200 mg, about 5 mg to about 175 mg, about 5 mg to about 120 mg, about 5 mg to about 100 mg, about 5 mg to about 80 mg, or about 5 mg to about 50 mg, for example about 5 mg, about 7.5 mg, about 10 mg, about 15mg, about 20 mg, about 30 mg, about 40 mg, about 50 mg, about 60 mg, about 70 mg, or about 80 mg.

### H2 Blockers

In one embodiment, compositions of the disclosure comprise one or more H2 blockers. The term "H₂-receptor antagonist" or "H₂RA" or "H₂ antagonist" or "H₂ blocker" or "H2 blocker" refers to any pharmaceutically acceptable substance that blocks the action of histamine on parietal cells in the stomach, whether directly or indirectly. The class of H₂ blockers are competitive inhibitors of histamine at the parietal cell H₂ receptor. The class of H₂ blockers may suppress the secretion of acid by blocking histamine from binding on parietal cell H₂ receptor, preventing the subsequent stimulation of acid secretion. When the receptors are blocked, other substances that promote acid secretion, including but not limited to gastrin and acetylcholine, have a reduced effect on parietal cells. Exemplary H2 blockers include, but are not limited to, cimetidine, ranitidine, famotidine, nizatidine, burimamide, ebrotidine, pabutidine and lafutidine.

H2 blockers should be present in an amount of about 1 mg to about 1200 mg, such as about 1 mg to about 900 mg, about 1 mg to about 800 mg, about 1 mg to about 700 mg, about 1 mg to about 600 mg, about 1 mg to about 500 mg, about 1 mg to about 400 mg, about 1 mg to about 300 mg, about 1 mg to about 200 mg, about 20 mg to about 500 mg, 20 mg to about 500 mg, about 20 mg to about 400 mg, about 20 mg to about 300 mg, about 20 mg to about 200 mg, about 40 mg to about 500 mg, about 40 mg to about 400 mg, about 40 mg to about 300 mg, about 40 mg to about 200 mg, about 60 mg to about 500 mg, about 60 mg to about 500 mg, about 60 mg to about 400 mg, about 60 mg to about 300 mg, about 60 mg to about 200 mg, for example about 5 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, or about 300 mg.

### Anti-Helicobacter pylori Active Substance

Helicobacter pylori (*H. pylori*) is a spiral-shaped bacterium that is found in the gastric mucous layer or adherent to the epithelial lining of the stomach. Several studies have linked *H. pylori* infection to gastrointestinal disease in humans. Specifically, *H. pylori* is the most common cause of gastritis in humans, and greater than 90% of duodenal ulcer patients are infected with *H. pylori.*

Traditional therapy for *H. pylori* infection consists of 10 days to 2 weeks of one or two effective antibiotics, such as amoxicillin, tetracycline (not to be used for children <12 yrs.), metronidazole, or clarithromycin, plus ranitidine bismuth citrate, bismuth subsalicylate, or an enteric coated proton pump inhibitor (PPI). Because these regimens often require multiple dosing per day over a period of many days, patient compliance is a significant barrier to effective treatment. Furthermore, the treatments, even when complied with, are unsuccessful in many patients.

In one embodiment, compositions of the disclosure comprise one or more anti-*Helicobacter pylori* active substances. The term "anti-*Helicobacter pylori* active substance" or "anti-*H*. *pylori* active substance" or "anti-*H*. *pylori* agent" refers to any pharmaceutically acceptable substance that kills, reduces and/or inhibits or disrupts the growth of *H. pylori,* whether directly or indirectly. In one embodiment, the anti-*H*. *pylori* active substance is an antibiotic. The term "antibiotic" herein refers to any drug or agent, whether naturally occurring, synthetically or semi-synthetically derived, that kills or reduces the growth of bacterial infection. In another embodiment, the anti-*H*. *pylori* active substance is effective against gram negative bacteria. In yet another embodiment, the anti-*H*. *pylori* active substance is not an antibiotic. In another embodiment, the anti-*H*. *pylori* active substance is a mucolytic agent or an essential oil. In one embodiment, compositions of the disclosure comprise one or more various anti-*H*. *pylori* active substances, for example one or more of an antibiotic, an essential oil and/or a mucolytic agent.

In another embodiment, the present invention provides pharmaceutical compositions comprising a proton pump inhibitor, a buffering agent, and an anti-*H*. *pylori* active substance, for example an antibiotic. Optionally, at least a therapeutically effective amount or portion of the proton pump inhibitor is not enteric coated. Methods of using such compositions to treat various diseases and disorders are also provided as well as methods for manufacturing such compositions.

In yet another embodiment, the present invention provides a method for treating a subject in need of *H. pylori* treatment and/or treatment of an acid related gastrointestinal disease or disorder such as ulcer, comprising administering to the subject a composition comprising an anti-*Helicobacter pylori* active substance, a buffering agent, and a PPI. Optionally, at least a therapeutically effective portion of the proton pump inhibitor is not enteric coated. In a related embodiment, upon administration of the composition to the subject, at least a therapeutically effective amount of the proton pump inhibitor and/or anti-*H. pylori* active substance is available for absorption in the subject's stomach.

In still another embodiment, the invention provides a method of treating a human subject in need of *H. pylori* treatment and/or treatment of an acid related gastrointestinal disease or disorder, comprising administering to the subject one or more compositions comprising at least one of (or all three of): anti-*H*. *pylori* active substance, a buffering agent, and a PPI, wherein a portion of the PPI is optionally enteric coated or the entire amount of PPI is free of enteric coating. In this embodiment, the composition is designed such that a therapeutically effective amount of the anti-*H*. *pylori* active substance is available in the subject's stomach at a time when the subject's stomach secretions are at a pH of about 5 to about 8 or about 5.5 to about 7.5. Such a result can be accomplished in any suitable manner, for example by coordinating the timing of administration of the one or more compositions, or by providing a single composition that provides controlled release of the anti-*H*. *pylori* active substance, buffering agent and proton pump inhibitor such that a therapeutically effective amount of the anti-*H*. *pylori* active substance is available for absorption in the subject's stomach when the subject's stomach secretions are at the desired pH.

Exemplary antibiotics include, but are not limited to, antibiotic penicillins (*e.g.* amoxicillin, benzylpenicillin, piperacillin, mecillinam, *etc.),* antibiotic cefems or cephalosporins (*e.g*. cefixime, cefuroxime, cefuroxime axetil, cefaclor, ceftizoxime, cefotaxime, ceftazidime, *etc*.)*,* antibiotic macrolides (*e.g.* erythromycin, clarithromycin, azithromycin, telithromycin, roxithromycin, *etc*.), antibiotic tetracylines (*e.g.* tetracyline, minocycline, doxycycline, tigecycline, *etc.),* antibiotic aminoglycosides (*e.g*. gentamicin, kanamycin, netilmicin, amikacin, tobramycin, *etc.),* antibiotic carbapenems (*e.g*. imipenem, meropenem, doripenem, *etc.),* carbapenem ester type prodrug (*e.g.* tebipenem pivoxil, faropenem daloxate; other oral carbapenem prodrugs include GV-118819, CS-834, L-084, DZ-2649, CL-191121, *etc*.; see below), antibiotic quinolones (*e.g*. norfloxacin, ofloxacin, levofloxacin, ciprofloxacin, sitafloxacin, clinafloxacin, gatifloxacin, moxifloxacin, pazufloxacin, prulifloxacin, olamufloxacin, ganefloxacin, gemifloxacin, trovafloxacin, *etc.*)*,* antibiotic nitroimidazoles (*e.g*. metronidazole, tinidazole), antibiotic rifamycin or ansamycin analogues (*e.g*. rifabutin, rifampicin, rifampin, rifaximin, rifalazil, and ryfamycin derivatives such as 3'-hydroxy-5'-(4-propylpiperazinyl)benzoxazinorifamycin- (shown as formula (IV) below), which are illustratively disclosed in H. Saito et al., In vitro antimycobacterial activities of newly synthesized benzoxazinorifamycins, Antimicrob. Agents Chemother., 1991 March; 35(3): 542-547, the entire disclosure of which is hereby incorporated by reference herein, and other antibiotics such as mupirocin.

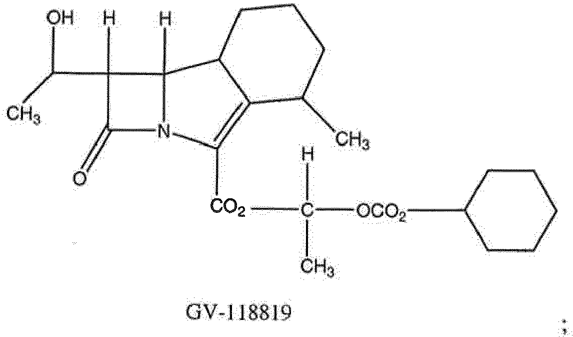

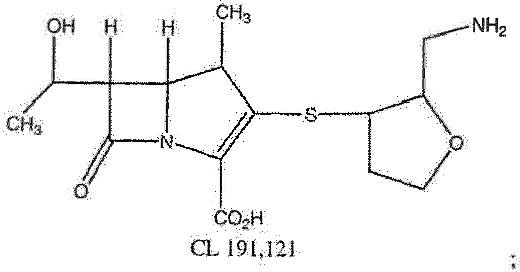

or

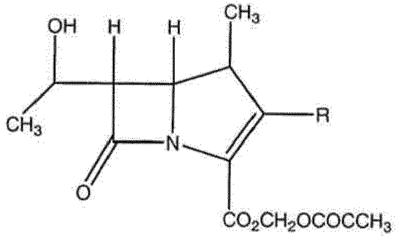

wherein R is:

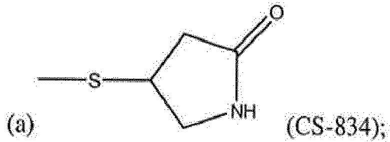

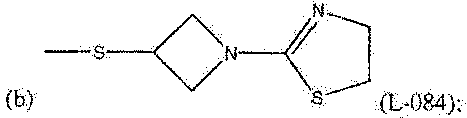

of

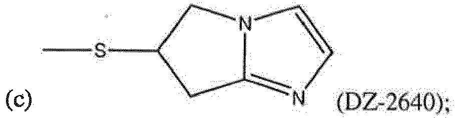

The compound 3'-hydroxy-5'-(4-propylpiperazinyl)benzoxazinorifamycin- has a chemical structure (IV) shown below.

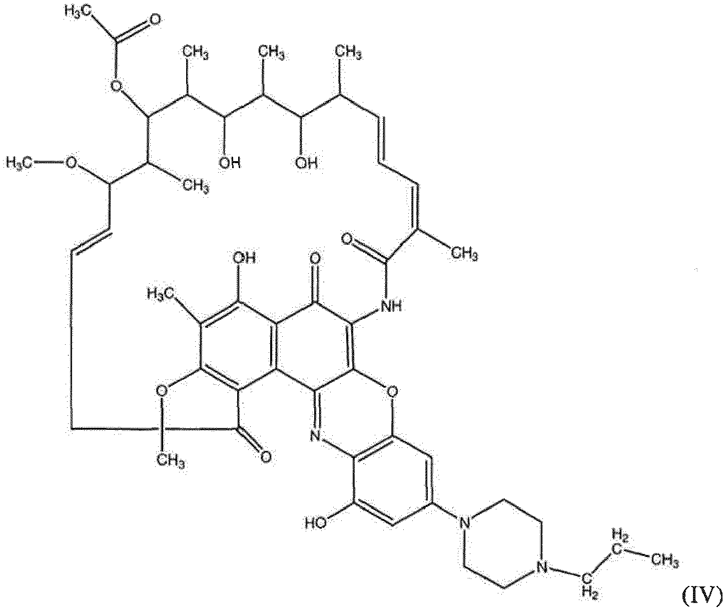

The above antibiotics are typically administered in daily amounts of about 250 mg to about 5 g. Compositions of the disclosure can comprise a full daily dose of anti-*H*. *pylori* active substance (e.g. 250 mg to about 5 g), or a fraction of the daily dose (*e.g.* 1/8^{th}, 1/6^{th}, 1/4^{th}, 1/3^{rd}, ½, *etc.*). Typical daily doses of antibiotics are as follows: furazolidone: about 400 mg (about 200 mg twice daily); tetracycline: about 250 to about 2000 mg (about 500 mg one to four times daily); amoxicillin: about 2000 mg (about 1000 mg twice daily); clarithromycin: about 1000 mg (about 500 mg twice daily); metronidazole: 800 mg (about 400 mg twice daily). Aminoglycosides such as gentamicin, tobramycin, amikacin, kanamycin, netilmicin, *etc.,* are typically administered in doses of about 25 mg to about 1 g about one to about four times per day. Quinolones such as norfloxacin, ofloxacin, levofloxacin, ciprofloxacin, gatifloxacin, moxifloxacin, trovafloxacin are typically administered in doses of about 50 mg to about 1 g about one to about four times per day.

Essential oils can also be used as an anti-*H*. *pylori* active substance according to various embodiments of the disclosure. Essential oils contain one or more compounds that make up the essential oil and can be synthesized or derived from any suitable naturally occurring substance such as carrot seed, cinnamon bark, clove, eucalyptus, white grapefruit, lemongrass, *etc.* For example, clove oil, contains: alpha-cubeben, alpha-pinene, beta-caryophyllene, eugenol, and isoeugenol. Illustrative pure compounds present in various essential oils can, individually or in combination, comprise an anti-*H*. *pylori* active substance according to various embodiments of the disclosure and include alpha-copaen, alpha-cubeben, alpha-pinene, alpha-selinen, beta-caryophyllene, beta-pinene, beta-selinen, camphor, carotol, carvacrol, cinnamaldehyde, citral, citronellal, decanal, eucalyptol, eugenol, gamma-terpinene, geraniol, geranyl acetate, isoeugenol, limonene, linalool, myrcene, nerol, nootkatone, octanal, *para*-cymene, sabinene, thymol, *etc.* In one embodiment, one or more essential oils, if present, are present in a composition of the disclosure in an amount of about 0.0025% to about 5%, about 0.005% to about 4%, or about 0.01% to about 3%, by weight of the composition.

Pronase is an illustrative mucolytic agent that can be used as an anti-*H*. *pylori* active substance. In one embodiment, if present, a mucolytic agent is present in a composition of the disclosure in an amount of about 2000 tyrosine units to about 100,000 tyrosine units, about 5,000 tyrosine units to about 90,000 tyrosine units, or about 10,000 tyrosine units to about 80,000 tyrosine units.

Cholestyramine can also be used as an anti-*H*. *pylori* active substance in compositions of the disclosure. If present, cholestyramine is present in an amount of about 50 mg to about 2 g, about 75 mg to about 1.75 g, about 100 mg to about 1.5 g or about 125 mg to about 1 g.

Lactoferrin can also be used as an anti-*H*. *pylori* active substance in compositions of the disclosure. If present, lactoferrin is present in an amount of about 20 mg to about 2 g, about 50 mg to about 2 g, about 75 mg to about 1.75 g, about 100 mg to about 1.5 g or about 125 mg to about 1 g.

Bile salts can also be used as an anti-*H*. *pylori* active substance in compositions of the disclosure. Bile salts are polar derivatives of cholesterol. These compounds are detergents because they contain both polar and nonpolar regions.

In some embodiments, the anti-*H*. *pylori* active substance, if present, is present in a total amount of about 0.1 % to about 90%, 0.2% to about 85%, about 0.5% to about 75%, or about 1 % to about 60%, by total weight of the composition. Illustratively, the anti-*H*. *pylori* active substance can be present in an amount of about 1%, about 2% about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 46%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83% about 84%, or about 85%, by weight of the total composition.

In another embodiment, an anti-*H*. *pylori* active substance, if present, is present in a total amount of about 1 mg to about 5000 mg, about 100 mg to about 4000 mg, about 150 mg to about 3000 mg, about 200 mg to about 2000 mg, about 200 mg to about 1500 mg or about 200 mg to about 1000 mg.

In other embodiments, an anti-*H*. *pylori* active substance is present in a composition of the disclosure in an amount of about 50 mg, about 100 mg about 150 mg, about 200 mg, about 250 mg, about 300 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1300 mg, about 1400 mg, about 1500 mg, about 1600 mg, about 1700 mg, about 1800 mg, about 1900 mg, about 2000 mg, about 2100 mg, about 2200 mg, about 2300 mg, about 2400 mg, about 2500 mg, about 2600 mg, about 2700 mg, about 2800 mg, about 2900 mg, about 3000 mg, about 3100 mg, about 3200 mg, about 3300 mg, about 3400 mg, about 3500 mg, about 3600 mg, about 3700 mg, about 3800 mg, about 3900 mg, about 4000 mg, about 4100 mg, about 4200 mg, about 4300 mg, about 4400, about 4500 mg, about 4600 mg, about 4700 mg, about 4800 mg, about 4900 mg or about 5000 mg.

In one embodiment, no portion of the anti-*H*. *pylori* active substance is enteric coated. In another embodiment, at least a portion of the anti-*H*. *pylori* active substance is not enteric coated. In another embodiment, at least a therapeutically effective portion of the anti-*H*. *pylori* active substance is not enteric coated.

In another embodiment, about 5%, about 15%, about 20%, about 30%, about 40%, about 50% or about 60%, by weight, of the anti-*H*. *pylori* active substance is not enteric coated. In another embodiment, a portion of the antibiotic comprises a "thin enteric coat" as is defined herein above.

The foregoing lists of anti-*H*. *pylori* active substance are meant to be illustrative and not exhaustive as a person of ordinary skill in the art would recognize that many other anti-*H*. *pylori* active substance could be created.

### NSAIDs

In one embodiment, compositions of the disclosure comprise an NSAID. The term "NSAID" as used herein refers to compounds acting as a nonsteroidal anti-inflammatory agent as identified as such by one of ordinary skill in the art.

Illustratively, the *Merck Manual,* 16th Edition, Merck Research Laboratories (1990) pp 1308-1309 provides well known examples of NSAIDs and is hereby incorporated by reference. Exemplary NSAIDs include, but are not limited to, salicylates and salicylic acid derivatives including but not limited to acetylsalicylic acid (aspirin), sodium acetylsalicylic acid, calcium acetylsalicylic acid, salicylic acid, sodium salicylate, acetaminosalol, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphtyl salicylate, olsalazine, parsalmide, phenyl acetylsalicylate, phenyl salicylate, salacetamide, salicylamide o-acetic acid, salicylsulfuric acid, salsalate, sulfasalazine; arylacetic acid derivatives including but not limited to aceclofenac, acemetacin, alclofenac, amfenac, amtolmetin guacil, bromfenac, bufexamac, cinmetacin, clopirac, diclofenac (e.g., Voltaren®, Voltaren-XR®, Cataflam®), etodolac (e.g., Lodine®), felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, and zomepirac; aminoarylcarboxylic acid derivatives including but not limited to enfenamic acid, etofenamate, flufenamic acid, isonixin, meclofenamic acid, mefenamic acid, niflumic acid, talniflumate, terofenamate, and tolfenamic acid; arylbutyric acid derivatives including but not limited to bumadizon, butibufen, fenbufen, xenbucin; arylcarboxylic acids including but not limited to clidanac, ketorolac, tinoridine; arylpropionic acid derivatives including but not limited to alminoprofen, benoxaprofin, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprofin, pirprofen, pranoprofen, protizinic acid, suprofen, tiaprofenic acid, ximoprofen, and zaltoprofen; pyrazoles including but not limited to difenamizole, and epirozole; pyrazolones including but not limited to apazone, benzpiperylon, feprazone, mofebutazone, morazone, oxyphenbutazone, phenylbutazone, pipebuzone, propyphenazone, prostaglandins, ramifenazone, suxibuzone, and thiazolinobutazone; thiazinecarboxamides including but not limited to ampiroxicam, droxica isoxicam, lomoxicam, piroxicam, and tenoxicam; and cyclooxygenase-II ("COX-II") inhibitors including but not limited to Celebrex® (celecoxib), Vioxx® (rofecoxib), Bextra® (valdecoxib); Arcoxia® (etoricoxib); Prexige® (lumiracoxib); and Dynastat® (parecoxib).

In various embodiments, the disclosure also provides for NSAIDs including, but not limited to: nabumetone (e.g., Relafen®), epsilon-acetamidocaproic acid, s-adenosylmethionine, 3-amino-4-hydroxybutytic acid, amixetrine, bendazac, benzydamine, alpha-bisabolol, bucololome, difenpiramide, ditazol, emorfazone, fepradinol, guaiazulene, nimesulide, oxaceprol, paranyline, perisoxal, proquazone, tebufelone, tenidap, zilenton, alcofenac, antipyrine, aminopyrine, dipyrone, aminopyrone, clofezone, prexazone, bucolome, cinchopen, clonixin, ditrazol, epirizole, floctafeninl, glaphenine, phenacetin, salidifamides.

one embodiment, an NSAID, if present, is present in a total amount of about 0.1% to about 85%, about 0.5% to about 75%, or about 1% to about 60%, by total weight of the composition. Illustratively, the NSAID can be present in an amount of about 1%, about 2% about 3%, about 4%, about 5%, about 6%, about 7%, about 8%, about 9%, about 10%, about 111%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 46%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83% about 84%, or about 85%, by weight of the total composition.

In another embodiment, an NSAID, if present, is present in a total amount of about 1 mg to about 1500 mg, about 1mg to about 1200 mg, about 1 mg to about 1000 mg, about 1 mg to about 800 mg or about 1 mg to about 500 mg.

In other embodiments, the NSAID is present in a composition of the disclosure in an amount of about 50 mg, about 100 mg about 150 mg, about 200 mg, about 250 mg, about 300 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg.

In one embodiment, no portion of the NSAID is enteric coated. In another embodiment, at least a portion of the NSAID is not enteric coated. In another embodiment, at least a therapeutically effective portion of the NSAID is not enteric coated. In another embodiment, at least about 5%, about 15%, about 20%, about 30%, about 40%, about 50% or about 60% of the NSAID is not enteric coated. In another embodiment, a portion of the NSAID comprises a "thin enteric coat" as is defined above.

The term "pain" includes all types of pain, including, but not limited to, chronic pain, such as arthritis pain (e.g. pain associated with osteoarthritis and rheumatoid arthritis), neuropathic pain, and post-operative pain, chronic lower back pain, cluster headaches, herpes neuralgia, phantom limb pain, central pain, dental pain, neuropathic pain, opioid-resistant pain, visceral pain, surgical pain, bone injury pain, pain during labor and delivery, pain resulting from bums, including sunburn, post partum pain, migraine, angina pain, and genitourinary tract-related pain including cystitis, the term also refers to nociceptive pain or nociception.

### Other Pharmaceutical Agents

In various embodiments, the compositions of the disclosure further comprise other pharmaceuticals agents such as sleep aids including but not limited to a benzodiazepine hypnotic, non-benzodiazepine hypnotic, antihistamine hypnotic, antidepressant hypnotic, herbal extract, barbiturate, peptide hypnotic, triazolam, brotizolam, loprazolam, lormetazepam, flunitrazepam, flurazepam, nitrazepam, quazepam, estazolam, temazepam, lorazepam, oxazepam, diazepam, halazepam, prazepam, alprazolam, chlordiazepoxide, clorazepate, an imidazopyridine or pyrazolopyrimidine hypnotic, zolpidem or zolpidem tartarate, zopiclone, eszopiclone, zaleplon, indiplone, diphenhydramine, doxylamine, phenyltoloxamine, pyrilamine, doxepin, amtriptyline, ramelteon, trimipramine, trazodone, nefazodone, buproprion, bupramityiptyline, an herbal extract such as valerian extract or amentoflavone, a hormone such as melatonin or gabapeptin

In another embodiment, other pharmaceutical agents include motility agents, including but not limited to 5-HT inhibitors such as erythromycin and erythromycin derivatives, alosetron, cilansetron, cisapride, domperidone, and metoclopramide, and agents useful for treating irritable bowel syndrome (IBS).

In still another embodiment, other pharmaceutical agents include pentagastrin.

### Iron

Compositions of the present disclosure optionally comprise iron. In one embodiment, the iron is in the form of a highly soluble ferrous salt or salts. Illustrative sources of iron include ferric ammonium citrate, ferrous sulfate, ferrous gluconate, ferrous fumarate and ferrous ammonium sulfate. Iron may be free iron (or non-heme iron), heme iron, iron polypeptide, or heme polypeptide.

In some embodiments of the disclosure, iron can be present in an amount of about 1 to about 25 mg, about 2.5 to about 20 mg, or about 5 to about 20 mg, for example, about 1 mg, about 2 mg, about 3 mg, about 4 mg; about 5 mg to about 100 mg or about 5 mg to about 50 mg, for example, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg.

In other embodiments, larger amounts of iron may be necessary, such as for patients suffering from iron deficiency, such as about 100 to about 500, about 200 to about 400, for example about 300 mg per dose.

### Vitamin B₁₂

In one embodiment, compositions of the disclosure optionally comprise vitamin B₁₂. The vitamin B₁₂ can be formulated together with either or both of the proton pump inhibitor and the buffering agent. Alternatively, the PPI, buffering agent and vitamin B₁₂ can be formulated separately for simultaneous or substantially simultaneous co-administration.

The term "vitamin B₁₂" herein includes cobalamin and cyanocobalamin, adnosylcobalamin, methylcobalamin, sulphitocobalamin, aquacobalamins as well as vitamin B_{12α}, hydroxocobalamin (B_{12b}), hydroxycobalamin, B_{12c}, and methyl B₁₂, including salts, esters and derivatives thereof and substantially pure forms or mixtures of any of the foregoing. Vitamin B₁₂ can be obtained from any suitable source, for example by fermentation of *Steptomyces griseus* or by isolation from aqueous liver extracts as is known in the art.

Compositions of the disclosure optionally comprise about 0.1 to about 15 µg, about 0.5 to about 12.5 µg, about 0.75 to about 10 µg or about 2 to about 8 µg of vitamin B₁₂, for example about 2, about 3, about 6, or about 8 µgs. The total amount of vitamin B₁₂ in one embodiment of the disclosure can be determined by the age, weight, health and condition of the subject to whom the composition is to be administered. Recommended oral daily intake of vitamin B₁₂ 2 is 2 µg for infants, 3 µg for children under 4 years, 6 µg for children over 4 years and for adults, and 8 µg for pregnant or lactating women. *See e.g.* Remington: The Science and Practice of Pharmacy, 20th ed. Chapter 106 (Vanderveen, E. and Vanderveen, J.E.), p.p. 1796 -1816 (2000).

In other embodiments, such as for passive absorption, larger amounts of vitamin B₁₂ may be needed, such as about 1 to about 5 mg per dose of vitamin B₁₂, for example hydroxycobalamin. In addition, since some degradation of vitamin B₁₂ can occur over time, it is envisioned that a suitable amount of vitamin B₁₂ be added so as to compensate for such degradation. For example in 30 days, about 50% degradation can occur. Accordingly, the use of twice as much vitamin B₁₂ could be needed to ensure that at the end of 30 days, the desired amount vitamin B₁₂ would remain.

### Protein Component

Compositions of the disclosure optionally comprise a protein component. The term "protein component" as used herein includes protein isolates, hydrolyzed proteins (protein hydrolysates) as well as protein concentrates. Also included within the definition of a protein component are peptone, tryptone, and peptides. A non-limiting example of a protein is lactoferrin. Another non-limiting example of a protein is 1-carnosine.

Compositions disclosed herein can comprise one or more of a protein isolate, a protein hydrolysate, a protein concentrate, peptone, tryptone, and/or peptides. A suitable protein component can be derived from any origin including plants, animals, or a combination thereof. Non-limiting examples of suitable sources of protein component include soy, com, whey, egg, casein, fish, meat, poultry *etc.*

Protein isolate typically comprises at least about 85%, for example about 85 - 95% protein on a dry basis. Suitable protein isolates can be prepared using any suitable procedure, for example by using an alcohol wash, water wash or ionization concentration techniques that separate at least a portion of carbohydrates and fats from the protein itself.

Protein concentrate typically comprises about 50% to about 85% protein on a dry basis, for example about 60 to about 85%. Protein concentrate can be prepared using any suitable process, for example by concentrating the desired protein through high heat drying (dehydration), acid extraction or filtration to reduce the original source to a more concentrated protein.

Protein hydrolysates are protein molecules that have been lysed, typically but not exclusively with water, into smaller peptides. Protein isolates suitable for disclosed embodiments include substantially pure protein isolate or protein isolate formulations, for example liquid or powder formulations. Non-limiting examples of powder protein hydrolysate formulations include Alimentum, Nutramigen, and Pregestimil.

In one embodiment, compositions of the disclosure comprise a protein component in a total amount of about 1% to about 95%, about 5% to about 90%, or about 10% to about 85% on a dry weight basis in the composition.

In another embodiment, compositions of the disclosure comprise a protein component in a total amount of about 1 mg to about 100 g, about 1 mg to about 20 g, about 1 mg to about 10 g, about 5 mg to about 5 g, about 10 mg to about 2.5 g, about 10 mg to about 1.0 g, or about 10 mg to about 0.5 g on a dry weight basis.

In another embodiment, the weight ratio of PPI to protein component, on a dry basis, is about 0.001 to about 1, about 0.0025 to about 0.5, or about 0.1 to about 0.05.

In another embodiment of the disclosure, the protein component has a Protein Digestibility-Corrected Amino Acid Score (PDCAAS) of at least about 0.68, at least about 0.75, at least about 0.80 at least about 0.85, at least about 0.90, at least about 0.92, at least about 0.95, at least about 0.98, or about 1.

In another embodiment of the disclosure, the protein component has a PDCAAS of about 0.68 to about 1, about 0.80 to about 1, about 0.90 to about 1, about 0. 92 to about 1 or about 0.95 to about 1.

Without being bound by theory, it is presently believed that upon administration of a composition of the disclosure to a subject, the protein component sacrificially combines with available hydrogen ion (in the GI tract) thereby preventing, slowing or delaying acid-related degradation of the PPI. In another embodiment, therefore, upon administration of a composition of the disclosure to a human subject, the PPI undergoes reduced gastrointestinal degradation by comparison with administration of PPI alone. This can be determined by any suitable method, for example by sampling and assaying contents of the subjects stomach at various time points after ingestion of a composition of the disclosure or a comparative PPI composition comprising no protein component (e.g. naked PPI).

In another embodiment, a composition of the disclosure does not contain an amino acid. In yet another embodiment, a composition of the disclosure does not contain an alkali earth metal buffering agent. In still another embodiment, a composition of the disclosure does not contain an alkaline earth metal buffering agent. In another embodiment, a composition of the disclosure does not contain aluminum and/or aluminum glycinate.

The foregoing list of suitable protein components is meant to be illustrative and not exhaustive as a person of ordinary skill in the art would recognize that other pharmaceutically acceptable protein components could be created.

### Buffering Agent

Compositions of the disclosure comprise one or more pharmaceutically acceptable buffering agents. Buffering agents useful in the present disclosure include agents possessing pharmacological activity as a weak or strong base. In one embodiment, the buffering agent, when formulated with or administered substantially simultaneously with a PPI, functions to raise the pH of gastrointestinal fluid and thereby to substantially prevent or inhibit acid degradation of the PPI by gastrointestinal fluid for a period of time.

In another embodiment, buffering agents useful in accordance with the present disclosure comprise, but are not limited to, a salt of a Group IA metal including, for example, a bicarbonate salt of a Group IA metal, a carbonate salt of a Group IA metal, an alkaline earth metal buffering agent, an amino acid, an alkaline salt of an amino acid, an aluminum buffering agent, a calcium buffering agent, a sodium buffering agent, or a magnesium buffering agent. Other suitable buffering agents include alkali (sodium and potassium) or alkaline earth (calcium and magnesium) carbonates, phosphates, bicarbonates, citrates, borates, acetates, phthalates, tartrates, succinates and the like, such as sodium or potassium phosphate, citrate, borate, acetate, bicarbonate and carbonate.

Non-limiting examples of suitable buffering agents include aluminum, magnesium hydroxide, aluminum hydroxide/magnesium hydroxide co-precipitate, aluminum hydroxide/sodium bicarbonate co-precipitate, calcium acetate, calcium bicarbonate, calcium borate, calcium carbonate, calcium bicarbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, L-arginine, magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, and trometarnol. (Based in part upon the list provided in The Merck Index, Merck & Co. Rahway, N.J. (2001)). In addition, due to the ability of proteins or protein hydrolysates to react with stomach acids, they too can serve as buffering agents in the present embodiments. Furthermore, combinations or mixtures of the above mentioned buffering agents can be used in the pharmaceutical formulations described herein.

Buffering agents also include buffering agents or combinations of buffering agents that interact with HCl (or other acids in the environment of interest) faster than the proton pump inhibitor interacts with the same acids. When placed in a liquid phase such as water, these buffering agents produce and maintain a pH greater than the pKa of the proton pump inhibitor.

Buffering agents also include peptides, such as L-carnosine. In one embodiment, a composition of the disclosure comprises L-carnosine. Still another embodiment of the disclosure comprises L-carnosine in a ratio of greater than about 20 parts L-carnosine to about 1 part PPI. Other embodiments comprise L-carnosine and PPI in an amount of about 20:1, about 25:1, about 30:1, about 35:1, about 40: 1, about 45:1, or about 50:1.

Other embodiments of the disclosure comprises a PPI, at least one buffering agent in an amount of about 20 parts to about 1 part PPI, and a protein component in an amount of about 20 parts to about 1 part PPI. For example, an embodiment of the disclosure comprises tenatoprazole, sodium bicarbonate in an amount of about 20 parts to about 1 part tenatoprazole, and L-carnosine in an amount of about 20 parts to about 1 part tenatoprazole. Another embodiment of the disclosure comprises about 40 mg tenatoprazole, about 1600 mg sodium bicarbonate, and about 1600 mg L-carnosine. Still another embodiment of the disclosure comprises about 40 mg tenatoprazole, about 1600 mg sodium bicarbonate and magnesium hydroxide, and about 1600 mg L-carnosine. Yet another embodiment of the disclosure comprises about 40 mg omeprazole, about 1600 mg sodium bicarbonate and magnesium hydroxide, and about 1600 mg L-carnosine.

Other embodiments of the disclosure comprise omeprazole, sodium bicarbonate in an amount of about 20 parts to about 1 part omeprazole, and L-carnosine in an amount of about 20 parts to about 1 part omeprazole. For example, an embodiment of the disclosure comprises about 40 mg omeprazole, about 1600 mg sodium bicarbonate, and about 1600 mg L-carnosine.

The foregoing list of suitable buffering agents is meant to be illustrative and not exhaustive as a person of ordinary skill in the art would recognize that other pharmaceutically acceptable buffering agents could be created.

In various other embodiments, the buffering agent is present in a total amount of about 0.1 mEq/mg to about 5 mEq/mg of the proton pump inhibitor, about 0.5 mEq/mg to about 3 mEq/mg of the proton pump inhibitor, about 0.6 mEq/mg to about 2.5 mEq/mg of the proton pump inhibitor, about 0.7 mEq/mg to about 2.0 mEq/mg of the proton pump inhibitor, about 0.8 mEq/mg to about 1.8 mEq/mg of the proton pump inhibitor, about 1.0 mEq/mg to about 1.5 mEq/mg of the proton pump inhibitor. In another embodiment, the buffering agent is present in an amount of about 0.5 mEq/mg of the proton pump inhibitor, about 0.75 mEq/mg of the proton pump inhibitor, or about 1 mEq/mg of the proton pump inhibitor on a dry weight basis.

In still another embodiment, one or more buffering agents are present in a total amount of about 0.5 mEq to about 160 mEq, about 1 mEq to about 150 mEq, about 10 mEq to about 150 mEq, about 10 mEq to about 75 mEq, about 10 mEq to about 60 mEq, or about 10 mEq to about 50 mEq. Illustratively, a composition of the disclosure can comprise about 1 mEq, about 5 mEq, about 10 mEq, about 15 mEq, about 20 mEq, about 25 mEq, about 30 mEq, about 35 mEq, about 40 mEq, about 45 mEq, about 50 mEq, about 60 mEq, about 70 mEq, about 80 mEq, about 90 mEq, about 100 mEq, about 110 mEq, about 120 mEq, about 130 mEq, about 140 mEq, about 150 mEq, or about 160 mEq of buffering agent.

In yet another embodiment, one or more buffering agents are present in a total amount of about 10 mEq, about 11 mEq, about 12 mEq, about 13 mEq, about 14 mEq, about 15 mEq, or at least about 16 mEq.

In another embodiment, one or more buffering agents and the mixture of the first and subsequent proton pump inhibitors or the salt form of a proton pump inhibitor and the free base form of a proton pump inhibitor are present in a weight ratio of about 5:1, about 7:1, about 10:1, about 20:1, greater than about 20:1, about 21:1, about 22:1, about 23:1, about 25:1, about 30:1, about 35:1, about 40:1, greater than about 40:1, about 45:1, about 53:3; about 11:1, about 28:3, about 28:5, about 23:3, about 26:1, about 27:2, or about 31:1.

In still another embodiment, PPI1, PPI2, and one or more buffering agents are present in a weight ratio of about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20.

In yet another embodiment, the salt form of a proton pump inhibitor ("PPI-salt"), the free base form of a proton pump inhibitor ("PPI-base"), and one or more buffering agents are present in a weight ratio of about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20.

In another embodiment, the amount of buffering agent present in a composition of the disclosure ranges from about 200 to about 3500 mg, about 300 to about 3000 mg, about 400 to about 2500 mg, or about 500 to about 2200 mg, about 600 to about 2000, or about 700 to about 1800 mg. In other embodiments, the amount of buffering agent present in a composition of the disclosure is about 200 mgs, or about 300 mgs, or about 400 mgs, or about 500 mgs, or about 600 mgs, or about 700 mgs, or about 800 mgs, or about 900 mgs, or about 1000 mgs, or about 1100 mgs, or about 1200 mgs, or about 1300 mgs, or about 1400 mgs, or about 1500 mgs, or about 1600 mgs, or about 1700 mgs, or about 1800 mgs, or about 1900 mgs, or about 2000 mgs, or about 2100 mgs, or about 2200 mgs, or about 2300 mgs, or about 2400 mgs, or about 2500 mgs, or about 2600 mgs, or about 2700 mgs, or about 2800 mgs, or about 2900 mgs, or about 3000 mgs, or about 3200 mgs, or about 3500 mgs.

In another embodiment, one or more buffering agents are present in a composition of the disclosure in a total amount that is greater than 800 mg, for example about 920 mg or at least about 1000 mg.

In still another embodiment, the buffering agent and the mixture of PPI1 and PPI2 or PPI-salt and PPI-base (hereinafter "proton pump inhibitor mixture") are present in a weight ratio greater than 20:1, not less than about 21:1, not less than about 22:1, not less than about 23:1, not less than about 24:1, not less than about 25:1, not less than about 26:1, not less than about 27:1, not less than about 28:1, not less than about 29:1, not less than about 30:1, not less than about 31:1, not less than about 32:1, not less than about 33:1, not less than about 34:1, not less than about 35:1, not less than about 36:1, not less than about 37:1, not less than about 38:1, not less than about 39:1, not less than about 40:1, not less than about 41:1, not less than about 42:1, not less than about 43:1, not less than about 44:1, not less than about 45:1, not less than about 46:1, not less than about 47:1, not less than about 48:1, not less than about 49:1, not less than about 50:1, not less than about 53:3; not less than about 11:1, not less than about 28:3, not less than about 21:1, not less than about 28:5, not less than about 23:3, not less than about 26:1, not less than about 53:3, not less than about 27:2, or not less than about 31:1.

In yet another embodiment, PPI1, PPI2, and the one or more buffering agents are present in a weight ratio of about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20.

In another embodiment, PPI-salt, PPI-base, and one or more buffering agents are present in a weight ratio of about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20.

In yet another embodiment, a composition is provided that comprises a combination of at least two non-amino acid buffering agents, wherein the combination of at least two non-amino acid buffering agents comprises substantially no aluminum hydroxide-sodium bicarbonate co-precipitate. In a related embodiment, if such a composition comprises a poly[phosphoryl/sulfon]-ated carbohydrate, the weight ratio of poly[phosphoryl/sulfon]-ated carbohydrate to buffering agent is less than 1:5 (0.2), less than 1:10 (0.1) or less than 1:20 (0.05). Alternatively, the poly[phosphoryl/sulfon]-ated carbohydrate is present in the composition, if at all, in an amount less than 50 mg, less than 25 mg, less than 10 mg or less than 5 mg. In another embodiment, the composition contains no poly[phosphoryl/sulfon]-ated carbohydrate.

In other embodiments, if the pharmaceutical composition comprises an amino acid buffering agent, the total amount of amino acid buffering agent present in the pharmaceutical composition is less than about 5 mEq, or less than about 4 mEq, or less than about 3 mEq.

The phrase "amino acid buffering agent" as used herein includes but is not limited to amino acids, amino acid salts, and amino acid alkali salts including, for example: glycine, alanine, threonine, isoleucine, valine, phenylalanine, glutamic acid, asparagininic acid, lysine and/or lysine glutamic acid salt, glycine hydrochloride, L-alanine, DL-alanine, L-threonine, DL-threonine, L-isoleucine, L-valine, L-phenylalanine, L-glutamic acid, L-glutamic acid hydrochloride, L-glutamic acid sodium salt, L-asparaginic acid, L-asparaginic acid sodium salt, L-lysine and L-lysine-L-glutamic acid salt. The term "non-amino acid buffering agent" herein includes buffering agents as defined hereinabove but does not include amino acid buffering agents.

The foregoing list of amino acid buffering agents is meant to be illustrative and not exhaustive as a person of ordinary skill in the art would recognize that other pharmaceutically acceptable amino acid buffering agents could be created.

In another embodiment, a composition of the disclosure comprises at least one non-amino acid buffering agent wherein the non-amino acid buffering agent is present in the composition in a total amount greater than 800 mg. In a related embodiment, if such a composition comprises a poly[phosphoryl/sulfon]-ated carbohydrate, the weight ratio of poly[phosphoryl/sulfon]-ated carbohydrate to buffering agent is less than 1:5 (0.2), less than 1:10 (0.1) or less than 1:20 (0.05). Alternatively, the poly[phosphoryl/sulfon]-ated carbohydrate is present in the composition, if at all, in an amount less than 50 mg, less than 25 mg, less than 10 mg or less than 5 mg.

In still another embodiment, a composition is provided which comprises at least one buffering agent in a total amount of at least about 10 mEq. In a related embodiment, if an amino acid buffering agent is present in the composition, at least one of the following conditions is met: (1) the weight ratio of amino acid buffering agent:proton pump inhibitor mixture is greater than 20:1; (2) the composition comprises at least two non-amino acid buffering agents; (3) the composition comprises at least one non-amino acid buffering agent wherein the weight ratio of the at least one non-amino acid buffering agent:proton pump inhibitor mixture is greater than 20:1; and/or (4) the weight ratio of total buffering agent:proton pump inhibitor mixture is greater than 40:1.

In yet another embodiment, a composition is provided which comprises at least one buffering agent in a total amount of at least about 10 mEq. In a related embodiment, if an amino acid buffering agent is present in the composition, at least one of the following conditions is met: (1) the weight ratio of PPI1:PPI2:amino acid buffering agent or PPI-salt:PPI-base:amino acid buffering agent is about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20; (2) the composition comprises at least two non-amino acid buffering agents; (3) the composition comprises at least one non-amino acid buffering agent wherein the weight ratio of PPI1:PPI2:non-amino acid buffering agent or PPI-salt:PPI-base:non-amino acid buffering agent is about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20; and/or (4) the weight ratio of PPI1:PPI2:total buffering agent or PPI-salt:PPI-base:total buffering agent is about 2:1:50, about 3:2:50, about 2:1:25, about 2:1:60, about 3:2:25, about 2:1:20, about 1:1:50, about 1:2:50, about 1:1:25, about 1:1:60, about 1:2:25, or about 1:1:20.

In other embodiments, where two or more buffering agents are present, the two or more buffering agents comprise at least two non-amino acid buffering agents, wherein the combination of at least two non-amino acid buffering agents comprises substantially no aluminum hydroxide-sodium bicarbonate co-precipitate.

In still another embodiment, the buffering agent comprises a mixture of sodium bicarbonate, calcium carbonate, and magnesium hydroxide, wherein the sodium bicarbonate, calcium carbonate, and magnesium hydroxide are each present in an amount of about 0.1 mEq/mg proton pump inhibitor mixture to about 5 mEq/mg of the proton pump inhibitor mixture.

In another embodiment, the buffering agent comprises a mixture of sodium bicarbonate, calcium carbonate, and magnesium hydroxide, wherein the sodium bicarbonate, calcium carbonate, and magnesium hydroxide are each present in an amount of about 0.1 mEq/mg proton pump inhibitor to about 5 mEq/mg of the either proton pump inhibitor.

Also provided herein are pharmaceutical compositions comprising at least one soluble buffering agent. The term "soluble buffering agent" as used herein refers to an antacid that has a solubility of at least about 500 mg/mL, or at least about 300 mg/mL, or at least about 200 mg/mL, or at least about 100 mg/mL in gastrointestinal fluid or simulated gastrointestinal fluid.

In some embodiments, the buffering agent has a defined particle size distribution. For example, in one embodiment, the D₅₀, D₇₀, D₈₀, or D₉₀ particle size of the buffering agent, by weight or by number, is no greater than about 10 µm, is no greater than about 20 µm, no greater than about 30 µm, no greater than about 40 µm, no greater than about 50 µm, no greater than about 60 µm, no greater than about 70 µm, no greater than about 80 µm, no greater than about 90 µm, no greater than about 100 µm in diameter, no greater than about 200 µm in diameter, no greater than about 300 µm in diameter, no greater than about 400 µm in diameter, no greater than about 1000 µm in diameter, no greater than about 2000 µm in diameter, no greater than about 3000 µm in diameter, no greater than about 4000 µm in diameter, no greater than about 6000 µm in diameter, or no greater than about 9000 µm in diameter.

The foregoing list of suitable buffering agents is meant to be illustrative and not exhaustive as a person of ordinary skill in the art would recognize that other pharmaceutically acceptable buffering agents could be created.

### Pharmaceutical Excipients

Various embodiments can, if desired, include one or more pharmaceutically acceptable excipients. The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition. Excipients include, by way of illustration and not limitation, diluents, disintegrants, binding agents, adhesives, wetting agents, lubricants, glidants, surface modifying agents, substances added to mask or counteract a disagreeable taste or odor, flavors, dyes, fragrances, and substances added to improve appearance of the composition. Any such excipients can be used in any dosage forms of according to the present disclosure, including liquid, solid or semi-solid dosage forms.

Excipients optionally employed in various embodiments can be solids, semi-solids, liquids or combinations thereof. Compositions of the disclosure including excipients can be prepared by various pharmaceutical techniques such as admixing an excipient with a drug or therapeutic agent.

In various embodiments, compositions optionally comprise one or more pharmaceutically acceptable diluents as excipients. Suitable diluents illustratively include, without limitation, either individually or in combination, lactose, including anhydrous lactose and lactose monohydrate; starches, including directly compressible starch and hydrolyzed starches (*e.g*., Celutab™ and Emdex™); mannitol; sorbitol; xylitol; dextrose (*e.g*., Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose (*e.g*., Rexcel™) and powdered cellulose; calcium carbonate; glycine; bentonite; polyvinylpyrrolidone; and the like. Such diluents, if present, may constitute in total about 5% to about 99%, about 10% to about 85%, or about 20% to about 80%, of the total weight of the composition. In various embodiments, the diluent or diluents selected may exhibit suitable flow properties and, where tablets are desired, compressibility.

The use of extragranular microcrystalline cellulose (that is, microcrystalline cellulose added to a wet granulated composition after a drying step) can be used to alter or control hardness (for tablets) and/or disintegration time.

In various embodiments, compositions optionally comprise one or more pharmaceutically acceptable disintegrants as excipients, such as in tablet formulations. Suitable disintegrants include, without limitation, either individually or in combination, starches, including crosslinked polyvinylpyrrolidone (crospovidone USP/NF), carboxymethyl cellulose (sodium CMC), chitin, chitosan, sodium starch glycolate (*e.g*., Explotab™ of Pen West) and pregelatinized corn starches (*e.g*., National™ 1551, National™ 1550, and Colocom™ 1500), clays (*e.g*., Veegum™ HV), celluloses such as purified cellulose, microcrystalline cellulose, methylcellulose, carboxyethylcellulose and sodium carboxymethylcellulose, croscarmellose sodium (*e.g*., Ac-Di-Sol™ of FMC), alginates, and gums such as agar, guar, xanthan, locust bean, karaya, pectin and tragacanth gums.

Disintegrants may be added at any suitable step during the preparation of the composition, particularly prior to a granulation step or during a lubrication step prior to compression. Such disintegrants, if present, may constitute in total about 0.2% to about 30%, about 0.2% to about 10%, or about 0.2% to about 5%, of the total weight of the composition.

In one embodiment, crosslinked polyvinylpyrrolidone (crospovidone USP/NF) is an optional disintegrant for tablet or capsule disintegration, and, if present, may optionally constitute about 1% to about 5% of the total weight of the composition.

In another embodiment, chitin is an optional disintegrant for tablet or capsule disintegration.

In still another embodiment, chitosan is an optional disintegrant for tablet or capsule disintegration.

In still another embodiment, carboxymethyl cellulose (sodium CMC) is an optional disintegrant for tablet or capsule disintegration.

In another embodiment, croscarmellose sodium is a disintegrant for tablet or capsule disintegration, and, if present, may optionally constitute about 0.2% to about 10%, about 0.2% to about 7%, or about 0.2% to about 5%, of the total weight of the composition.

Various embodiments described herein optionally comprise one or more pharmaceutically acceptable binding agents or adhesives as excipients, particularly for tablet formulations. Such binding agents and adhesives may impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, without limitation, either individually or in combination, acacia; tragacanth; sucrose; gelatin; glucose; starches such as, but not limited to, pregelatinized starches (*e.g*., National™ 1511 and National™ 1500); celluloses such as, but not limited to, methylcellulose and carmellose sodium (*e.g.,* Tylose™); alginic acid and salts of alginic acid; magnesium aluminum silicate; PEG; guar gum; polysaccharide acids; bentonites; povidone, for example povidone K-15, K-30 and K-29/32; polymethacrylates; HPMC; hydroxypropylcellulose (*e.g*., Klucel™); and ethylcellulose (*e.g*., Ethocel™). Such binding agents and/or adhesives, if present, may constitute in total about 0.5% to about 25%, about 0.75% to about 15%, or about 1% to about 10%, of the total weight of the composition.

Compositions described herein optionally comprise one or more pharmaceutically acceptable wetting agents as excipients. Non-limiting examples of surfactants that can be used as wetting agents in various compositions include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e.g*., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e.g*., Tween™ 80 of ICI), propylene glycol fatty acid esters, for example propylene glycol laurate (*e.g*., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, may constitute in total about 0.25% to about 15%, about 0.4% to about 10%, or about 0.5% to about 5%, of the total weight of the composition.

Compositions described herein optionally comprise one or more pharmaceutically acceptable lubricants (including anti-adherents and/or glidants) as excipients. Suitable lubricants include, without limitation, either individually or in combination, glyceryl behapate (*e.g*., Compritol™ 888); stearic acid and salts thereof, including magnesium (magnesium stearate), calcium and sodium stearates; hydrogenated vegetable oils (*e.g*., Sterotex™); colloidal silica; talc; waxes; boric acid; sodium benzoate; sodium acetate; sodium fumarate; sodium chloride; DL-leucine; PEG (*e.g*., Carbowax™ 4000 and Carbowax™ 6000); sodium oleate; sodium lauryl sulfate; and magnesium lauryl sulfate. Such lubricants, if present, may constitute in total about 0.1% to about 10%, about 0.2% to about 8%, or about 0.25% to about 5%, of the total weight of the composition.

Suitable anti-adherents include, without limitation, talc, cornstarch, DL-leucine, sodium lauryl sulfate and metallic stearates. Talc is a anti-adherent or glidant used, for example, to reduce formulation sticking to equipment surfaces and also to reduce static in the blend. Talc, if present, may constitute about 0.1% to about 10%, about 0.25% to about 5%, or about 0.5% to about 2%, of the total weight of the composition.

Glidants can be used to promote powder flow of a solid formulation. Suitable glidants include, without limitation, colloidal silicon dioxide, starch, talc, tribasic calcium phosphate, powdered cellulose and magnesium trisilicate.

Compositions described herein can comprise one or more flavoring agents, sweetening agents, and/or colorants. Flavoring agents useful in the present embodiments include, without limitation, acacia syrup, alitame, anise, apple, aspartame, banana, Bavarian cream, berry, black currant, butter, butter pecan, butterscotch, calcium citrate, camphor, caramel, cherry, cherry cream, chocolate, cinnamon, citrus, citrus punch, citrus cream, cocoa, coffee, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, MagnaSweet®, maltol, mannitol, maple, menthol, mint, mint cream, mixed berry, nut, orange, peanut butter, pear, peppermint, peppermint cream, Prosweet® Powder, raspberry, root beer, rum, saccharin, safrole, sorbitol, spearmint, spearmint cream, strawberry, strawberry cream, stevia, sucralose, sucrose, Swiss cream, tagatose, tangerine, thaumatin, tutti fruitti, vanilla, walnut, watermelon, wild cherry, wintergreen, xylitol, and combinations thereof, for example, anise-menthol, cherry-anise, cinnamon-orange, cherry-cinnamon, chocolate-mint, honey-lemon, lemon-lime, lemon-mint, menthol-eucalyptus, orange-cream, vanilla-mint, etc.

Sweetening agents that can be used in the present embodiments include, by way of example and not limitation, acesulfame potassium (acesulfame K), alitame, aspartame, cyclamate, cylamate, dextrose, isomalt, MagnaSweet®, maltitol, mannitol, neohesperidine DC, neotame, Prosweet® Powder, saccharin, sorbitol, stevia, sucralose, sucrose, tagatose, thaumatin, xylitol, and the like.

The foregoing excipients can have multiple roles. For example, starch can serve as a filler as well as a disintegrant. The classification of excipients listed herein is not to be construed as limiting in any manner.

### Pharmaceutical Dosage Forms

In various embodiments, compositions can be formulated as oral solid, liquid, or semi-solid dosage forms. In one embodiment, such compositions are in the form of discrete dose units or dosage units. The terms "dose unit" and/or "dosage unit" herein refer to a portion of a pharmaceutical composition that contains an amount of a therapeutic agent suitable for a single administration to provide a therapeutic effect. Such dosage units may be administered one to a small plurality (*i.e*. 1 to about 4) of times per day, or as many times as needed to elicit a therapeutic response. A particular dosage form can be selected to accommodate any desired frequency of administration to achieve a specified daily dose. Typically one dose unit, or a small plurality (*i.e*. up to about 4) of dose units, provides a sufficient amount of the active drug (*e.g*. at least one PPI) to result in the desired response or effect.

Alternatively, compositions of the disclosure can also be formulated for rectal, topical, or parenteral (*e.g*. subcutaneous, intramuscular, intravenous and intradermal or infusion) delivery.

In one embodiment, compositions of the disclosure are suitable for rapid onset of therapeutic effect, particularly with respect to the PPI components. In another embodiment, upon oral administration of a composition to a subject, at least a therapeutically effective amount of the PPIs is available for absorption by the subject. As discussed above, most commercially available PPIs require enteric coating to prevent exposure of the PPI to gastrointestinal fluids (and consequent drug degradation) by way of pH dependent coatings. Such coating, in turn, prevents rapid PPI absorption and therapeutic onset of action. Compositions of the present disclosure, by contrast, do not require enteric coating to maintain drug stability in gastrointestinal fluids and thereby provide for rapid absorption and onset of therapeutic effect. In fact, in one embodiment, a composition comprises at least a therapeutically effective amount of at least one PPI that is not enteric coated. However, other embodiments are compositions which optionally include at least part of one proton pump inhibitor that is enteric coated.

In another embodiment, a single dosage unit, be it solid or liquid, comprises a therapeutically and/or prophylactically effective amount of PPIs. The term "therapeutically effective amount" or "therapeutically and/or prophylactically effective amount" as used herein refers to an amount of compound or agent that is sufficient to elicit the required or desired therapeutic and/or prophylactic response, as the particular treatment context may require.

It will be understood that a therapeutically and/or prophylactically effective amount of a drug for a subject is dependent *inter alia* on the body weight of the subject. A "subject" herein to which a therapeutic agent or composition thereof can be administered includes a human subject of either sex and of any age, and also includes any nonhuman animal, particularly a domestic or companion animal, illustratively a cat, dog or a horse.

### Solid Dosage Forms

In various embodiments, compositions of the disclosure are in the form of solid dosage forms or units. Non-limiting examples of suitable solid dosage forms include tablets (*e.g*. suspension tablets, bite suspension tablets, rapid dispersion tablets, chewable tablets, effervescent tablets, bilayer tablets, *etc*), caplets, capsules (*e.g.* a soft or a hard gelatin capsule), powder (*e.g.* a packaged powder, a dispensable powder or an effervescent powder), lozenges, sachets, cachets, troches, pellets, granules, microgranules, encapsulated microgranules, powder aerosol formulations, or any other solid dosage form reasonably adapted for oral administration.

Tablets are an illustrative dosage form for compositions of the disclosure. Tablets can be prepared according to any technique used in the pharmaceutical industry. In one embodiment, tablets or other solid dosage forms can be prepared by processes that employ one or a combination of methods including, without limitation, (1) dry mixing, (2) direct compression, (3) milling, (4) dry or non-aqueous granulation, (5) wet granulation, or (6) fusion.

The individual steps in the wet granulation process of tablet preparation typically include milling and sieving of the ingredients, dry powder mixing, wet massing, granulation and final grinding. Dry granulation involves compressing a powder mixture into a rough tablet or "slug" on a heavy-duty rotary tablet press. The slugs are then broken up into granular particles by a grinding operation, usually by passage through an oscillation granulator. The individual steps include mixing of the powders, compressing (slugging) and grinding (slug reduction or granulation). Typically, no wet binder or moisture is involved in any of the steps.

In another embodiment, solid dosage forms such as tablets can be prepared by mixing PPI1 and PPI2 or PPI-salt and PPI-base with at least one buffering agent as described herein above and, if desired, with one or more optional pharmaceutical excipient to form a substantially homogeneous preformulation blend. The preformulation blend can then be subdivided and optionally further processed (*e.g*. compressed, encapsulated, packaged, dispersed, *etc*.) into any desired dosage forms.

Compressed tablets can be prepared by compacting a powder or granulation composition of the disclosure. The term "compressed tablet" generally refers to a plain, uncoated tablet suitable for oral ingestion, prepared by a single compression or by pre-compaction tapping followed by a final compression. Tablets of the present disclosure may be coated or otherwise compounded to provide a dosage form affording the advantage of improved handling or storage characteristics. In one embodiment, such coating will be selected so as to not substantially delay onset of therapeutic effect of a composition upon administration to a subject. The term "suspension tablet" as used herein refers to a compressed tablet that rapidly disintegrates after placement in water.

one embodiment, a composition comprises a multi-layer tablet having a core comprising two proton pump inhibitors; the core is substantially or completely surrounded by the buffering agent. The buffering agent layer can optionally be coated with one or more coating materials. In one embodiment, the optional coating is optionally an enteric coating. In a related embodiment, the buffering agent layer completely surrounds the core. In another embodiment, the buffering agent layer partially surrounds the core. In yet another embodiment, the buffering agent layer is in contact with a portion of or with all of the surface area of the core.

In another embodiment, a composition comprises a multi-layer tablet having a core comprising the first proton pump inhibitor. The core is substantially or completely surrounded by a second layer comprising a buffering agent. A third layer comprising the subsequent proton pump inhibitor substantially or completely surrounds the second layer comprising the buffering agent. The third layer is substantially or completely surrounded by a fourth layer comprising the same or different buffering agent. The fourth layer can optionally be coated with one or more coating materials. The successive layering of additional proton pump inhibitors and buffering agents can be continued for any number of iterations with the same or different proton pump inhibitors and buffering agents. In one embodiment, the optional coating is optionally an enteric coating. In a related embodiment, the second layer comprising buffering agent completely surrounds the core. In another embodiment, the second layer comprising buffering agent partially surrounds the core. In yet another embodiment, the second layer comprising buffering agent is in contact with a portion of or with all of the surface area of the core. In a related embodiment, the fourth layer comprising buffering agent completely surrounds the third layer comprising the subsequent proton pump inhibitor. In another embodiment, the fourth layer comprising buffering agent partially surrounds the third layer comprising the subsequent proton pump inhibitor. In yet another embodiment, the fourth layer comprising buffering agent is in contact with a portion of or with all of the third layer comprising the subsequent proton pump inhibitor.

In another embodiment, a composition comprises a multi-layer tablet having a core comprising either PPI-salt or PPI-base. The core is substantially or completely surrounded by a second layer comprising a buffering agent. A third layer comprising another form of PPI substantially or completely surrounds the second layer comprising the buffering agent. The third layer is substantially or completely surrounded by a fourth layer comprising the same or different buffering agent. The fourth layer can optionally be coated with one or more coating materials. The successive layering of additional forms proton pump inhibitors and buffering agents can be continued for any number of iterations with the same or different proton pump inhibitors and buffering agents. In one embodiment, the optional coating is optionally an enteric coating. In a related embodiment, the second layer comprising buffering agent completely surrounds the core. In another embodiment, the second layer comprising buffering agent partially surrounds the core. In yet another embodiment, the second layer comprising buffering agent is in contact with a portion of or with all of the surface area of the core. In a related embodiment, the fourth layer comprising buffering agent completely surrounds the third layer comprising the subsequent proton pump inhibitor. In another embodiment, the fourth layer comprising buffering agent partially surrounds the third layer comprising the subsequent proton pump inhibitor. In yet another embodiment, the fourth layer comprising buffering agent is in contact with a portion of or with all of the third layer comprising the subsequent proton pump inhibitor.

In still another embodiment, one or more intermediate layers exists in between the core and the buffering agent. The intermediate layers can comprise any pharmaceutically acceptable material, particularly inert and non-pH sensitive coating materials such as polymer based coatings.

In yet another embodiment, a composition comprises a multi-layer tablet having a core comprising a first PPI; the core is substantially or completely surrounded by the subsequent PPI and the buffering agent. Optionally, an intermediate layer can exist between the first PPI core and the subsequent PPI/buffering agent surrounding the core. In one embodiment, the optional intermediate layer is a coating layer. In another embodiment, the coating layer is optionally an enteric coating.

In another embodiment, a composition comprises a multi-layer tablet having a core comprising either PPI-salt or PPI-base; the core is substantially or completely surrounded by another form of PPI and the buffering agent. Optionally, an intermediate layer can exist between the PPI-salt or PPI-base core and the other form of PPI/buffering agent surrounding the core. In one embodiment, the optional intermediate layer is a coating layer. In another embodiment, the coating layer is optionally an enteric coating.

In one such embodiment, the buffering agent/PPI layer completely surrounds the core. In another embodiment, the buffering agent/PPI layer partially surrounds the core. In yet another embodiment, the buffering agent/PPI layer is in contact with a portion of or with all of the surface area of the core.

In another embodiment, compositions can be microencapsulated wherein the first PPI, subsequent PPI, and one or more buffering agents are microencapsulated together, for example as modified from the description in U.S. Patent Publication No. 2005/0037070, hereby incorporated by reference herein in its entirety.

In still another embodiment, compositions can be microencapsulated wherein the PPI-salt, PPI-base, and one or more buffering agents are microencapsulated together, for example as modified from the description in U.S. Patent Publication No. 2005/0037070, hereby incorporated by reference herein in its entirety.

In another embodiment, a composition comprises a first proton pump inhibitor, one or more additional proton pump inhibitors, and one or more buffering agents mixed together in powder form and optionally filled into a capsule, for example a hard or soft gelatin or HPMC capsule.

In another embodiment, a composition of the invention is in the form of a molded article, for example a pellet. The term "molded article" herein refers to a discrete dosage form that can be formed by compression, extrusion, or other similar processes. In one embodiment, the molded article is moldable. The term "moldable" in the present context means capable of being shaped or molded by hand. A moldable article herein will therefore have a hardness lower than a conventional pharmaceutical tablet. Such a moldable article will also be capable of being chewed by an animal, for example a horse.

Such an article can comprise, in addition to the PPI and buffering agent, and other excipients described herein, a filler, a sweetener and a flavoring agent. Extrusion is a process of shaping material by forcing it to flow through a shaped opening in a die or other solid. Extruded material emerges as an elongated article with substantially the same profile as the die opening.

In yet another embodiment, a composition comprises PPI-salt, PPI-base, and one or more buffering agents mixed together in powder form and optionally filled into a capsule, for example a hard or soft gelatin or HPMC capsule.

### Liquid Dosage Forms

In another embodiment, compositions described herein can be in the form of liquid dosage forms or units. Non-limiting examples of suitable liquid dosage forms include solutions, suspension, elixirs, syrups, liquid aerosol formulations, etc.

In one embodiment, a liquid composition comprising water or other solvent, first PPI, one or more additional PPIs and a buffering agent can be prepared. In another embodiment, compositions described herein are in the form of a powder for suspension that can be suspended in a liquid vehicle prior to administration to a subject. While the powder for suspension itself, can be a solid dosage form of the present disclosure, the powder dispersed in liquid also comprises a liquid embodiment of the disclosure.

In another embodiment, a liquid composition comprising water or other solvent, PPI-salt, one or more additional forms PPIs and one or more buffering agents can be prepared. In another embodiment, compositions described herein are in the form of a powder for suspension that can be suspended in a liquid vehicle prior to administration to a subject. While the powder for suspension itself, can be a solid dosage form of the present disclosure, the powder dispersed in liquid also comprises a liquid embodiment of the disclosure.

Suspension compositions comprise a first PPI, one or more additional PPIs, one or more buffering agents, a liquid media (*e.g*. water, de-ionized water, *etc*.), and one or more optional pharmaceutical excipients. Such compositions, upon storage in a closed container maintained at either room temperature, refrigerated (e.g. about 5-10 °C) temperature, or freezing temperature for a period of about 1, 2, 3, 4, 5, 6, 7, 8, 9, 10, 11, or 12 months, exhibit about 90%, about 92.5%, about 95%, or about 97.5% of the original first PPI and/or the one or more additional PPIs present therein.

Other suspension compositions comprise PPI-salt, one or more additional PPIs, one or more buffering agents, a liquid media (e.g. water, de-ionized water, *etc*.), and one or more optional pharmaceutical excipients. Such compositions, upon storage in a closed container maintained at either room temperature, refrigerated (e.g. about 5-10 °C) temperature, or freezing temperature for a period of about 1, 2, 3, 4, 5, 6, 7, 8, 9, 10, 11, or 12 months, exhibit about 90%, about 92.5%, about 95%, or about 97.5% of the original PPI-salt and/or the one or more additional PPIs present therein.

### Storage Stability

In one embodiment, compositions are in the form of a powder for suspension that is ultimately to be suspended in a liquid vehicle prior to administration to a subject. Liquid compositions comprising an acid labile PPI suspended in a liquid vehicle, without more, would typically exhibit relatively short periods of stability, even when maintained under refrigerated conditions. This is particularly inconvenient in the hospital setting as fresh batches of suspension are continually required. Suspension compositions of the disclosure are believed to exhibit improved storage stability.

Illustrative suspension compositions comprise a first PPI, one or more additional PPIs, at least one buffering agent, water, and one or more optional pharmaceutical excipients. Such compositions, upon storage in a closed container maintained at room temperature, refrigerated (e.g. about 5 to about 5-10 °C) temperature, or frozen for a period of about 1, 2, 3, 4, 5, 6, 7, 8, 9, 10, 11, or 12 months, may exhibit about 90%, about 92.5%, about 95%, or about 97.5% of the original first PPI and/or subsequent PPI present therein.

### Administration and Bioavailability

In one embodiment, compositions of the disclosure are suitable for rapid onset of therapeutic effect, particularly with respect to the PPI components. In another embodiment, upon oral administration of a composition to a subject, at least a therapeutically effective amount of at least one PPI is available for absorption and metabolism by the subject. The phrase "available for absorption" in reference to an active ingredient such as a PPI means that the ingredient remains intact and in active form in the stomach for a sufficient amount of time to allow for absorption into the blood. As discussed above, most commercially available PPIs require enteric coating to prevent exposure of the PPI to gastrointestinal fluids (and consequent drug degradation) by way of pH dependent coatings. Such coating, in turn, prevents rapid PPI absorption and therapeutic onset of action. Compositions of the present disclosure, by contrast, do not require but can optionally include an enteric coating to maintain drug stability in gastrointestinal fluids and thereby provide for rapid absorption and onset of therapeutic effect. In fact, in one embodiment, a composition comprises at least a therapeutically effective amount of at least one PPI that is not enteric coated. Another embodiment provides for compositions having at least one PPI which is optionally enteric coated.

In another embodiment, upon oral administration of a composition described herein to a plurality of fasted adult human subjects, the subjects exhibit an average Tₘₐₓ of active ingredient, (*e.g*. at least one PPI) within about 30 seconds to about 90 minutes, within about 1 minute to about 80 minutes, within about 5 minutes to about 60 minutes, within about 10 minutes to about 50 minutes, or within about 15 minutes to about 45 minutes.

In still another embodiment, upon administration of a composition described herein to a plurality of fasted adult human subjects, the subjects exhibit an average plasma concentration of at least one PPI of about 0.1 µg/ml, about 0.15 µg/ml, about 0.2 µg/ml, about 0.3 µg/ml, about 0.4 µg/ml, about 0.5 µg/ml, about 0.6 µg/ml, about 0.7 µg/ml, about 0.8 µg/ml, about 0.9 µg/ml, about 1 µg/ml, about 1.5 µg/ml, or about 2.0 µg/ml at any time within about 90 minutes, within about 75 minutes, within about 60 minutes, within about 55 minutes, within about 50 minutes, within about 45 minutes, within about 40 minutes, within about 35 minutes, within about 30 minutes, within about 25 minutes, within about 20 minutes, within about 17 minutes, within about 15 minutes, within about 12 minutes, or within about 10 minutes after administration.

In yet another embodiment, upon administration of a composition described herein to a plurality of fasted adult human subjects, the subjects exhibit a plasma concentration of active ingredient (*e.g.* at least one PPI) of about 0.1 µg/ml, about 0.15 µg/ml, about 0.2 µg/ml, about 0.3 µg/ml, about 0.4 µg/ml, about 0.5 µg/ml, about 0.6 µg/ml, about 0.7 µg/ml, about 0.8 µg/ml, about 0.9 µg/ml, about 1.0 µg/ml, about 1.5 µg/ml or about 2.0 µg/ml, maintained from about 15 minutes to about 60 minutes after administration, about 15 minutes after administration to about 90 minutes after administration, about 15 minutes to about 120 minutes after administration, or about 15 minutes to about 180 minutes after administration.

In another embodiment, upon administration of a composition described herein to a plurality of fasted adult human subjects, the subjects exhibit at least one of: a mean Cₘₐₓ of PPI1 and/or PPI2 of about 500 µg/ml to about 2000 µg/ml, about 600 µg/ml to about 1900 µg/ml, or about 700 ng/ml to about 1800 µg/ml; a mean Tₘₐₓ of PPI and/or PPI2 of about 0.15 to about 2 hours, about 0.25 to about 1.75 hours, or about 0.3 hours to about 1 hour; and/or a mean AUC_{(0-inf)} of PPI and/or PPI2 of about 1000 to about 3000 µg*hr/ml, about 1500 to about 2700 µg*hr/ml, or about 1700 to about 2500 µg*hr/ml.

In yet another embodiment, upon administration of a composition described herein to a plurality of fasted adult human subjects, the subjects exhibit at least one of: a mean Cₘₐₓ of the salt form of PPI (PPI-salt) and/or base form of PPI (PPI-base) of about 500 µg/ml to about 2000 µg/ml, about 600 µg/ml to about 1900 µg/ml, or about 700 ng/ml to about 1800 µg/ml; a mean Tₘₐₓ of PPI and/or PPI2 of about 0.15 to about 2 hours, about 0.25 to about 1.75 hours, or about 0.3 hours to about 1 hour; and/or a mean AUC_{(0-inf)} of PPI and/or PPI2 of about 1000 to about 3000 µg*/hr/ml, about 1500 to about 2700 µg*hr/ml, or about 1700 to about 2500 µg*hr/ml.

In another embodiment, upon administration of a composition described herein to a plurality of fasted adult human subjects, the subjects exhibit: a mean Cₘₐₓ of PPI1 and/or PPI2 of about 500 µg/ml to about 2000 µg/ml, about 600 µg/ml to about 1900 µg/ml, or about 700 µg/ml to about 1800 µg/ml; a mean Tₘₐₓ of PPI1 and/or PPI2 of about 0.15 to about 2 hours, about 0.25 to about 1.75 hours, or about 0.3 hours to about 1 hour; and a mean AUC_{(0-inf)} of PPI1 and/or PPI2 of about 1000 to about 3000 ng*hr/ml, about 1500 to about 2700 ng*hr/ml, or about 1700 to about 2500 ng*hr/ml.

In another embodiment, upon administration of a composition described herein to a plurality of fasted adult human subjects, the subjects exhibit: a mean Cₘₐₓ of PPI-salt and/or PPI-base of about 500 µg/ml to about 2000 µg/ml, about 600 µg/ml to about 1900 µg/ml, or about 700 µg/ml to about 1800 µg/ml; a mean Tₘₐₓ of PPI1 and/or PPI2 of about 0.15 to about 2 hours, about 0.25 to about 1.75 hours, or about 0.3 hours to about 1 hour; and a mean AUC_{(0-inf)} of PPI1 and/or PPI2 of about 1000 to about 3000 ng*hr/ml, about 1500 to about 2700 ng*hr/ml, or about 1700 to about 2500 ng*hr/ml.

### Parietal Cell Activators

In one embodiment, a composition of the present disclosure can further include one or more parietal cell activators. Parietal cell activators such as chocolate, calcium and sodium bicarbonate and other alkaline substances stimulate the parietal cells and enhance the pharmacologic activity of the PPI administered. For the purposes of this application, "parietal cell activator" or "activator" shall mean any compound or mixture of compounds possessing such stimulatory effect including, but not limited to, chocolate, sodium bicarbonate, calcium (for example, calcium carbonate, calcium bicarbonate, calcium gluconate, calcium hydroxide, calcium acetate and calcium glycerophosphate), peppermint oil, spearmint oil, coffee, tea and colas (even if decaffeinated), caffeine, theophylline, theobromine, and amino acids (particularly aromatic amino acids such as phenylalanine and tryptophan) and combinations thereof.

Parietal cell activators, if desired, are typically present in a composition of the disclosure in an amount sufficient to produce the desired stimulatory effect without causing untoward side effects to subjects. For example, chocolate, such as raw cocoa, is administered in an amount of about 5 mg to 2.5 g per 20 mg dose of omeprazole (or comparable pharmacologic dose of another proton pump inhibiting agent). The dose of activator administered to a subject, for example, a human, in the context of the present disclosure should be sufficient to result in enhanced effect of a PPI over a desired time frame.

Illustratively, the approximate effective ranges for various parietal cell activators per 20 mg dose of omeprazole (or comparable dose of other PPI) include, Chocolate (raw cocoa) - about 5 mg to about 2.5 g; Sodium bicarbonate - about 7 mEq to about 25 mEq; Calcium carbonate - about 1 mg to about 1.5 g; Calcium gluconate - about 1 mg to about 1.5 g; Calcium lactate - about 1 mg to about 1.5 g; Calcium hydroxide - about 1 mg to about 1.5 g; Calcium acetate - about 0.5 mg to about 1.5 g; Calcium glycerophosphate - about 0.5 mg to about 1.5 g; Peppermint oil - (powdered form) about 1 mg to about 1 g; Spearmint oil - (powdered form) about 1 mg to about 1 g; Coffee - about 20 ml to about 240 ml; Tea - about 20 ml to about 240 ml; Cola - about 20 ml to about 240 ml; Caffeine - about 0.5 mg to about 1.5 g; Theophylline - about 0.5 mg to about 1.5 g; Theobromine - about 0.5 mg to about 1.5g; Phenylalanine - about 0.5 mg to about 1.5 g; and Tryptophan - about 0.5 mg to about 1.5 g.

### Gastrointestinal disorders

In various embodiments, the present disclosure provides for therapy for various diseases and disorders. Such diseases and disorders include, *inter alia,* gastrointestinal disorders and, in particular, acid related gastrointestinal disorders. The phrase "acid related gastrointestinal disorder" or "acid related gastrointestinal disease" refers generally to a disease or disorder that occurs due to an imbalance between acid and pepsin production on the one hand, so-called aggressive factors, and mucous, bicarbonate, and prostaglandin production on the other hand, so-called defensive factors.

The term "therapy" as used herein refers to treatment and/or prevention of a disorder or disease, such as a gastrointestinal disorder.

The term "treat" or "treatment" as used herein refers to any treatment of a disorder or disease, and includes, but is not limited to, preventing the disorder or disease from occurring in a subject that may be predisposed to the disorder or disease but has not yet been diagnosed as having the disorder or disease; inhibiting the disorder or disease, for example, arresting the development of the disorder or disease; relieving the disorder or disease, for example, causing regression of the disorder or disease; or relieving the condition caused by the disease or disorder, for example, stopping the symptoms of the disease or disorder.

The term "prevent" or "prevention," in relation to a disorder or disease, means preventing the onset of gastrointestinal disorder or disease development if none had occurred, or preventing further disorder or disease development if the disorder or disease was already present.

Compositions of the present disclosure can be in the form of an orally deliverable dosage unit. The terms "oral administration" or "orally deliverable" herein include any form of delivery of a therapeutic agent or a composition thereof to a subject wherein the agent or composition is placed in the mouth of the subject, whether or not the agent or composition is swallowed. Thus, "oral administration" includes buccal and sublingual as well as esophageal administration.

Various disorders or diseases include, but are not limited to, nocturnal acid breakthrough (NAB), gastroparesis (slow stomach emptying), duodenal ulcer, gastric ulcer, stress erosions and ulceration, stress-related mucosal damage, gastric and duodenal erosions and ulceration, acid dyspepsia, gastroesophageal reflux disease (GERD), severe erosive esophagitis, poorly responsive symptomatic gastroesophageal reflux disease, acid reflux, heartburn, nighttime heartburn symptoms, esophageal ulcers and erosions, Barrett's esophagus, precancerous and cancerous lesions of the esophagus induced by acid exposure, acid hypersecretory conditions, gastrointestinal pathological hypersecretory conditions (such as Zollinger Ellison Syndrome), gastrointestinal bleeding, acute upper gastrointestinal bleeding, non-ulcer dyspepsia, heartburn, ulcers induced by NSAIDs, atypical reflux conditions, laryngitis, chronic cough, otitis media, sinusitis, eye pain, globus sensation, esophagitis, erosive esophagitis, adenocarcinoma of the esophagus, gastrinoma, *Helicobacter pylori* (*H. pylori*) infection, irritable bowel syndrome (IBS), inflammatory bowel disease (IBD), ulcerative colitis, pre- and post-operative acid aspiration, Crohn's disease, asthma, laryngitis, sleep apnea, sleep disturbance, psoriasis, intensive care therapy, and diseases related to any of the above-mentioned conditions.

The foregoing lists of disorders or diseases are meant to be illustrative and not exhaustive as a person of ordinary skill in the art would recognize that there are many other disorders or diseases to which the embodiments of the present disclosure could treat and/or prevent.

In one embodiment, compositions provide a method for treating and/or preventing a disorder or disease by administering a pharmaceutical composition comprising a first and a second proton pump inhibitor wherein the first proton pump inhibitor, having a therapeutically effective portion which is optionally enteric coated, has an elimination rate which is different from the elimination rate of the second proton pump inhibitor, having a therapeutically effective portion which is optionally enteric coated, and a buffering agent, such as sodium bicarbonate.

In another embodiment, compositions provide a method for treating and/or preventing a disorder or disease by administering a pharmaceutical composition comprising a salt and a free base form of a proton pump inhibitor wherein the proton pump inhibitor has a therapeutically effective portion which is optionally enteric coated, and a buffering agent, such as sodium bicarbonate.

In still another embodiment, compositions provide a method for treating and/or preventing a disorder or disease by administering a pharmaceutical composition comprising tenatoprazole, having a therapeutically effective portion which is optionally enteric coated, and a buffering agent.

In yet another embodiment, compositions provide a method for treating and/or preventing a disorder or disease by administering a pharmaceutical composition comprising tenatoprazole, having a therapeutically effective portion which is optionally enteric coated, omeprazole, having a therapeutically effective portion which is optionally enteric coated, and a buffering agent.

In still another embodiment, compositions provide a method for treating and/or preventing a disorder or disease by administering a pharmaceutical composition comprising the salt form of tenatoprazole, having a therapeutically effective portion which is optionally enteric coated, the base form of tenatoprazole, having a therapeutically effective portion which is optionally enteric coated, and a buffering agent.

In another embodiment, compositions provide a method for treating and/or preventing a disorder or disease by administering a pharmaceutical composition comprising the sodium hydrate salt form of tenatoprazole, having a therapeutically effective portion which is optionally enteric coated, the free base form of tenatoprazole, having a therapeutically effective portion which is optionally enteric coated, and a buffering agent. For example, one embodiment combines tenatoprazole sodium hydrate (about 40 mg tenatoprazole), tenatoprazole in free base form (about 40 mg tenatoprazole) and sodium bicarbonate (about 20 mEq).

Yet another embodiment of the disclosure provides for a composition comprising a short acting PPI, having a therapeutically effective portion which is optionally enteric coated (40 mg), the sodium hydrate form of a PPI, having a therapeutically effective portion which is optionally enteric coated (about 20 mg), and a buffering agent (about 20 mEq).

Another embodiment of the disclosure provides for a composition comprising omeprazole, having a therapeutically effective portion which is optionally enteric coated (40 mg), tenatoprazole sodium hydrate, having a therapeutically effective portion which is optionally enteric coated (about 20 mg tenatoprazole), and sodium bicarbonate (about 20 mEq).

Still another embodiment of the disclosure provides for a composition comprising tenatoprazole sodium hydrate, having a therapeutically effective portion which is optionally enteric coated (about 80 mg tenatoprazole) and sodium bicarbonate (about 20 mEq).

Yet another embodiment of the disclosure provides for a composition comprising tenatoprazole free base, having a therapeutically effective portion which is optionally enteric coated (about 80 mg tenatoprazole) and sodium bicarbonate (about 20 mEq).

In another embodiment, compositions provide a method for treating and/or preventing a disorder or disease by administering a pharmaceutical composition comprising a first and a second proton pump inhibitor wherein the first proton pump inhibitor, having a therapeutically effective portion which is optionally enteric coated, has an elimination rate which is different from the elimination rate of the second proton pump inhibitor, having a therapeutically effective portion which is optionally enteric coated, buffering agent, and optionally any combination of one or more of the following: L-carnosine, H2 blockers, NSAIDs, anti-H. pylori active substances, protein components, motility agents, iron, Vitamin B₁₂, pentagastrin or other pharmaceutical agents.

In still another embodiment, compositions provide a method for treating and/or preventing a disorder or disease by administering a pharmaceutical composition comprising the salt form of tenatoprazole, having a therapeutically effective portion which is optionally enteric coated, the base form of tenatoprazole, having a therapeutically effective portion which is optionally enteric coated, and optionally any combination of one or more of the following: L-carnosine, H2 blockers, NSAIDs, anti-H. pylori active substances, protein components, motility agents, iron, Vitamin B₁₂, pentagastrin or other pharmaceutical agents.

In still another embodiment, compositions provide a method for treating and/or preventing *H. pylori* infection by administering a pharmaceutical composition a comprising first and a second proton pump inhibitor wherein the first proton pump inhibitor, having a therapeutically effective portion which is optionally enteric coated, has an elimination rate which is different from the elimination rate of the second proton pump inhibitor, having a therapeutically effective portion which is optionally enteric coated, one or more buffering agents, and one or more anti-*H*. *pylori* active substances.

In yet another embodiment, compositions provide a method for treating and/or preventing *H. pylori* infection administering a pharmaceutical composition comprising the salt form of tenatoprazole, having a therapeutically effective portion which is optionally enteric coated, the base form of tenatoprazole, having a therapeutically effective portion which is optionally enteric coated, one or more buffering agents, and one or more anti-*H*. *pylori* active substances.

Another embodiment provides for a method for treating and/or preventing a disorder or disease by administering a pharmaceutical composition comprising tenatoprazole, esomeprazole (or other PPI), and a buffering agent in an enteric coated formulation. Various embodiments may further include any combination of one or more of the following: L-carnosine, H2 blockers, NSAIDs, anti-H. pylori active substances, protein components, motility agents, iron, Vitamin B₁₂, pentagastrin or other pharmaceutical agents.

In another embodiment, either or both PPIs could be enteric coated as tablets.

In various embodiments, the acid labile pharmaceutical agents can be mixed together and then enteric-coated as granules.

In still another embodiment, tenatoprazole can be enteric coated as granules and the other one or more proton pump inhibitors could be enteric coated as granules.

In yet another embodiment of the disclosure, compositions provide a method for treating and/or preventing a disorder or disease by administering a pharmaceutical composition comprising an H2 blocker, at least one proton pump inhibitor and, optionally, a buffering agent. For example, an exemplary method of the disclosure administers a pharmaceutical composition comprising at least one proton pump inhibitor (PPI), having a therapeutically effective portion which is optionally enteric coated; an H2 blocker; and optionally one or more of the following: buffering agents, L-carnosine, H2 blockers, NSAIDs, anti-H. pylori active substances, protein components, motility agents, iron, Vitamin B₁₂, pentagastrin or more other pharmaceutical agents.

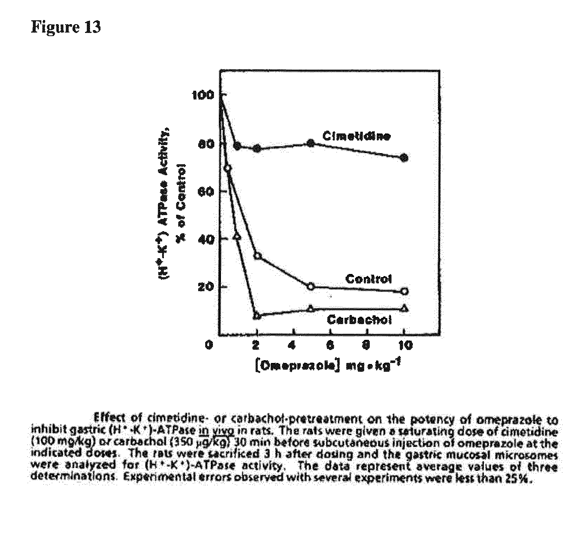

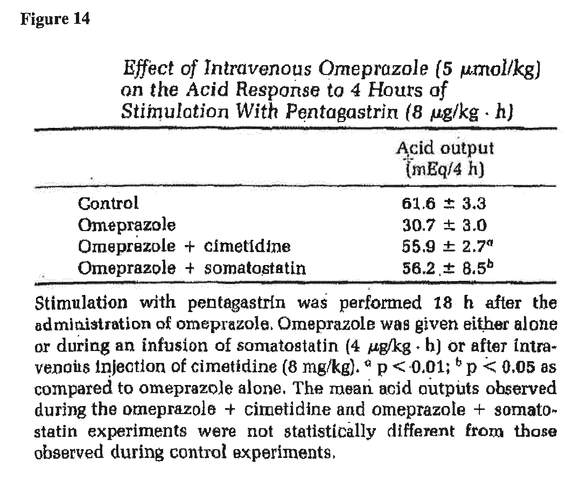

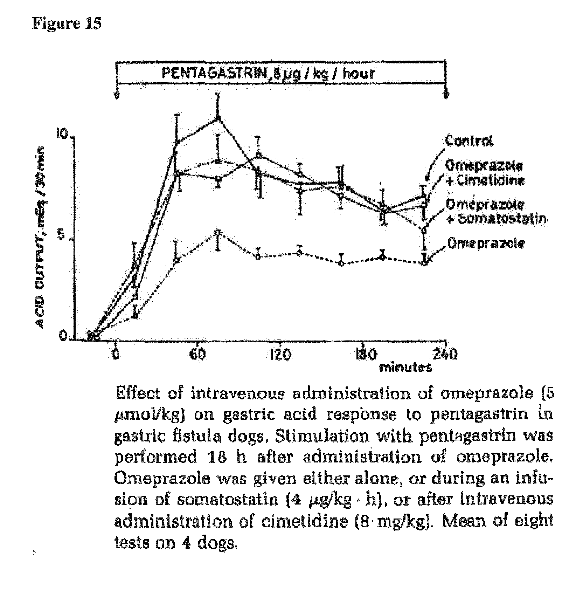

This finding is unexpected, and is taught away from by current references. For example, the textbook by Sleisinger and Fordtran, *Gastrointestinal and Liver Disease,* 6th edition, teaches "[c]ontinuing H2RAs during a transition period to a PPI will be counterproductive. Combination therapy with H2RAs is never appropriate." In addition, studies teach that there is no difference on acid output between a placebo and a composition comprising an H2 Blocker and a PPI, such as cimetidine and omeprazole. *See* W.B. Im, 260 J Biol. Chem. 4591-4597 (1985); De Graef, J et al., 91 Gastroenterology 333-337 (1986); and Figures 13-15.

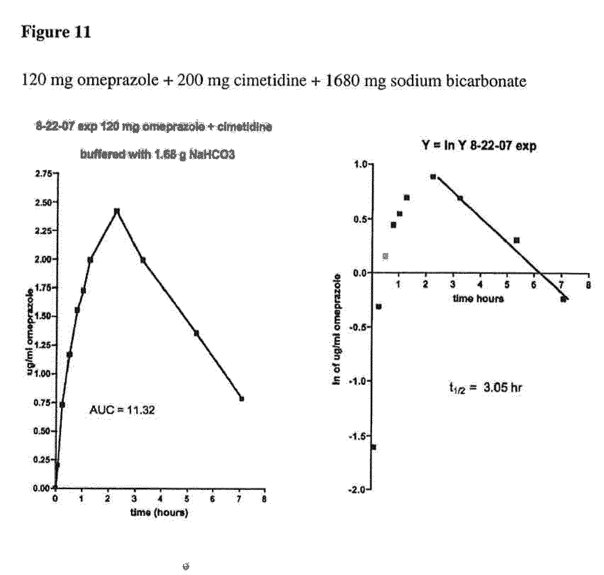

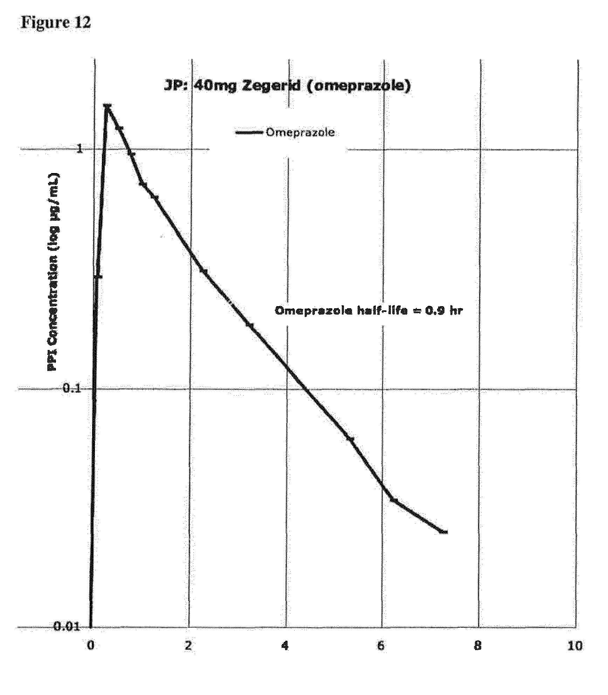

The present disclosure demonstrates that the half-life of omeprazole is three hours when administered with cimeditine, instead of just about one hour when administered without an H2 Blocker. *See* Figures 11 and 12. Based on studies with 120 mg omeprazole, 200 mg cimetidine and 1680 mg sodium bicarbonate, pH measurements indicated dramatically less acid output as measured by integrated acidity of 55.

Integrated gastric acidity is calculated as the cumulative time-weighted average of the gastric acid concentration. Integrated gastric acidity is sensitive to the change from baseline for gastric acidity, whereas mean or median gastric pH has low sensitivity in detecting change (*i.e.,* drug induced) from baseline. F.F.I. Rebecchi *et al.* investigated the prognostic value of the area under the H⁺ curve (i.e., integrated acidity) in 36 healthy volunteers and 60 GERD patients. Based on a receiver operating characteristic analysis, the authors found that an area under the curve of hydrogen ion activity of above 100 mmol/L.min was very sensitive and specific (100% and 97%, respectively) for identifying patients with erosive esophagitis, and also had a good sensitivity and specificity (76% and 93%) for identifying patients with non-erosive GERD. *See* F.F.I. Rebecchi et al., Improving the analysis of esophageal acid exposure by a new parameter: area under H+. 97 Am. J. Gastroenterol. 568-74 (2002); J.D. Gardner et al., Determination of the reduction in gastric acidity necessary to prevent pathological oesophageal reflux in patients with gastro-oesophageal reflux disease treated with a proton pump inhibitor. 17 Aliment. Pharmacol. Ther. 955-64 (2003).

The graphs in Figure 11 reveal that the combination treatment of omeprazole and an H2 blocker produces an immediate and long-lasting effect on gastric pH, beginning within the first half hour and continuing for 24 hours. In contrast, using a PPI in isolation or an H2 blocker in isolation has little or no effect on gastric pH on the first day of administration.

In another embodiment, a composition of the disclosure comprises a PPI, a buffering agent, an optional H2 blocker and pentagastrin in an amount of about 2.5 mg to about 20 mg a day.

In still another embodiment, compositions provide a method for treating and/or preventing a disorder or disease by administering a pharmaceutical composition comprising a PPI, a buffering agent, an optional H2 blocker and pentagastrin in an amount of about 2.5 mg to about 20 mg a day.

In another embodiment, compositions of the disclosure are part of a "starter packet," with which patients begin therapy. The starter packet comprises composition sufficient for treating patients, with or without gastroparesis, in need of therapy from a gastrointestinal disorder for five days.

### Inhibition, Superinhibition and Successive Superinhibition

In various embodiments of the disclosure, the onset of effect of acid inhibition may be influenced, *inter alia,* by the release of proton pump inhibitor from the dosage form into the blood stream, which also may influence the amount of the proton pump inhibitor in systemic circulation. In some embodiments, the onset of effect of acid inhibition may be affected by factors that include, but are not limited to: the time required for sufficient concentrations of proton pump inhibitor to migrate from the blood stream to the parietal cell secretory canalicular lumen, the amount of acid present in the canalicular lumen, or the binding to proton pumps in the parietal cell secretory canalicular luminal membrane.

In one embodiment, an immediate release formulation of a proton pump inhibitor, such as omeprazole or lansoprazole, with a buffer allows for the rapid release of proton pump inhibitors into systemic circulation.

In another embodiment, a rapid release of proton pump inhibitor into systemic circulation is achieved by, *inter alia,* administering proton pump inhibitor in formulations that include, but are not limited to: "thin enteric coated" granules in the presence of buffer, enteric coated granules in the presence of buffer, enteric coated granules, or tablets.

In various embodiments, non-limiting examples of proton pump inhibitors with a rapid onset of effect include, but are not limited to: rabeprazole, lansoprazole, omeprazole, esomeprazole, or pantoprazole.

In another embodiment, a particular example of a proton pump inhibitor with a slow onset of effect includes, but is not limited to, tenatoprazole.

The foregoing list of factors, formulations, inhibitors and compositions that could affect the onset of effect of acid inhibition is meant to be illustrative and not exhaustive as a person of ordinary skill in the art would recognize that other factors, formulations, inhibitors and compositions could affect the onset of effect of acid inhibition.

In various embodiments of the disclosure, the duration of the effect of acid inhibition may be influenced by factors that include, without limitation: the duration of time proton pump inhibitor is in systemic circulation, the concentration of proton pump inhibitor, or the amount of proton pumps available for binding (in the "active state").

In other embodiments of the disclosure, the duration of proton pump inhibitor in systemic circulation is prolonged by factors that include, without limitation: pharmaceutical compositions with longer half-lives, enteric coated dosage forms (including but not limited to thin enteric coat, enteric coated granule, enteric coated tablet), or sustained release dosage forms.

In various embodiments of the disclosure, the concentration of proton pump inhibitor is increased by methods that include, but are not limited to: increasing the dose of pharmaceutical composition or administering one or more proton pump inhibitors with a buffer in a non-enteric coated form.

In one embodiment of the disclosure the amount of proton pumps in the active state is increased by methods that include, *inter alia,* administering a buffer with the proton pump inhibitor.

The foregoing list of factors, formulations and compositions that could affect the duration of the effect of acid inhibition is meant to be illustrative and not exhaustive as a person of ordinary skill in the art would recognize that other factors, formulations and compositions could affect the duration of the effect of acid inhibition.

Current methods that administer a dosage of proton pump inhibitor every 24 hours provide that a significant portion of proton pumps can be blocked, but then subsequent pump activity can diminish for that dosing period because there is a "fixed number" of proton pumps available to be blocked each day. If an insufficient number of pumps are blocked by a dosage of proton pump inhibitor, then the effects of the initial dosage of proton pump inhibitor will greatly diminish throughout the dosing interval.

"Superinhibition" of proton pumps is achieved by maximal inhibition of proton pumps during a period of significant proton pump activity. In one embodiment, superinhibition is achieved by maximizing the amount of proton pump inhibitor in the parietal cell during the time when the proton pumps of the parietal cell are in the active state.

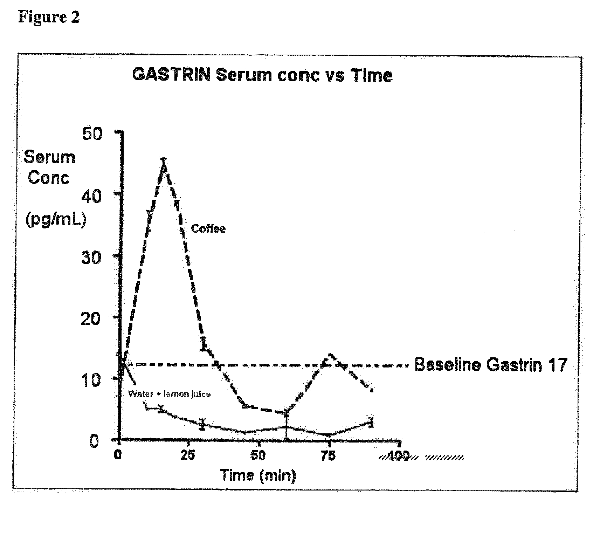

"Successive superinhibition" may be achieved when a significant number of proton pumps are blocked by an initial dose of an acid labile pharmaceutical agent and then sufficient amount of proton pump inhibitor remains present in the body to correspond to subsequent stimulation of proton pumps. In various embodiments, the embodiments disclosed herein achieve periods of successive superinhibition. See Figure 1 and Figure 2.

In one embodiment, successive superinhibitions may be achieved by administering a composition comprising at least two different proton pump inhibitors with differing elimination profiles, and at least one of the proton pump inhibitors has a therapeutically effective portion which is optionally enteric coated.

In another embodiment, a substantial number of proton pumps may be blocked by a proton pump inhibitor with a shorter elimination rate. Additional proton pumps may be blocked by administering one or more additional proton pump inhibitors with a longer elimination rate that corresponds with this subsequent stimulation of proton pumps.

In various embodiments, a substantial number of proton pumps may be activated by administering a composition comprising a rapid-acting buffering agent. In one embodiment, the rapid-acting buffering agent is sodium bicarbonate. Since only the activated proton pumps are capable of being blocked, then the more proton pumps activated, the more pumps are blocked. Sodium bicarbonate is a particular activator of proton pumps. Serum concentrations of proton pump inhibitor, which are reflective of proton pump inhibitor concentrations in the parietal cell cytosol and hence the amount of proton pump inhibitor reaching the site of action at the parietal cell secretory canalicular luminal surface, the site of the activated proton pumps, should overlay, from the perspective of time, the parietal cell activation profile in order for successive superinhibitions to be possible.

In another embodiment, successive superinhibitions may be achieved by administering a composition comprising a buffer, an immediate release proton pump inhibitor, having a therapeutically effective portion which is optionally enteric coated, and a delayed release proton pump inhibitor, having a therapeutically effective portion which is optionally enteric coated.

For example, successive superinhibitions may be achieved by administering a pharmaceutical composition comprising about 5 mg to about 80 mg non-enteric coated esomeprazole magnesium, about 5 mg to about 80 mg enteric coated esomeprazole magnesium and about 5 mEq to about 50 mEq sodium bicarbonate. In another embodiment, the mixture of the previous sentence comprises about 40 mg non-enteric coated esomeprazole magnesium, about 40 mg enteric coated magnesium and about 20 mEq sodium bicarbonate.

In another embodiment, successive superinhibitions may be achieved by administering a pharmaceutical composition comprising about 5 mg to about 80 mg non-enteric coated crushed or powderized esomeprazole magnesium granules, about 5 mg to about 80 mg enteric coated whole esomeprazole magnesium and about 5 mEq to about 50 mEq sodium bicarbonate. In another embodiment, the mixture of the previous sentence comprises about 40 mg non-enteric coated esomeprazole magnesium, about 40 mg enteric coated magnesium and about 20 mEq sodium bicarbonate.

In yet another embodiment, successive superinhibitions may be achieved by administering a composition comprising at least two different proton pump inhibitors with differing onset of activity profiles, and at least one of the proton pump inhibitors has a therapeutically effective portion which is optionally enteric coated.

For example, successive superinhibitions may be achieved by administering a pharmaceutical composition comprising about 5 mg to about 100 mg non-enteric coated omeprazole, about 5 mg to about 100 mg non-enteric coated tenatoprazole and about 5 mEq to about 50 mEq sodium bicarbonate. In another embodiment, the mixture of the previous sentence comprises about 40 mg non-enteric coated omeprazole, about 40 mg non-enteric coated tenatoprazole and about 20 mEq sodium bicarbonate.

In various embodiments, successive superinhibitions may be achieved by administering a composition comprising a first proton pump inhibitor, having a therapeutically effective portion which is optionally enteric coated, at least one additional proton pump inhibitor, and a buffering agent.

In other embodiments, successive superinhibitions may be achieved by administering a composition comprising the slat form of a proton pump inhibitor, having a therapeutically effective portion which is optionally enteric coated, the free base form of a proton pump inhibitor, and a buffering agent.

### Multi-Chambered Apparatus

Embodiments of this disclosure also provide for a multi-chambered apparatus comprising (a) multiple chambers wherein one chamber comprises a pharmaceutically active agent, an optional protective buffer and/or an optional thickener, a second chamber comprises one or more flavoring agents, sweeteners, disintegrants, additives, buffering agents, or thickeners, and the formulations in the chambers can be in the form of a suspension, liquid, reagent, solution or dry formulations constituted with water or other suitable liquid; (b) a dispenser head with multiple pumps that offer volumetric dispensing; and (c) connectors connecting the dispenser head to the chambers.

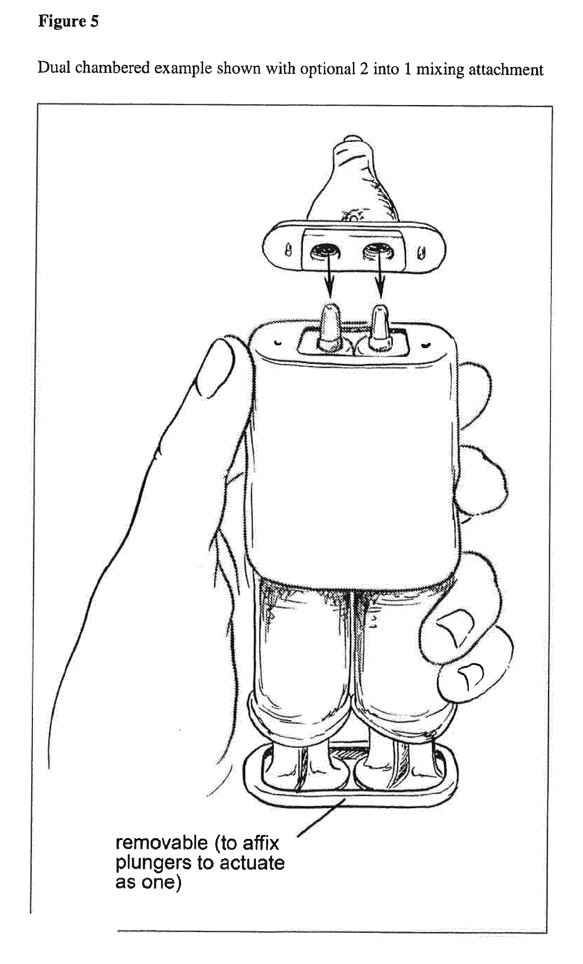

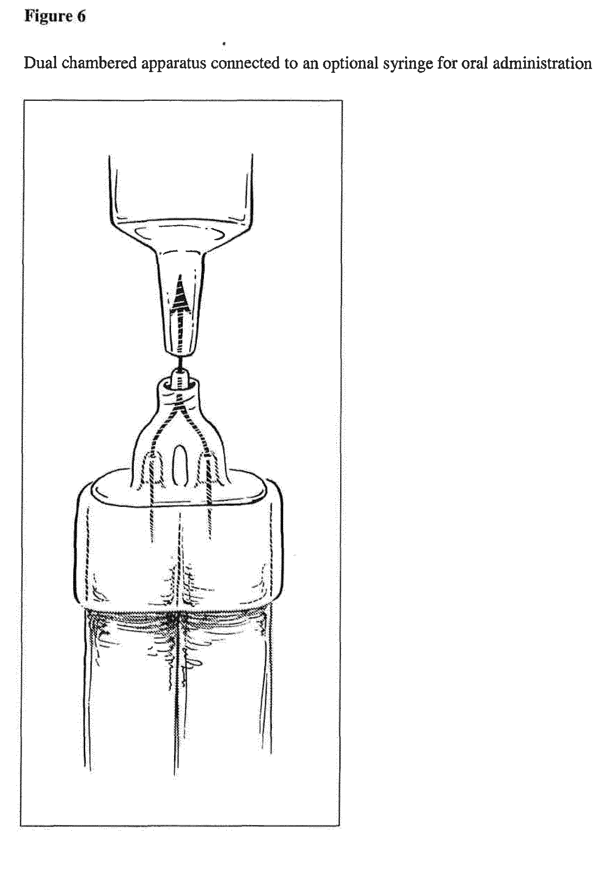

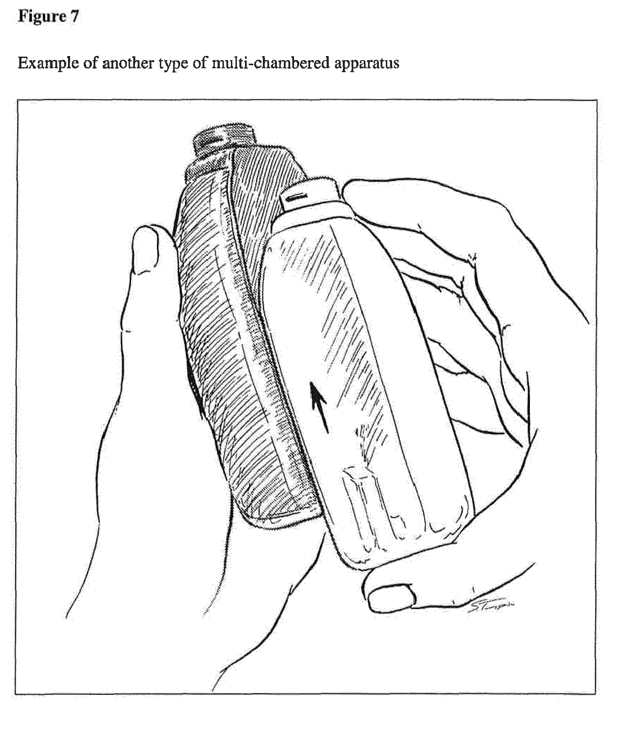

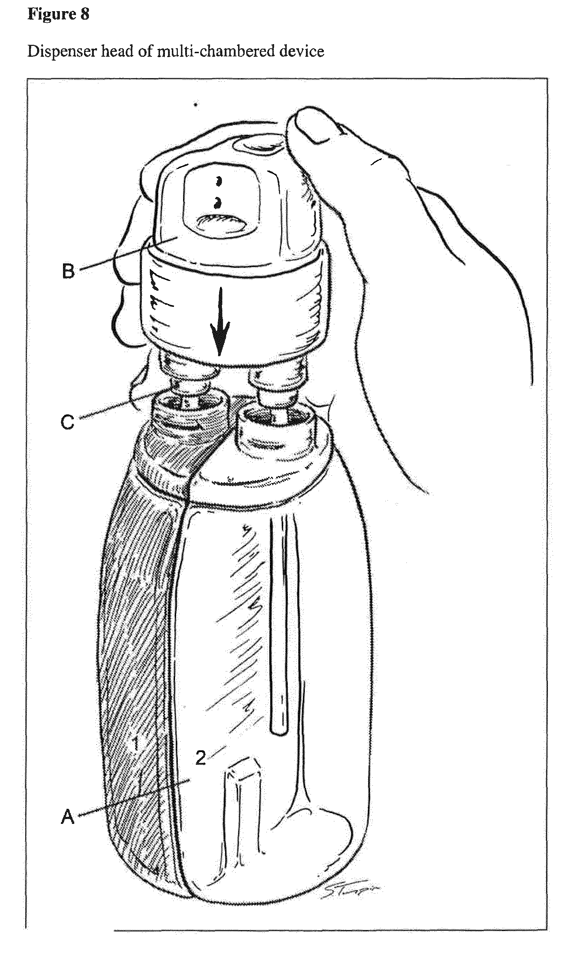

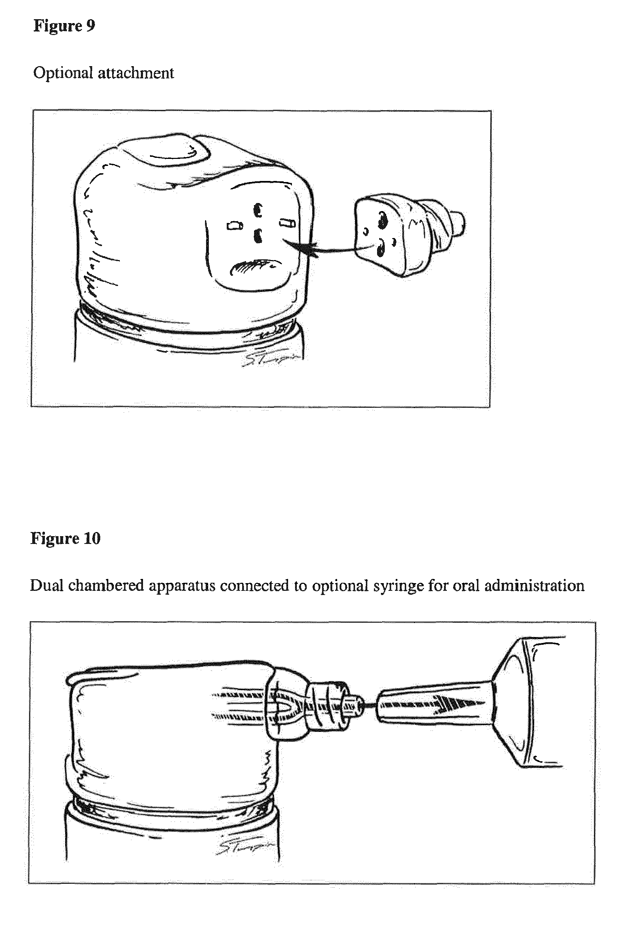

By way of illustration, Figures 5 through 8 each show an example of a multi-chambered dispenser. In one embodiment, the present disclosure provides for an apparatus comprising two syringe bodies and a component that attaches the plungers together. See, for example, Figures 5 and 6. In another embodiment, the component that attaches the plungers is optionally removable for cleaning and reuse. In other embodiments, the apparatus pf the present disclosure comprises an optionally removable component that combines the output from the two chambers and delivers it into a dosing syringe. See Figures 7 through 10.

In another embodiment, the apparatus is a multi-chambered syringe wherein one chamber comprises a pharmaceutically active agent, an optional protective buffer and/or an optional thickener, a second chamber comprises one or more flavoring agents, sweeteners, disintegrants, additives, buffering agents, or thickeners, and the formulations in the chambers can be in the form of a suspension, liquid, reagent, solution or dry formulations constituted with water or other suitable liquid.

In one embodiment, the present disclosure provides a method or process for pre-preparing multidosing formulations for oral administration of pharmaceuticals, additives, excipients, buffering agents, suspensions, liquids, reagents, solutions or dry formulations constituted with water or other suitable liquid.

In various embodiments, pharmaceuticals that can be used with the apparatus include one or more acid labile pharmaceutical agents, one or more proton pump inhibitors, or one or more of the following: L-carnosine, H2 blockers, NSAIDs, anti-H. pylori active substances, protein components, motility agents, iron, Vitamin B₁₂, pentagastrin or other pharmaceutical agents. The pharmaceuticals can be stored separately until dosing, at which time the antibiotics can be dispensed out according to the preset volume ratio.

Other embodiments disclosed herein provide for the administration of two or more pharmaceutical in one dose for multiple doses. For example, when two types of antibiotics, such as amoxicillin/clarithromycin, azithromycin/moxifloxacin, azithromycin/sulfisoxazole, or cefuroxime/levofloxacin, need to administered in one dose for multiple doses over a extended period, one antibiotic of the pair can be contained in the first chamber, while the remaining antibiotic in the second chamber.

In another embodiment, the method employing a multi-chambered dispenser with at least two chambers separated from each other and a dispenser head with a volumetric dial for preparing multidosing formulations comprises the steps of: (1) loading a first pharmaceutical, additive, buffering agent, suspension, liquid, reagent or solution in a first chamber; (2) optionally loading a liquid or suspension form of a second pharmaceutical, additive, buffering agent, suspension, liquid, reagent or solution in a second chamber; (3) optionally loading one or more additional pharmaceuticals, additives, excipients, buffering agents, suspensions, liquids, reagents or solutions in one or more additional chambers; (4) setting the dial to a predetermined volume ratio for multiple chambers; and (5) operating the dispenser head to dispense a mixture from the first and each additional chambers for oral administration at each dosing. Because the chambers are separate, loading of each chamber can be done before the addition of water to constitute the product as a liquid suspension. Pre-loading is the action of adding the dry contents of the formulation to their perspective chambers. See Figures 5 through 10.

In still another embodiment, the present disclosure provides for a multiple-dose suspension product for administering one or more proton pump inhibitors. For example, in the first chamber, loading a PPI in a suspension with a pH ranging from about 7, about 6.5 to about 15, or about 8 to about 12; and in the second chamber, loading a buffer solution with a pH ranging from about 6, about 5 to about 10, or about 6.5 to about 8.5.

In another embodiment, a third chamber can be employed, to administer additives such as flavoring additives. At each dosing, the volumetric dial can be set to an appropriate volume ratio between two or more chambers.

In still another embodiment, the present disclosure provides examples and proofs that a suspension of proton pump inhibitor with a pH greater than about 10 can be stored with minimum degradation for an extended period as compared to a suspension of proton pump inhibitor with a lower pH.

The various embodiments disclosed herein help achieve a multiple-dose administration product, enhanced stability of a pharmaceutical agent, and reduced interference of additives, excipients, buffering agents, suspensions, liquids, reagents or solutions with the pharmaceutical agent.

Furthermore, the apparatus and its method of use can be used for administering pharmaceuticals for the therapy of a variety of disorders or diseases in patients (with or without gastroparesis including but not limited to: NAB, gastrointestinal disorders, intensive care therapy, and diseases related to any of the above-mentioned conditions.

### Veterinary Indications

Gastrointestinal disorders such as active duodenal ulcers, gastric ulcers, and GERD are disorders that impact animals as well as humans. Horses are particularly susceptible to such gastrointestinal disorders. Studies revealed that 80% - 90% of racehorses in training have gastric ulcers. Performance horses that are stabled on high grain low roughage diets also experience a high incidence of ulcers. Moreover, additional studies have shown that 60 - 70% of event horses, 50 - 60% of endurance horses and 50 - 60% of show horses have ulcers. In fact, it has been estimated that up to 50% of all foals will have gastric ulcers and that all horses will have some degree of gastric ulceration at sometime in their life.

It has been has proposed that the major cause of gastric ulcers in the horse is prolonged exposure of the squamous mucosal lining to gastric acid. The stomach of the horse is essentially separated into two regions covered by different cell types. The upper area is called the non-glandular or squamous region, while the lower portion is known as the glandular portion of the stomach. The glands lining this lower portion secrete pepsin, an enzyme, and hydrochloric acid, both of which are responsible for the breakdown of undigested feed. Additionally these glands secrete bicarbonate, a buffer, and mucus. These two secretions are protective of the stomach lining. The majority of ulcers occur in the upper or non-glandular portion of the stomach. The only protection that this portion of the stomach has from gastric acid and pepsin comes from saliva production. If adequate saliva is not produced to buffer the gastric acid and coat the surface of this part of the stomach, then gastric irritation occurs and ulcers may develop.

Turning a horse out to graze without work for a month may help heal some ulcers, but this option is often not practical. Therefore, treating ulcers involves either inhibiting gastric acid secretion or neutralizing the acid produced. There are two classes of drugs that can be used to inhibit gastric acid secretion, histamine type-2 (H₂) antagonists and substituted benzimidazoles.

H₂ antagonists act by competing for histamine type-2 receptor sites on the parietal cell and thus blocking histamine-stimulated gastric acid secretion. The two most popular H₂ antagonists used in horses are cimetidine (Tagamet) and ranitidine (Zantac). These are widely used in humans and foals, but the adult horse dose is expensive and can't be used right up to racing in standardbreds. A ranitidine paste for horses (Ulcerguard®) is available, but, disadvantageously, it must be administered several times per day because of its rapid clearance.

Direct inhibition of the proton pump can be achieved by a class of substituted benzimidazoles known as proton pump inhibitors or PPIs. At neutral pH, these PPIs are chemically stable, lipid-soluble compounds that have little or no inhibitory activity. Unfortunately, most commercially available PPIs are unstable at neutral or acidic pH and undergo decomposition in gastrointestinal fluid upon oral administration, thereby resulting in loss of therapeutic activity.

To overcome this acid instability, such compounds have formulated for oral delivery to humans as enteric coated solid dosage forms, for example enteric coated tablets; the enteric coating protects the drug from contact with acidic stomach secretions. An undesirable consequence of such enteric coating is that therapeutic onset time is significantly delayed by comparison with non-enteric coated dosage forms. Such prolonged time to therapeutic onset would also be particularly undesirable for animals in need of rapid relief from one or more of the above described disorders or symptoms. Enteric coated formulations are also expensive and time consuming to manufacture, and require elaborate technology

The only proton pump inhibitor currently approved for horses is omeprazole (Gastrogard®). Gastrogard® is a flavored paste that is administered in an adjustable dose syringe. Unfortunately, the high cost of this product prohibits widespread use in adult horses. Furthermore, syringe administration to a horse or companion animal can be difficult to accomplish. Therefore, there is a significant unmet need in the treatment of gastrointestinal disorders in animals, for example horses.

Therefore, embodiments of the disclosure provide for a composition for administration to a horse, as set forth below in Example 17.

### EXAMPLES

The examples below are for illustrative purposes only and are not to be construed as limiting the invention in any manner.

### Example 1

Compositions can be prepared including one or more of an immediate-release component, a delayed immediate release component or a timed release component. For this example, the following abbreviations apply.

**IR**: Immediate-release means that this portion of the composition is formulated so that delivery begins in the stomach and there is no enteric coating or timed release coating involved. The term is intended to refer to any PPI formulation in which all or part of the PPI is in solution either before administration or immediately (i.e., within about 30 minutes) after administration. For example, with an "immediate release" formulation, oral administration results in immediate release of the agent from the composition into gastric fluid.

**DIR**: Delayed immediate-release means that release of this component of the formulation begins in the stomach but is delayed by comparison with an IR component (such delay is dependent upon pH induced dissolution of enteric coat or thin enteric coat).

**DR**: Delayed release means that release of this component of the formulation begins at a time other than promptly after administration. Delayed release formulations includes any nonimmediate release formulation, including but not limited to, film-coated formulations, enteric-coated formulations, encapsulated formulations, sustained release formulations and pulsatile release formulations. For delayed release formulations, the rate of release of drug from the dosage form is the rate-limiting step in the delivery of the drug to the target area.

**TR**: Timed release means that there is a pH independent time period that must pass before dissolution of the coating occurs.

The following composition types can be prepared according to various embodiments of the present disclosure.

Composition Type 1 contains: PPI1 immediate-release + PPI2 immediate-release and buffering agent (PPI1 and PPI2 delivery begins in stomach). The components of such a composition include: **IR** PPI1 + **IR** PPI2 + Buffer. Such a composition can comprise other components as described in detail above.

Composition Type 2 contains: PPI-salt immediate-release + PPI-base immediate-release and buffering agent. The components of such a composition include: **IR** PPI-salt + **IR** PPI-base + Buffer. Such a composition can comprise other components as described in detail above.

Composition Type 3 contains: PPI1 immediate-release with buffering agent (PPI1 delivery begins in stomach) + PPI2 enteric-coated or thin enteric coated. The components of such a composition include: **IR** PPI1 + **DIR** PPI2 + Buffer. Such a composition can comprise other components as described in detail above.

Composition Type 4 contains: PPI-salt immediate-release with buffering agent + PPI-base enteric-coated or thin enteric coated. The components of such a composition include: **IR** PPI-salt + **DIR** PPI-base + Buffer. Such a composition can comprise other components as described in detail above.

Composition Type 5 contains: PPI-salt enteric-coated or thin enteric coated with buffering agent + PPI-base immediate release. The components of such a composition include: **DIR** PPI-salt + **IR** PPI-base + Buffer. Such a composition can comprise other components as described in detail above.

Composition Type 6 contains: PPI1 immediate-release with buffering agent for gastric delivery (PPI1 delivery begins in stomach) + PP12 delayed-release. The components of such a composition include: **IR** PPI1 + **DR** PPI2 + Buffer. Such a composition can comprise other components as described in detail above.

Composition Type 7 contains: PPI-salt immediate-release with buffering agent + PPI-base delayed-release. The components of such a composition include: **IR** PPI-salt + **DR** PPI-base + Buffer. Such a composition can comprise other components as described in detail above.

Composition Type 8 contains: PPI-salt delayed-release with buffering agent + PPI-base immediate-release. The components of such a composition include: **DR** PPI-salt + **IR** PPI-base + Buffer. Such a composition can comprise other components as described in detail above.

Composition Type 9 contains: PPI1 immediate-release with buffering agent for gastric delivery (PPI1 delivery begins in stomach) + PPI2 timed-release (PPI2 delivery begins in duodenum, e.g. 1.5 to 2 hrs after ingestion). The components of such a composition include: **IR** PPI1 + **TR** PPI2 + Buffer. Such a composition can comprise other components as described in detail above.

Composition Type 10 contains: PPI-salt immediate-release with buffering agent + PPI-base timed-release. The components of such a composition include: **IR** PPI-salt + **TR** PPI-base + Buffer. Such a composition can comprise other components as described in detail above.

Composition Type 11 contains: PPI-salt timed-release with buffering agent + PPI-base immediate-release. The components of such a composition include: **TR** PPI-salt + **IR** PPI-base + Buffer. Such a composition can comprise other components as described in detail above.

Composition Type 12 contains: PPI1 enteric-coated or thin enteric-coated with buffering agent for gastric delivery + PPI2 enteric-coated or thin enteric coateded. The components of such a composition include: **DIR** PP1 + **DIR** PPI2 + Buffer. Such a composition can comprise other components as described in detail above.

Composition Type 13 contains: PPI-salt enteric-coated or thin enteric-coated with buffering agent + PPI-base enteric-coated or thin enteric coated. The components of such a composition include: **DIR** PPI-salt + **DIR** PPI-base + Buffer. Such a composition can comprise other components as described in detail above.

Composition Type 14 contains: PPI1 enteric-coated or thin enteric-coated with buffering agent for gastric delivery + PPI2 delayed release. The components of such a composition include: **DIR** PPI1 + **DR** PPI2 + Buffer. Such a composition can comprise other components as described in detail above.

Composition Type 15 contains: PPI-salt enteric-coated or thin enteric-coated with buffering agent + PPI-base delayed release. The components of such a composition include: **DIR** PPI-salt + **DR** PPI-base + Buffer. Such a composition can comprise other components as described in detail above.

Composition Type 16 contains: PPI-salt delayed release with buffering agent + PPI-base enteric-coated or thin enteric-coated. The components of such a composition include: **DR** PPI-salt + **DIR** PPI-base + Buffer. Such a composition can comprise other components as described in detail above.

Composition Type 17 contains: PPI1 enteric-coated or thin enteric-coated with buffering agent for gastric delivery + PPI2 timed-release (PPI2 delivery begins in duodenum, e.g. 1.5 to 2 hrs after ingestion). The components of such a composition include: **DIR** PPI1 + **TR** PPI2 + Buffer. Such a composition can comprise other components as described in detail above.

Composition Type 18 contains: PPI-salt enteric-coated or thin enteric-coated with buffering agent + PPI-base timed-release. The components of such a composition include: **DIR** PPI-salt + **TR** PPI-base + Buffer. Such a composition can comprise other components as described in detail above.

Composition Type 19 contains: PPI-salt timed-release with buffering agent + PPI-base enteric-coated or thin enteric-coated. The components of such a composition include: **TR** PPI-salt + **DIR** PPI-base + Buffer. Such a composition can comprise other components as described in detail above.

Composition Type 20 contains: PPI1 delayed-release + PPI2 delayed-release with buffering agent. The components of such a composition include: **DR** PPI1 + **DR** PPI2 + Buffer. Such a composition can comprise other components as described in detail above.

Composition Type 21 contains: PPI-salt delayed-release + PPI-base delayed-release with buffering agent. The components of such a composition include: **DR** PPI-salt + **DR** PPI-base + Buffer. Such a composition can comprise other components as described in detail above.

Composition Type 22 contains: PPI1 delayed-release + PPI2 timed-release with buffering agent (PPI2 delivery begins in duodenum, e.g. 1.5 to 2 hrs after ingestion). The components of such a composition include: **DR** PPI1 + **TR** PPI2 + Buffer. Such a composition can comprise other components as described in detail above.

Composition Type 23 contains: PPI-salt delayed-release + PI-base timed-release with buffering agent. The components of such a composition include: **DR** PPI-salt + **TR** PPI-base + Buffer. Such a composition can comprise other components as described in detail above.

Composition Type 24 contains: PPI-salt timed-release + PPI-base delayed-release with buffering agent. The components of such a composition include: **TR** PPI-salt + **DR** PPI-base + Buffer. Such a composition can comprise other components as described in detail above.

Composition Type 25 contains: PPI1 timed-release (PPI1 delivery begins in duodenum, e.g. 1.5 to 2 hrs after ingestion) + PPI2 timed-release (PPI2 delivery begins in duodenum, e.g. 1.5 to 2 hrs after ingestion) with buffering agent. The components of such a composition include: **TR** PPI1 + **TR** PPI2 + Buffer. Such a composition can comprise other components as described in detail above.

Composition Type 26 contains: PPI-salt timed-release + PPI-base timed-release with buffering agent. The components of such a composition include: **TR** PPI-salt + **TR** PPI-base + Buffer. Such a composition can comprise other components as described in detail above.

Compositions Types 1 through 26 optionally further include one or more of the following: an NSAID, an anti-*H*. *pylori* agent, or another pharmaceutical agent.

Compositions Types 1 through 26 may optionally further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 2

Applicant will conduct a double-blind, randomized, cross-over trial of patients with gastroesophageal reflux who will receive a single bedtime dose of PPI dosage formulation.

In this study, Applicant will enroll patients (sample size = 30 subjects) in a clinical trial having five (5) treatment arms as follows:

| Arm | Test Article Formulation |
|---|---|
| 1. | Enteric coated esomeprazole granules (Nexium®) 40 mg x 2 (80 mg esomeprazole) |
| 2. | Non-enteric coated tenatoprazole (80 mg) in sodium bicarbonate (20 mEq) |
| 3. | Non-enteric coated tenatoprazole (40 mg) + omeprazole (40 mg) in sodium bicarbonate (20 mEq) |
| 4. | Non-enteric coated omeprazole (40 mg) in sodium bicarbonate (20 mEq) + Nexium® (40 mg) |
| 5. | Non-enteric coated tenatoprazole (40 mg) + lansoprazole (40 mg) in sodium bicarbonate (20 mEq) |

Methodology: For each patient in each arm, gastric pH will be evaluated (e.g., by Bravo pH probe) to obtain:
(1) Median pH ± s.d., range
(2) Lowest pH
(3) Time pH < 4
(4) Time pH < 3.5;
(5) Time pH < 3;
(6) Integrated acidity;
   Each of these will be broken down into:
   a. Nighttime;
   b. Daytime; and
   c. Total 24 hr
   pH recordings will be taken at:
   a. First 24 hours;
   b. Second 24 hours;
   c. Day 5;
   d. Day 6;
After a 10 to 14 day washout period, the patients will be crossed over to another arm, and the crossover is repeated so that each patient will be treated under each arm. PPI serum concentration vs. time data will be collected on Day 1 and Day 5. Gastrin data (e.g., Gastrin 17 data) will be collected on Day 1 and Day 5, corresponding to the nighttime dose given on those perspective days.

Expectations: Based upon preliminary data the "optimal" pH control will be achieved by the following drug arms:
Day 1: Tenatoprazole (40 mg) + omeprazole (40 mg) in sodium bicarbonate (20 mEq)
Day 5: Tenatoprazole (40 mg) + omeprazole (40 mg) in sodium bicarbonate (20 mEq)
"Optimal" is defined as:
   o Few (if any) pH values < 3; thus time < 3 minimal (defined as < 0.5 hour)
   o Few (if any) pH values < 3; thus time < 4 minimal (defined as < 0.75 hour)
   o Integrated acidity lowest and below 10 mmol-hr/L per 24 hours
   o No NAB (nocturnal acid breakthrough, defined as pH < 4 for 1 hour or more)

Further studies using the compositions set forth in the 5 clinical arms may also be conducted where the compositions optionally further include one or more of the following: L-camosine, H2 blockers, NSAID, anti-H. pylori active substances, protein components, motility agents, iron, Vitamin B₁₂, pentagastrin or other pharmaceutical agents.

The compositions may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 3

Compositions can be prepared including a salt form and a free base form of a proton pump inhibitor. For example, a composition may comprise a buffering agent, a fast release dosage form of a rapid acting proton pump inhibitor with a short half-life, and a fast release dosage form of a slow acting proton pump inhibitor with a long half-life.

Illustratively, one example of the embodiments disclosed herein provides for a non-enteric coated composition comprising: sodium bicarbonate (20 mEq) + omeprazole sodium powder (42 mg) + tenatoprazole powder (40 mg).

Another example of the embodiments disclosed herein provides for a non-enteric coated composition comprising: sodium bicarbonate (20 mEq) + omeprazole sodium powder (40 mg) + tenatoprazole powder (40 mg).

The compositions may optionally further optionally further include one or more of the following: L-camosine, H2 blockers, NSAID, anti-H. pylori active substances, protein components, motility agents, iron, Vitamin B₁₂, pentagastrin or other pharmaceutical agents.

The compositions may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 4

The following non-enteric coated compositions, C27 - C34, are prepared as shown in Table 1. Such compositions can, if desired, be tableted or encapsulated or prepared as other dosage forms as described hereinabove.

| **Table** 1 | | | | | | | | |
|---|---|---|---|---|---|---|---|---|
| **Component** | **Amount (mg)** | | | | | | | |
| | **C27** | **C28** | **C29** | **C30** | **C31** | **C32** | **C33** | **C34** |
| ***PPI 1*** | | | | | | | | |
| Omeprazole | 5-90 | | | | 5-90 | | | |
| Esomeprazole | - | | - | 5-90 | | | | |
| Lansoprazole | - | | 5-120 | | | | | |
| Pantoprazole | | 5-120 | | | | | | |
| Rabeprazole | | | | | | 5-90 | | |
| S-lansoprazole | | | | | | | 5-120 | |
| Tenatoprazole | | | | | | | | 5-120 |
| S-tenatoprazote | | | | | | | | |

| ***PPI 2*** | | | | | | | | |
|---|---|---|---|---|---|---|---|---|
| Omeprazole | | | | | | | | |
| Esomeprazole | | | | | | | | |
| Lansoprazole | | | | | | | | |
| Pantoprazole | 5-120 | | | | | | | 5-120 |
| Rabeprazole | | 5-90 | | | | | | |
| S-lansoprazole | | | | | | | | |
| Tenatoprazole | | | | 5-120 | 5-120 | 5-120 | | |
| S-tenatoprazole | | | 5-120 | | | | 5-120 | |

| ***Buffering Agent**** | | | | | | | | |
|---|---|---|---|---|---|---|---|---|
| Sodium bicarbonate | 0-2000 | 0-2000 | 0-2000 | 0-2000 | 0-2000 | 0-2000 | 0-2000 | 0-2000 |
| Magnesium hydroxide | 0-2000 | 0-2000 | 0-2000 | 0-2000 | 0-2000 | 0-2000 | 0-2000 | 0-2000 |
| Calcium carbonate | 0-2000 | 0-2000 | 0-2000 | 0-2000 | 0-2000 | 0-2000 | 0-2000 | 0-2000 |

| | | | | | | | | |
|---|---|---|---|---|---|---|---|---|
| *One or more buffering agents may be included in each formulation. | | | | | | | | |

The compositions C27 - C34 of Table 1 may optionally further include one or more of the following: L-camosine, H2 blockers, NSAIDs, anti-H. pylori active substances, protein components, motility agents, iron, Vitamin B₁₂, pentagastrin or other pharmaceutical agents.

The compositions C27 - C34 of Table 1 may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 5

In various embodiments, the disclosure provides for tenatoprazole, esomeprazole (or any other proton pump inhibitor), and buffer in an enteric-coated formulation.

In one embodiment, the proton pump inhibitors can be mixed together and then enteric-coated as granules.

In another embodiment, tenatoprazole could be enteric coated as granules and the additional proton pump inhibitor could be separately enteric coated as granules.

In still another embodiment, either or both proton pump inhibitors could be enteric coated as tablets.

In another embodiment, either or both proton pump inhibitors could be in salt or free base form.

In still another embodiment, either or both proton pump inhibitors could be in sodium hydrate form.

The various compositions may optionally further optionally further include one or more of the following: L-camosine, H2 blockers, NSAIDs, anti-H. pylori active substances, protein components, motility agents, iron, Vitamin B₁₂, pentagastrin or other pharmaceutical agents.

The compositions may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 6

Compositions can be prepared including a salt form and a free base form of a proton pump inhibitor.

One example provides for gastric acid control by administering a composition comprising a mixture of sodium hydrate salt of tenatoprazole (or any other proton pump inhibiting agent such as s-tenatoprazole, omeprazole, esomeprazole, lansoprazole, or any isomer thereof), with the free base of tenatoprazole (or any other proton pump inhibiting agent such as s-tenatoprazole, omeprazole, esomeprazole, lansoprazole, or any isomer thereof), and a buffering agent.

Illustratively, one example of the embodiments disclosed herein provides for a non-enteric coated composition comprising: sodium hydrate salt of tenatoprazole (about 40 mg tenatoprazole) + free base of tenatoprazole (40 mg) + sodium bicarbonate (20 mEq).

The compositions may optionally further optionally further include one or more of the following: L-camosine, H2 blockers, NSAIDs, anti-H. pylori active substances, protein components, motility agents, iron, Vitamin B₁₂, pentagastrin or other pharmaceutical agents.

The compositions may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 7

Compositions can be prepared including a salt form of a proton pump inhibitor and a short-acting proton pump inhibitor.

One example provides for gastric acid control by administering a composition comprising a mixture of omeprazole (or any other short-acting proton pump inhibiting inhibiting agent or isomer thereof), the sodium hydrate salt of tenatoprazole (or any other proton pump inhibiting agent or isomer thereof), and a buffering agent.

Illustratively, one example of the embodiments disclosed herein provides for a non-enteric coated composition comprising: omeprazole (40 mg) + sodium hydrate salt of tenatoprazole (about 20 mg tenatoprazole) + sodium bicarbonate (20 mEq).

The compositions may optionally further optionally further include one or more of the following: L-carnosine, H2 blockers, NSAIDs, anti-H. pylori active substances, protein components, motility agents, iron, Vitamin B₁₂, pentagastrin or other pharmaceutical agents.

The compositions may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 8

Methodology: Applicant conducted a 3 subject cross-over study, where each subject served as its own control.

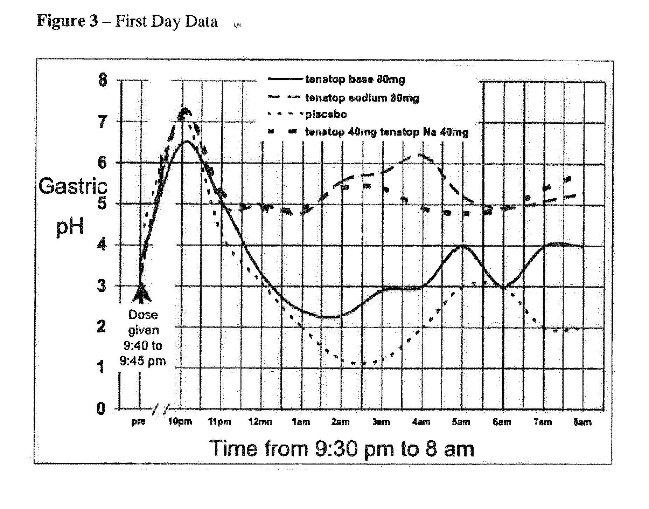

Results: tenatoprazole sodium (80 mg) and sodium bicarbonate (20 mEq) more quickly affected gastric pH than tenatoprazole base (80 mg) and sodium bicarbonate (20 mEq). First dose mean data is displayed as Figure 3.

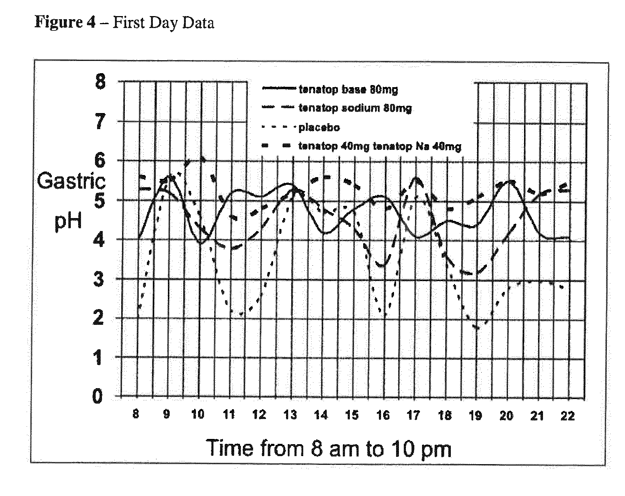

For the rest of the day (from 8 am to 10 pm - without redosing), the non-enteric coated mixture, in powder form, of tenatoprazole sodium (40 mg), tenatoprazole base (40 mg), and sodium bicarbonate (20 mEq) controlled gastric pH better than either the salt or free base form of tenatoprazole alone over the entire 24 hr period. See Figure 4.

The compositions of this study, for future studies, may optionally further optionally further include one or more of the following: L-carnosine, H2 blockers, NSAIDs, anti-H. pylori active substances, protein components, motility agents, iron, Vitamin B₁₂, pentagastrin or other pharmaceutical agents.

The compositions of this study, for future studies, may optionally further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 9

The following non-enteric coated compositions, C35 - C43, are prepared as shown in Table 2. Such compositions may be tableted or encapsulated or prepared as other dosage forms (with optional excipients) as described hereinabove.

| **Table 2** | | | | | | | | | |
|---|---|---|---|---|---|---|---|---|---|
| **Component** | **Amount (mg)** | | | | | | | | |
| | **C35** | **C36** | **C37** | **C38** | **C39** | **C40** | **C41** | **C42** | **C43** |
| ***PPI-Salt*** | | | | | | | | | |
| Tenatoprazole sodium hydrate | 5-100 | | | 5-100 | | | | 5-100 | |
| Omeprazole sodium | | 5-100 | | | | | | | |
| S-tenatoprazole sodium hydrate | | | 5-100 | | 5-100 | 5-100 | | | |
| Pantograzole sodium | | | | | | | 5-100 | | |

| ***PPI-Base*** | | | | | | | | | |
|---|---|---|---|---|---|---|---|---|---|
| Tenatoprazole | 5-100 | | | | | 5-100 | | | 5-100 |
| Omeprazole | | 5-100 | | 5-100 | 5-100 | | | | |
| S-tenatoprazole | | | 5-100 | | | | | | |
| Pantoprazole | | | | | | | 5-100 | | |
| Lansoprazole | | | | | | | 5-100 | 5-100 | 5-100 |

| ***Buffering Agent*** | | | | | | | | | |
|---|---|---|---|---|---|---|---|---|---|
| Sodium bicarbonate | 0-3000 | 0-3000 | 0-3000 | 0-3000 | 0-3000 | 0-3000 | 0-3000 | 0-3000 | 0-3000 |
| Magnesium hydroxide | 0-3000 | 0-3000 | 0-3000 | 0-3000 | 0-3000 | 0-3000 | 0-3000 | 0-3000 | 0-3000 |
| Calcium carbonate | 0-3000 | 0-3000 | 0-3000 | 0-3000 | 0-3000 | 0-3000 | 0-3000 | 0-3000 | 0-3000 |

The compositions C35 - C43 of Table 2 may optionally further include one or more of the following: L-camosine, H2 blockers, NSAIDs, anti-H. pylori active substances, protein components, motility agents, iron, Vitamin B₁₂, pentagastrin or other pharmaceutical agents.

The compositions C35 - C43 of Table 2 may further include disintegrants such as crosslinked polyvinylpyrrolidone (Crospovidone USP/NF) in an amount, for example, of about 1 to 5% weight to weight. Alternatively, other disintegrants include sodium CMC (carboxymethyl cellulose), chitin, or chitosan.

### Example 10

One example of the disclosure provides for an apparatus and method of administering one or more proton pump inhibitors and one or more buffering agents in a two (or more) part formulation.

The method of administration should enhance the stability of a pharmaceutical agent suspension, reduce the interference of additives, excipients, buffering agents, suspensions, liquids, reagents or solutions with pharmaceutical agents, and enables a product that can administer multiple doses of the suspension.

In one embodiment, such a method may be achieved by administering the suspensions in a multi-chambered apparatus. See Figures 5 to 10.

The pharmaceutical agent may include one or more of the following: one or more acid labile pharmaceutical agents, proton pump inhibitors, L-camosine, H2 blockers, NSAIDs, anti-H. pylori active substances, protein components, motility agents, iron, Vitamin B₁₂, pentagastrin or other pharmaceutical agents.

### Example 11

Several studies were planned and conducted to determine the stability of PPIs in solution. Results are summarized in the following charts.

Chart 1- Relationship of concentration to stability: This study showed that higher concentrations of PPIs were more stable than lower concentrations. When using a two chambered apparatus, for instance, the PPI was doubly concentrated on one side of the apparatus. Thus, the PPI solution was only at a 1x concentration for a very short time, while being dispensed and combined with the contents of the other chamber. Therefore, the PPI was not subject to storage degradation. 4 mg/ml, not shown in table, had 0% degradation

**Chart 1**

| | **Using Zegerid (omeprazole immediate release suspension) to make concentrations of : 0.6mg.mL, 1.2mg/mL, 3mg/mL, 4mg/mL** Burnett J. Balkin E Am J Health-Syst Pharm, 2006; 63:2240-7 | | |
|---|---|---|---|
| | **Concentration omeprazol (mg/mL)** | **% degradation 168 hr** | |
| | 0.6 | 13% | |
| | 1.2 | 7% | |
| | 2.0 | 0.5% to 2.5% | |
| | 3.0 | 0% | |
| | **Storage conditions: room temperature, 22-25 °C., light *n* = 3** | | |

Chart 2 - Stability Studies of PPI at high PH (pH = 10.4), low concentration buffer with 1 and 2 x concentration: The results summarized below show that at a pH of 10.4 or greater there was greater stability and that this stability was further increased when the concentration was doubled.

**Chart 2**

| | **Stability of Prevacid (lansoprazole) in 0.1 % Na2CO₃ (stored at 4° C, in the dark)** | | | | | |
|---|---|---|---|---|---|---|
| | **Day** | **2 X concentration Prevacid 9-7-05 exp #7-9** | | **1X concentration Prevacid 6-14-05 exp** | | |
| | | **mg/ml** | **% deg** | **mg/ml** | **% deg** | |
| | 0 | 5.88 | - | 3.47 | - | |
| | 7 | 5.81 | 1% | 3.30 | 5% | |
| | 14 | 5.71 | 3% | 3.29 | 5% | |
| | 28 | 5.73 | 3% | 3.23 | 7% | |
| | | | | | | |

Chart 3: Results from stability studies of PPI at high pH (pH> 10), low concentration buffer with 1 and 2 x concentration are summarized below in Chart 3.

**Chart 3**

| **Notes about PPI sample preparation** | | |
|---|---|---|
| PPI lested and concentration | In Na2CO3 pH (10.4) 0.1% 0.15% 0.2% | In alkaline water pH - 11.5 |
| Prevacid (lansoprazole) 6 mg/ml | Homogeneous suspension in about 1 hour without crushing. | Homogeneous suspension in about 1 hour without crushing |
| Prilosec (omeprazole) 4 mg/ml | Homogeneous suspension in about 3 hours without crushing | Homogenous suspension in about 6 hours without crushing. |
| Generic omeprazole 4mg/ml | Without crushing beads first, suspension turned purple 2 days after preparation, Also, without filtration, there was some chunks noticed in suspension. #46-48 crush first with mortar and pestle, no filtration, 80 chunks seen. | Beads would not go in suspension without crushing first, so crush first with mortar and pestle. |
| Nexum (esomeprazole) 4 mg/ml | Beads would not go in suspension without crushing first, so crush first with mortar and pestle | Beads would not go in suspension without crushing fust, so crush first with) mortar and paste Particles seen sticking to inside of tube after 1 weak. |
| Protonix (pantoprazole) 6 mg/ml | Cut tablets in half, then put in Na2CO3. After 6 hours, all of the tablet (including coaling) dissolves. | Cut tablets in half, then put in alkaline water. After sitting overnight, all the tablet (including coating) dissolves, |
| Aciphex (rabeprazole) 4 mg/ml | Cut tablets in half, then put in Na2CO3. After sitting overnight, all of the tablet dissolves, but coating remains, so use mesh filter to remove coating pieces. | Cut tablets in half, then put in alkaline water. After sitting overnight, all of the tablet dissolves, but coating remains, so use mesh filter to remove coating pieces. |

Chart 4: The results from one stability study are summarized below and reveal improved stability of PPIs at a high pH of 10.4.

**Chart 4**

| Stability of PPI's in 0.1 % Na₂CO₃ (pH=10.4) stored at 4° C in the dark (mean ± S.D. n=3) | | | | | | | | | | | | |
|---|---|---|---|---|---|---|---|---|---|---|---|---|
| Day | Prevacid (Suspend without crushing) | | Generic Omeprazole (crush beads) | | Prilosec (Suspend without crushing) | | Nexium (crush beads) | | Protonix (Cut tablet in half, suspend without crushing) | | Aciphex (Cut tablet in half, suspend without crushing) | |
| | mg/ml | % deg | mg/ml | % deg | mg/ml | % deg | mg/ml | % deg | mg/ml | % deg | mg/ml | % deg |
| 0 | 5.88 ± .041 | - | 3.86 ± .069 | - | 4.23 ± 131 | - | 417 ± .165 | - | 850 ± .093 | - | - | - |
| 1 | - | - | 3.90 ± .093 | 0.22% ± .37 | - | - | 3.92 ± .134 | 6.06% ± .56 | 6.38 ± .103 | 1.80% ± 1.67 | 4.00 ± .135 | - |
| 7 | 5.81 ± .132 | 1.19% ± 1.67 | 3.93 ± .089 | 0% ± 0 | 4.17 ± .138 | 1.58% ± .51 | 3.90 ± .125 | 6.46± ± 1.05 | 8.31 ± .042 | 2.22% ± 1.54 | 3.84 ± .072 | 4.09% ± 1.46 |
| 14 | 5.71 ± .066 | 2.90% ± .44 | 3.66 ± .055 | 5.09% ± 2.79 | 4.12 ± .066 | 2.66% ± 1.29 | 3.89 ± .065 | 6.60% ± 2.54 | 8.28 ± .051 | 2.65% ± .76 | 3.52 ± .103 | 11.9% ± 2.75 |
| 21 | 5.82 ± .128 | 1.24% ± 1.12 | 3.74 ± .123 | 3.22% ± 1.55 | 4.20 ± .132 | 1.62% ± 2.80 | 4.04 ± .077 | 3.12% ± 2.91 | 8.53 ± .101 | 0.27% ± .46 | 3.28 ± .073 | 18.0% ± 2.97 |
| 28 | 5.73 ± .058 | 2.62% ± .98 | 3.64 ± .100 | 5.74% ± 3.46 | 4.16 ± .038 | 2.14% ± 1.96 | 4.06 ± .113 | 2.68% ± 1.59 | 8.64 ± .132 | 0% ± 0 | 2.99 ± .050 | 25.1% ± 2.16 |
| BOLD indicates greater than 10% degradation | | | | | | | | | | | | |

Chart 5: This study revealed improved stability with increasing pH of Sodium Carbonate (0.1% pH = 10.4; 0.15% pH= 10.6; 0.2% pH=10.8).

**Chart 5**

| | Stability of PPI's in 0.1 %, 0.15 % & 0.2 % Na₂CO₃ stored at 4° C in the dark (mean ± S.D. n=3) | | | | | | | | | | | |
|---|---|---|---|---|---|---|---|---|---|---|---|---|
| | Crush omeprazole beads first no filtration | | | | | | Cut Aciphex tablet in half, next day use mesh filter | | | | | |
| Day | Generic Omeo in .1% Na₂CO₃ | | Generic Omeo in .15% Na₂CO₃ | | Generic Omeo in .2% Na₂CO₃ | | Aciphex in .1% Na₂CO₃ | | Aciphex in .15% Na₂CO₃ | | Aciphex in .2% Na₂CO₃ | |
| | mg/ml | % deg | mg/ml | % deg | mg/ml | % deg | mg/ml | % deg | mg/ml | % deg | mg/ml | % deg |
| 0 | 3.86 ± .069 | - | 3.92 ± .061 | - | 3.92 ± .031 | - | - | - | - | - | - | - |
| 1 | 3.90 ± .093 | 0.22% ± .37 | 3.93 ± .047 | 077% ± 1.33 | 3.88 ± .061 | 1.5% ± 1.82 | 4.00 ± .135 | - | 4.01 ± .057 | - | 4.05 ± .067 | - |
| 7 | 3.93 ± .089 | 0% ± 0 | 3.92 ± .038 | 0.23% ± .39 | 3.93 ± .033 | 0% ± 0 | 3.84 ± .072 | 4.09% ± 1.46 | 3.88 ± .111 | 3.2% ± 1.48 | 3.96 ± .046 | 2.18% ± .77 |
| 14 | 3.66 ± .056 | 5.09% ± 2.79 | 4.02 ± .127 | 0% ± 0 | 3.96 ± .047 | 0% ± 0 | 3.52 ± .103 | 11.9% ± 2.75 | 3.81 ± .022 | 4.84% ± 1.59 | 3.85 ± .071 | 4.84% ± 1.73 |
| 21 | 3.74 ± .123 | 3.22% ± 1.55 | 3.86 ± .069 | 1.58% ± .44 | 3.88 ± .006 | 1.08% ± .76 | 3.28 ± .073 | 18.0% ± 2.97 | 3.64 ± .050 | 9.15% ± 0.30 | 3.72 ± .156 | 8.11% ± 3.94 |
| 28 | 3.64 ±.100 | 5.74% ± 3.46 | 3.71 ± .087 | 5.22% ± 2.22 | 3.82 ± .135 | 2.81% ± 2.68 | 2.99 ± .050 | 25.1% ± 2.18 | 3.47 ± .027 | 13.4% ± .71 | 3.58 ± .089 | 11.5% ± 3.13 |
| BOLD indicates greater than 10% degradation | | | | | | | | | | | | |

Chart 6: This study revealed improved stability of PPIs at a high pH of 11.5.

**Chart 6**

| Stability of PPI's in Alkaline water pH =11.5 stored at 4° C in the dark (mean ± S.D. n=3) | | | | | | | | | | | | |
|---|---|---|---|---|---|---|---|---|---|---|---|---|
| Day | Prevacid (Suspend without crushing) | | Genenric Omeprazole (crush beads) | | Prilosec (Suspend without crushing) | | Nexium (crush beads) | | Protonix (Cut tablet in half and suspend without crushing) | | Aciphex (Cut tablet in half and suspend without crushing) | |
| | mg/ml | % deg | mg/ml | % deg | mg/ml | % deg | mg/ml | % deg | mg/ml | % deg | mg/ml | % deg |
| 0 | 5.08 ± .038 | - | 3.80 ± .079 | - | - | - | 3.70 ± .117 | - | - | - | - | - |
| 1 | - | - | 4.24 ± .087 | - | 3.88 ± .071 | - | 3.63 ± .093 | 1.98% ± .62 | 8.08 ± .072 | - | 4.05 ± .016 | - |
| 7 | 5.96 ± .111 | 2.03% ± 1.29 | 4.42 ± .035 | 0% ± 0 | 3 88 ± .081 | 0.40% ± .69 | 3.76 ± .075 | 0% ± 0 | 8.16 ± .013 | 0% ± 0 | 4.10 ± .033 | 0% ± 0 |
| 14 | 6.05 ± .095 | 0.66% ± .82 | 4.44 ± .072 | 0% ± 0 | 3.99 ± 0.73 | 0% ± 0 | 3.74 ± .085 | 0.65% ± .80 | 8.34 ± .191 | 0% ± 0 | 4.09 ± .046 | 0.14% ± .24 |
| 21 | 5.65 ± .133 | 7.14% ± 1.69 | 4.54 ± .198 | 0% ± 0 | 3.90 ± .058 | 0,13% ± .23 | 3.69 ± .026 | 1.49% ± 2.58 | 8.09 ± .159 | 037% ± .58 | 4.12 ± .061 | 0.05% ± .09 |
| 28 | 5.72 ± .035 | 5.91% ± 1.08 | 4.42 ± .210 | 0 % ± 0 | 3.90 ± .079 | 0.26% ± .31 | 3.66 ± .026 | 1.76% ± 2.66 | 8.12 ± .147 | 0.17% ± .30 | 4.02 ± .069 | 0.95% ± 1.65 |
| BOLD Indicates greater than 10% degradation | | | | | | | | | | | | |

Chart 7: Data showing lower pH and effect on stability are summarized below.

**Chart 7**

| PPI's in sodium bicarbonate store 4° C dark (pH 8 to 8.4) | | | | | | |
|---|---|---|---|---|---|---|
| day | Prevacid (lansoprazole) n =2 8.4% NaHCO3 | | Protonix (pantoprazole) n = 2 4.2 % NaHCO3 | | Aciphex (rabeprazole) n = 1 0.8% NaHCO3 | |
| | mg/ml | % deg | mg/ml | % deg | mg/ml | % deg |
| 0 | 2.98 | - | 2.13 | - | 2.19 | - |
| 7 | 2.95 | 1.3% | 2.06 | 4.7% | 1.34 | 39% |
| 14 | 2.80 | 6% | 2.01 | 5% | 0.82 | 63% |

### Example 12

Illustrative example of a multi-chambered apparatus (pump-dispenser or syringe):

Chamber 1 comprises PPI or PPIs, optionally a small amount of buffer with a high pH (e.g., alkaline water pH > about 11, sodium carbonate 0.2% pH about 10.8) such that the buffer has no neutralizing capacity, and optionally a thickening agent that is stable in alkaline pH.

Chamber 2 comprises buffer (e.g., sodium bicarbonate, calcium carbonate, magnesium hydroxide, or any buffer disclosed herein) and optionally any combination of the following: excipients, flavors, sugars, thickeners, or any other compositions, substances or agents disclosed herein.

Upon actuation of the pump head or the plunger, the contents of the separate compartments mix together. The neutralizing capacity may be determined by the buffer in Chamber 2, and the pH of the combined two compartments may reach the pH of the buffer in Chamber 2.

The concentration of PPI in Chamber 1 may be greater than the desired final concentration of PPI for administration after mixing with Chamber 2. For example, if both chambers are the same size, the PPI concentration in Chamber 1, may be 2X, where X is the final concentration desired for administration to a patient.

### Example 13

The following non-enteric coated compositions, C44 - C51, are prepared as shown in Table 3. Such compositions may be tableted or encapsulated or prepared as other dosage forms (with optional excipients) as described hereinabove.

| **Table 3** | | | | | | | | |
|---|---|---|---|---|---|---|---|---|
| **Component** | **Amount (mg)** | | | | | | | |
| | **C44** | **C45** | **C46** | **C47** | **C48** | **C49** | **C50** | **C51** |
| ***Chamber 1*** | | | | | | | | |
| Lansoprazole | | | | 5-800 | 5-800 | | 5-800 | 5-800 |
| Omeprazole | 5-800 | | | | | | | |
| Omeprazole sodium | | | 5-800 | | | | | |
| Tenatoprazole | 5-800 | 5-800 | | 5-800 | | 5-800 | | |
| Pantoprazole | | | 5 - 1200 | | | 5 - 1200 | | 5-1200 |
| S-tenatoprazole | | | | | | | 5-800 | |
| Tenatoprazole sodium hydrate | | 5-800 | | | 5-800 | | | |
| Water / Sodium carbonate 0.1 to 0.2% | qs to 100 mL | qs to 100 mL | | qs to 100 mL | | | | |
| Alkaline water pH approx 11 to 11.5 | | | qs to 100 mL | | qs to 100 mL | qs to 100 mL | qs to 100 niL | qs to 100 mL |

| ***Chamber 2*** | | | | | | | | |
|---|---|---|---|---|---|---|---|---|
| Sweetener | sucrose, aspartame, sucralose | sucralose, thaumatin, sorbitol | acesulfame -K, aspartame, sucrose | neotame, sucrose | neotame, acesulfame -K, sucrose | Sucrose, neotame, sucralose | sucrose, aspartame, sucralose | aspartame, saccharin, sucrose |
| Flavoring agent | cherry | chocolate | fruit | bubble-gum | caramel | coffee | butter-scotch | milk |
| Water | qs to 100 mL | qs to 100 mL | qs to 100 mL | qs to 100 mL | qs to 100 mL | qs to 100 mL | qs to 100 mL | qs to 100 mL |
| Sodium bicarbonate | 0-3500 | 0-3500 | 0-3500 | 0-3500 | 0-3500 | 0-3500 | 0-3500 | 0-3500 |
| Calcium carbonate | 0-3500 | 0-3500 | 0-3500 | 0-3500 | 0-3500 | 0-3500 | 0-3500 | 0-3500 |
| Magnesium hydroxide | 0-3500 | 0-3500 | 0-3500 | 0-3500 | 0-3500 | 0-3500 | 0-3500 | 0-3500 |

The compositions C44 - C51 include at least one buffer listed in the table or an equivalent amount of buffer, based upon acid neutralizing capacity, disclosed herein.

The compositions C44 - C51 of Table 3 may optionally further include one or more of the following: L-carnosine, H2 blockers, NSAIDs, anti-H. pylori active substances, protein components, motility agents, iron, Vitamin B₁₂, pentagastrin or other pharmaceutical agents.

### Example 14

Embodiments of the disclosure comprising an immediate-release PPI may provide therapy in subjects with gastroparesis (slow stomach emptying). For example, immediate-release omeprazole (40 mg powder) may be better absorbed in patients with gastroparesis than delayed-release omeprazole (40 mg capsule). A delayed-release PPI has a protective coating, such as an enteric coating, to prevent the coated PPI from being neutralized by stomach acid. In contrast, embodiments comprising immediate-release PPI such as omeprazole and a buffering agent such as sodium bicarbonate, do not need a protective coating. As such, various dosage forms of the disclosed invention, comprising an immediate-release PPI may have a more rapid pharmacokinetic profile and greater overall drug absorption in gastroparesis than delayed-release PPI.

### Example 15

Powder for oral suspension:
200 mg cimetidine
80 mg omeprazole
80 mg lansoprazole
1680 mg sodium bicarbonate
600 mg croscarmellose sodium
2.00 g sugar (sucrose)
30 mg banana flavor
15 mg tutti frutti flavor
40 mg Beflora
15 mg Talin
2 mg Neotame

The composition may optionally further include one or more of the following: L-camosine, H2 blockers, NSAIDs, anti-H. pylori active substances, protein components, motility agents, iron, Vitamin B₁₂, pentagastrin or other pharmaceutical agents.

The above compositions, can be prepared as other dosage forms (with optional excipients), such as tablets, capsules, chewable tablets, powders, granules, powder for suspension, or otherwise as described hereinabove.

### Example 16

A tablet (or caplet) or capsule would contain the following:
200 mg cimetidine
80 mg omeprazole
80 mg lansoprazole
1680 mg sodium bicarbonate
600 mg croscarmellose sodium

The composition may optionally further include one or more of the following: L-camosine, H2 blockers, NSAIDs, anti-H. pylori active substances, protein components, motility agents, iron, Vitamin B₁₂, pentagastrin or other pharmaceutical agents.

The above compositions, can be prepared as other dosage forms (with optional excipients), such as tablets, capsules, chewable tablets, powders, granules, powder for suspension, or otherwise as described hereinabove.

### Example 15

Powder for oral suspension:
200 mg cimetidine
80 mg omeprazole
80 mg lansoprazole
1680 mg sodium bicarbonate
600 mg croscarmellose sodium
2.00 g sugar (sucrose)
30 mg banana flavor
15 mg tutti frutti flavor
40 mg Beflora
15 mg Talin
2 mg Neotame

The composition may optionally further include one or more of the following: L-carnosine, H2 blockers, NSAIDs, anti-H. pylori active substances, protein components, motility agents, iron, Vitamin B₁₂, pentagastrin or other pharmaceutical agents.

The above compositions, can be prepared as other dosage forms (with optional excipients), such as tablets, capsules, chewable tablets, powders, granules, powder for suspension, or otherwise as described hereinabove.

### Example 17

Several pellets were prepared, each comprising non-enteric coated omeprazole, sodium bicarbonate, pregelatinized starch, sucrose and flavoring agent (apple). These ingredients were dry blended and compressed in Parr pellet press. The pellets were then administered to a horse as follows.

Little Joe, a 545 kg horse experiencing gastric ulcer, was given several large pellets as described above. The total dose of omeprazole administered was 1090 mg (2 mg/kg). Blood was drawn at several time points over a period of four hours following administration. Plasma was collected from the horse in tubes without anti-coagulant. The blood was allowed to clot and was then centrifuged; serum was frozen for later extraction. Extraction was performed using 75 microliter of 0.1 M sodium bicarbonate and 3 microgram/ml of lansoprazole (internal standard) were added to 0.5 ml serum. Five ml of ether:dichloromethane (7:3) were added to each tube. The tubes were mixed by vortexing for 20 seconds, and centrifuged for 10 min at 2500 RPM. A 2.5 ml aliquot of the top organic layer was transferred to a new tube and the solvent was evaporated using a stream of oxygen. The dry samples were reconstituted in 0.25 ml of the HPLC mobile phase.

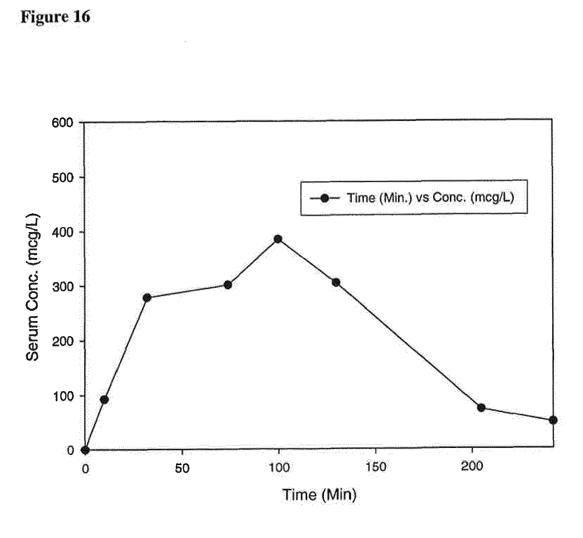

The HPLC column was an Alltech Prevail C18 5 micron, 15 cm length. The mobile phase consisted of 50% of 15 mM sodium phosphate pH 7.4, 40% methanol and 10% acetonitrile used at a flow rate of 1.1 ml/min. A UV detector was used to detect the peaks at 300 nm. Data are shown in Figure 16, below.

### Example 18

The following formulations, shown in Table 4, can be prepared as tablets, capsules, or other solid dosage forms.

**Table 4**

| | **Amount (mg)** | | | | | | | |
|---|---|---|---|---|---|---|---|---|
| **Component** | **1** | **2** | **3** | **4** | **5** | **6** | **7** | **8** |
| Polymerized omeprazole | 10 | 20 | 40 | 60 | | | | |
| Polymerized lansoprazole | | | | | 10 | 15 | 30 | 45 |
| Polymerized tenatoprazole | 10 | 20 | 40 | 60 | | | | |
| Polymerized pantoprazole | 10 | 20 | 40 | 60 | | | | |
| Sodium bicarbonate | 900 | 800 | 600 | 1600 | 1200 | 800 | 600 | 1400 |
| Calcium carbonate | | | | 100 | | 200 | | |
| Sodium carbonate | | | 600 | | | | 300 | |
| Magnesium hydroxide | 300 | 400 | | | | 100 | | |

### Example 19

The following compositions, shown in Table 5 can, if desired, be tableted or encapsulated or prepared as other dosage forms as described hereinabove. Furthermore, the PPIs, anti-*H*. *pylori* active substance and buffering agents are interchangeable with on another.

**Table 5**

| **Component** | **Amount (mg)** | | | | | | |
|---|---|---|---|---|---|---|---|
| | **1** | **2** | **3** | **4** | **5** | **6** | **7** |
| ***PPI*** | | | | | | | |
| Omeprazole | 10-60 | - | 10 - 60 | - | - | - | - |
| Tenatoprazole | - | 20-80 | - | | | | |
| Esomeprazole | - | - | - | 10-60 | - | - | - |
| Lansoprazole | - | - | - | - | 10-60 | - | - |
| Pantoprazole | - | 20-80 | - | - | - | - | - |
| Rabeprazole | - | - | - | - | - | 10-60 | - |
| S-lansoprazole | - | - | - | - | - | - | 10-90 |

| ***Anti-H. pylori active substance*** | | | | | | | |
|---|---|---|---|---|---|---|---|
| Tetracycline | 200-1000 | - | - | - | 200-1000 | - | - |
| Amoxicillin | - | 500 -2000 | - | - | - | 500 -2000 | - |
| Clarithromycin | - | - | 500 -2000 | - | - | - | 500 -2000 |
| Metronidazole | - | - | - | 200-1000 | - | - | - |

| ***Buffering Agent*** | | | | | | | |
|---|---|---|---|---|---|---|---|
| Sodium bicarbonate | 800-2000 | - | - | 800-2000 | - | - | 800 -2000 |
| Magnesium hydroxide | - | 600-2000 | - | - | 600-2000 | - | - |
| Calcium carbonate | - | - | 600 -2000 | - | - | 600-2000 | - |

The above compositions may optionally comprise a bismuth compound, for example colloidal bismuth subcitrate, in an amount of about 100 mg to about 800 mg.

### Example 20

The measure of a PPIs activity can be predicted by looking at AUC above 1 micromole. The elimination rate (Kel) is the only variable that can be changed to increase the AUC for a given PPI. Half-life = 0.693/Kel

Integrated Gastric Acidity (IGA) is a sensitive and specific measure of acid secretion and acid inhibition. (Integrated gastric acidity is calculated as the cumulative time-weighted average of the gastric acid concentration. Integrated gastric acidity is sensitive to the change from baseline for gastric acidity, whereas median gastric pH has low sensitivity in detecting change (ie, drug induced) from baseline.)

Significant control (IGA < 100 mmol.h/L) of gastric pH on the first 24 hours (Day 1) of treatment has not been possible with standard treatments.

These are all n of 1 studies all performed on the same subject. (Unless otherwise stated, 1.68g NaHCO3 was used as buffering agent)

Baseline = no treatment
o Day 1 Baseline: IGA with no treatment (1 meal) is 600 to 650 mmol.
Day 1 Baseline: IGA with no treatment (no meal) is 750 to 800 mmol.h/L

Standardly available treatment
Day 1 Esomeprazole 40mg (Nexium): IGA is 317 mmol.h/L; half-life esomeprazole = 1.4hr
Day 1 Omeprazole Immediate-release (OME-IR) 40mg: IGA is 202 mmol.h/L; half-life omeprazole = 1.1hr

Experimental treatment
Day 1 Tenatoprazole IR 40mg: IGA is 255 mmol.h/L; half-life tenatoprazole = 7 hr
Day 1 Tenatoprazole IR 120mg: IGA is 242 mmol.h/L; half-life tenatoprazole = 8.7 hr
Day 1 Omeprazole IR 120mg: IGA was not recorded; half-life omeprazole = 1.8 hr
Day 1 OME-IR 80mg + Lansoprazole IR 80mg: IGA is 117 mmol.h/L; half-life omeprazole = 1.4hr; half-life lansoprazole = 3.1 hr
Day 1 OME-IR 120mg + Tenatoprazole IR 40mg: IGA is 28 mmol.h/L; half-life omeprazole = 1.8hr; half-life tenatoprazole = 12.9 hr
Day 1 OME-IR 120mg + Cimetidine 200mg: IGA is 56 mmol.h/L; half-life omeprazole = 2.1hr
Day 1 OME-IR 120mg + Tenatoprazole IR 40mg + Cimetidine 200mg: IGA is 28 mmol.h/L; half-life omeprazole = 1.8hr; half-life tenatoprazole = 12.9 hr

All references, including publications, patent applications, and patents, cited herein are hereby incorporated by reference to the same extent as if each reference there individually and specifically indicated to be incorporated by reference and were set forth in its entirety herein.

The use of the terms "a" and "an" and "the" and similar referents in the context of this disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., such as, preferred, preferably, particularly) provided herein, is intended merely to further illustrate the content of the disclosure and does not pose a limitation on the scope of the claims. No language in the specification should be construed as indicating any non-claimed element as essential to the practice of the claimed invention.

Alternative embodiments of the claimed invention are described herein, including the best mode known to the inventors for carrying out the claimed invention. Of these, variations of the disclosed embodiments will become apparent to those of ordinary skill in the art upon reading the foregoing disclosure. The inventors expect skilled artisans to employ such variations as appropriate, and the inventors intend for the claimed invention to be practiced otherwise than as specifically described herein.

Accordingly, the claimed invention includes all modifications and equivalents of the subject matter recited in the claims appended hereto as permitted by applicable law. Moreover, any combination of the above-described elements in all possible variations thereof is encompassed by the claimed invention unless otherwise indicated herein or otherwise clearly contradicted by context.

The use of individual numerical values are stated as approximations as though the values were preceded by the word "about" or "approximately." Similarly, the numerical values in the various ranges specified in this application, unless expressly indicated otherwise, are stated as approximations as though the minimum and maximum values within the stated ranges were both preceded by the word "about" or "approximately." In this manner, variations above and below the stated ranges can be used to achieve substantially the same results as values within the ranges. As used herein, the terms "about" and "approximately" when referring to a numerical value shall have their plain and ordinary meanings to a person of ordinary skill in the art to which the disclosed subject matter is most closely related or the art relevant to the range or element at issue. The amount of broadening from the strict numerical boundary depends upon many factors. For example, some of the factors which may be considered include the criticality of the element and/or the effect a given amount of variation will have on the performance of the claimed subject matter, as well as other considerations known to those of skill in the art. As used herein, the use of differing amounts of significant digits for different numerical values is not meant to limit how the use of the words "about" or "approximately" will serve to broaden a particular numerical value. Thus, as a general matter, "about" or "approximately" broaden the numerical value. Also, the disclosure of ranges is intended as a continuous range including every value between the minimum and maximum values plus the broadening of the range afforded by the use of the term "about" or "approximately." Thus, recitation of ranges of values herein are merely intended to serve as a shorthand method of referring individually to each separate value falling within the range, unless otherwise indicated herein, and each separate value is incorporated into the specification as if it there individually recited herein.

It is to be understood that any ranges, ratios and ranges of ratios that can be formed by, or derived from, any of the data disclosed herein represent further embodiments of the present disclosure and are included as part of the disclosure as though they were explicitly set forth. This includes ranges that can be formed that do or do not include a finite upper and/or lower boundary. Accordingly, a person of ordinary skill in the art most closely related to a particular range, ratio or range of ratios will appreciate that such values are unambiguously derivable from the data presented herein.

## Claims

1. A multi-chambered apparatus comprising:
(a) a first chamber wherein one or more first pharmaceutically active agents, protective buffers or thickeners can be contained;
(b) a second chamber wherein one or more second pharmaceutically active agents, flavoring agents, sweeteners, disintegrants, additives, buffering agents, or thickeners can be contained; and
(c) a dispenser head.

2. The apparatus of claim 1, further comprising an outlet to connect to a syringe.

3. The apparatus of claim 1, further comprising a connector between the dispenser and first and second chambers.

4. The apparatus of claim 1 wherein the first chamber is a syringe body with first plunger and the second chamber is a syringe body with second plunger, wherein the apparatus preferably further comprises an attachment that connects the first plunger with the second plunger, wherein the attachment is optionally removable for cleaning and reuse.

5. The apparatus of any of the preceding claims 1-4 further comprising an optionally removable component that combines the one or more first pharmaceutically active agents, protective buffers or thickeners from the first chamber with the one or more second pharmaceutically active agents, flavoring agents, sweeteners, disintegrants, additives, buffering agents, or thickeners from the second chamber and delivers them into a dosing syringe.

6. The apparatus of claim 1 wherein the multi-chambered apparatus is a multi-chambered syringe.

7. The apparatus of any of the preceding claims 1-6 wherein the dispenser comprises a volumetric dial for preparing multidosing formulations.

8. The apparatus of any of the preceding claims 1-7 wherein the first pharmaceutically active agent is selected from the group consisting of one or more proton pump inhibitors, or one or more of the following: L-carnosine, H2 blockers, NSAIDs, anti-H. pylori active substances, protein components, motility agents, iron, Vitamin B₁₂, pentagastrin or other pharmaceutical agents.

9. The apparatus of claim 8 wherein the second chamber comprises an antibiotic.

10. The apparatus of any of the preceding claims 1-9, further comprising a third chamber.

11. A method of using the apparatus of any of the preceding claims 1 to 10.

12. The method of claim 11 for pre-preparing multidose formulations for oral administration of pharmaceuticals, additives, excipients, buffering agents, suspensions, liquids, reagents, solutions or dry formulations constituted with water or other suitable liquid.

13. The method of claim 11, using the apparatus of claim 9, wherein the pharmaceuticals are stored separately until dosing.

14. The method according to claim 11, wherein the first chamber contains a first antibiotic, the second chamber contains a second antibiotic, and said first and second antibiotics are administered in one dose for multiple doses.

15. The method of claim 11, using the apparatus of claim 7, comprising the steps of:
loading a first pharmaceutical, additive, buffering agent, suspension, liquid, reagent or solution in the first chamber;
optionally loading a liquid or suspension form of a second pharmaceutical, additive, buffering agent, suspension, liquid, reagent or solution in the second chamber;
optionally loading one or more additional pharmaceuticals, additives, excipients, buffering agents, suspensions, liquids, reagents or solutions in one or more additional chambers;
setting the dial to a predetermined volume ratio for multiple chambers; and
operating the dispenser to dispense a mixture from the first and each additional chambers for oral administration at each dosing.

16. A method according to claim 11 of administering one or more proton pump inhibitors in multiple doses comprising:
loading a PPI in a suspension with a pH ranging from about 7, about 6.5 to about 15 in the first chamber; and
loading a buffer solution with a pH ranging from about 5 to about 10 in the second chamber.

17. Use of the apparatus of any of the preceding claims 1-10 for administration of one or more pharmaceutically active compounds to treat a subject who suffers from a condition selected from an acid-caused gastrointestinal disorder selected from the group consisting of duodenal ulcer, gastric ulcer, stress ulcer, acid dyspepsia, gastroesophageal reflux disease (GERD), gastroparesis, severe erosive esophagitis, poorly responsive symptomatic gastroesophageal reflux disease, acid reflux, heartburn, nighttime heartburn symptoms, Barrett's esophagus, acid hypersecretory conditions, gastrointestinal pathological hypersecretory conditions (such as Zollinger Ellison Syndrome), gastrointestinal bleeding, acute upper gastrointestinal bleeding, non-ulcer dyspepsia, heartburn, ulcers induced by NSAIDs, atypical reflux conditions, laryngitis, chronic cough, otitis media, sinusitis, eye pain, globus sensation, esophagitis, erosive esophagitis, adenocarcinoma of the esophagus, gastrinoma, *Helicobacter pylori (H. pylori*) infection, irritable bowel syndrome (IBS), inflammatory bowel disease (IBD), ulcerative colitis, pre- or post-operative acid aspiration, Crohn's disease, asthma, sleep apnea, sleep disturbance, psoriasis, and diseases related to any of the above-mentioned conditions.
